# EUROPEAN PATENT APPLICATION

(11) **EP 3 156 398 A1**
(43) Date of publication of application: **19.04.2017**
(21) Application number: 15190211.1
(22) Date of filing: 16.10.2015
(51) Int. Cl.: C07D 217/26, A61K 31/472, A61P 29/00

(54) **AGONISTS OF THE CHEMOKINE RECEPTOR CXCR3**

(71) Applicant: Friedrich-Alexander-Universität Erlangen-Nürnberg, 91054 Erlangen (DE)
(72) Inventor: Tschammer, Nuska, 81379 Munich (DE); Milanos, Lampros, 91088 Bubenreuth (DE); Brox, Regine, 91054 Erlangen (DE)
(74) Representative: Engelhard, Markus

(57) **Abstract**

The present invention relates to agonists of the chemokine receptor CXCR3, methods of their synthesis and uses thereof.

## Description

The present invention relates to agonists of the chemokine receptor CXCR3, methods of their synthesis and uses thereof.

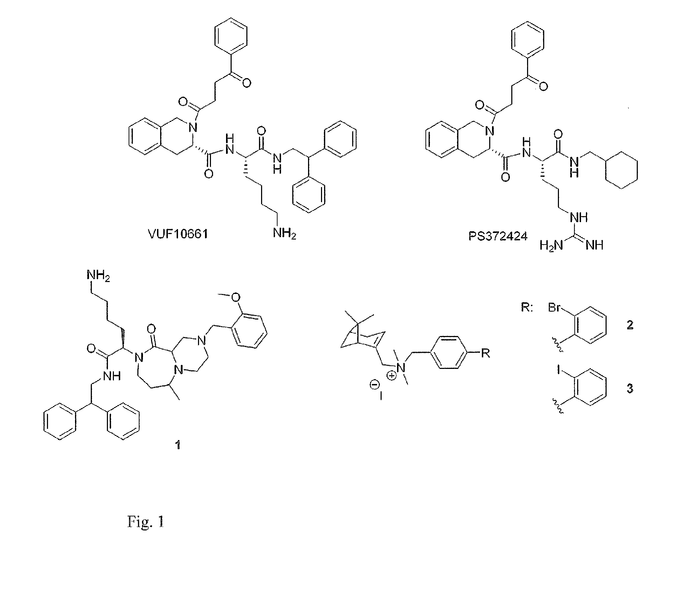

Biased signaling or functional selectivity are the terms that describe a now well recognized phenomenon observed in the G protein coupled receptors (GPCRs). The foundation of this phenomenon rests in the ability of ligands acting at the same GPCR to stabilize distinct receptor conformations that, in turn, are linked to different functional outcomes¹⁻³. As in the case of the chemokine receptor CXCR3, which binds three endogenous chemokines CXCL9, CXCL10 and CXCL11, biased signaling is used by nature to fine tune the responses of CXCR3⁺ cells upon physiological insult⁴. CXCR3 expression is rapidly induced on naive T cells following activation, and preferentially remains highly expressed on type1 helper (Thl) CD4⁺ T cells, effector CD8⁺ T cells and innate-type lymphocytes, such as natural killer (NK) and natural killer T (NKT) cells^{5,6}. Depending on the endogenous chemokine bound, the activation of CXCR3 leads to distinct functional outcomes, which considerably influence the course of inflammation and healing processes^{5,7}. The opportunity to mimic this mechanism by small molecule allosteric ligands has remained largely unexploited, even though CXCR3 arose as a promising therapeutic target in pathologies like autoimmune diseases (e.g., rheumatoid arthritis, multiple sclerosis), cancer and metastasis⁸⁻¹². To inhibit the inflammatory response driven by the CXCR3⁺ cells, published research was focused solely on the development of small-molecules with inhibitory effects on the CXCR3, which ultimately failed to deliver theraputics¹³⁻¹⁵. The development of small-molecule agonists of CXCR3 was largely neglected and only few agonists were reported (Figure 1)¹⁶⁻¹⁸, despite rising indications that chemokine receptor agonists may have significant therapeutic potential in wound healing¹⁹ and anti-inflammatory therapy^{20,21}.

Current treatment of inflammatory diseases follows various strategies none of which are ideal. For example, treatment with corticosteroids is hampered by considerable side effects. Therefore, there is a need in the art for novel strategies and novel means for treating inflammatory diseases and/or disorders associated with an inflammatory component, such as Alzheimer disease, diabetes, ischemia, autoimmune diseases, such as rheumatoid arthritis, multiple sclerosis, and cancer. It was therefore an object of the present invention to provide for new means for treating such diseases. In particular, it was an object of the present invention to provide for means that make use of an interaction with the chemokine receptor CXCR3.

All these objects are solved by a compound having the structure represented by formula I 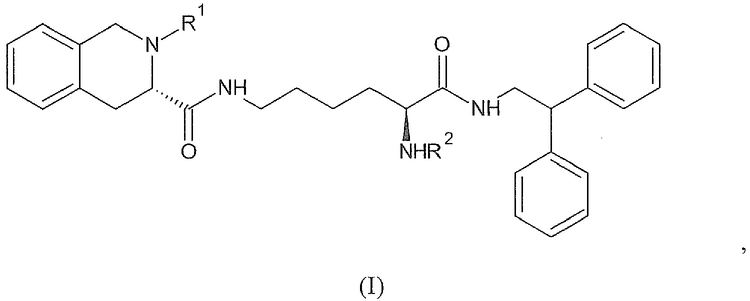 wherein
R¹ is H or a radical represented by formula II 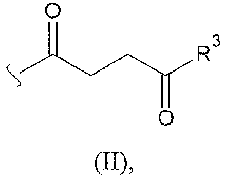 or formula III 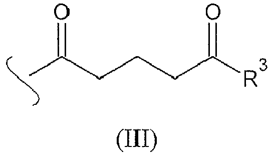 wherein R² is H or tert-butyloxycarbonyl (Boc),
with R³ being methyl,
phenyl 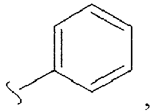 or 4-methoxy-phenyl 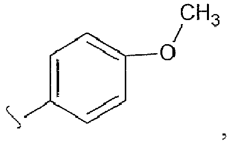 or epimers of said compound,
or a pharmaceutically acceptable salt thereof or of its epimers.

In one embodiment, R¹ is H, R² is tert-butyloxycarbonyl (Boc) or H.

In one embodiment R¹ is
a radical represented by formula II 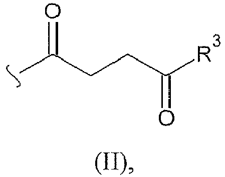 R² is tert-butyloxycarbonyl (Boc) or H,
with R³ being methyl or phenyl.

In one embodiment, R¹ is
a radical represented by formula II 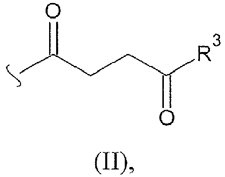 R² is tert-butyloxycarbonyl (Boc) or H,
with R³ being 4- methoxy-phenyl.

In one embodiment, R¹ is a radical represented by formula III

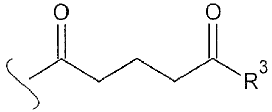

R² is tert-butyloxycarbonyl (Boc) or H, and R³ is phenyl.

In one embodiment, the compound according to the present invention is selected from 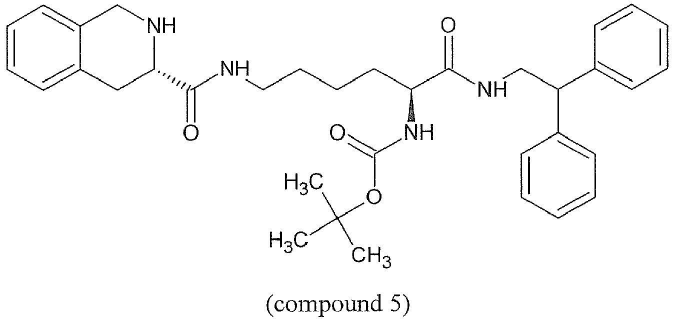 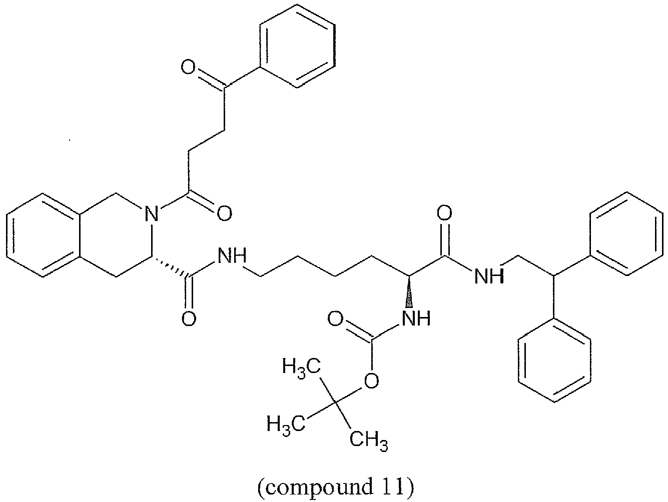 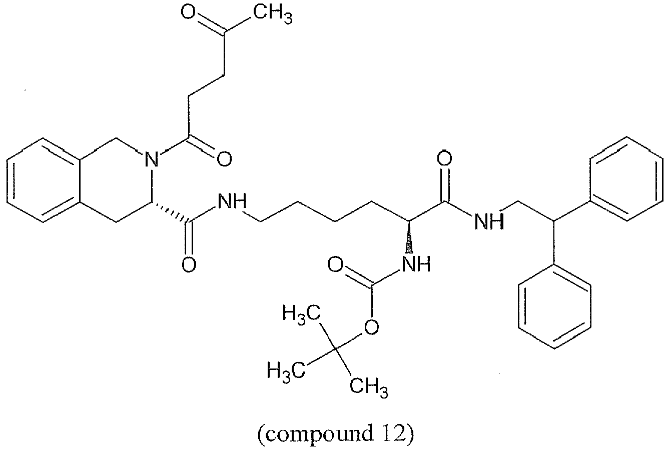 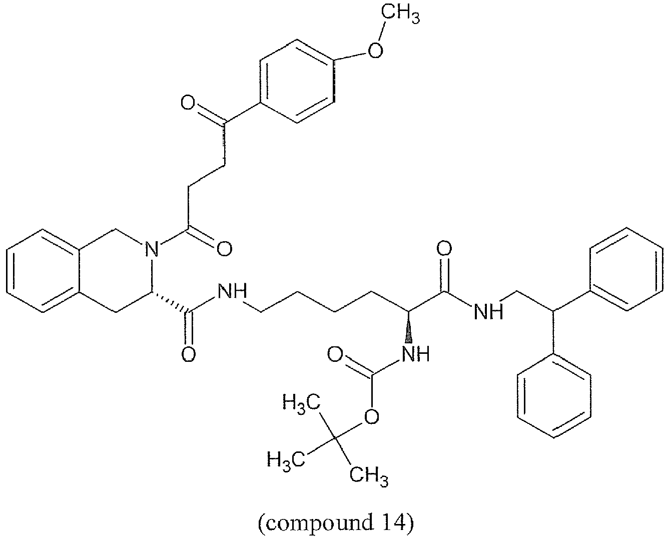 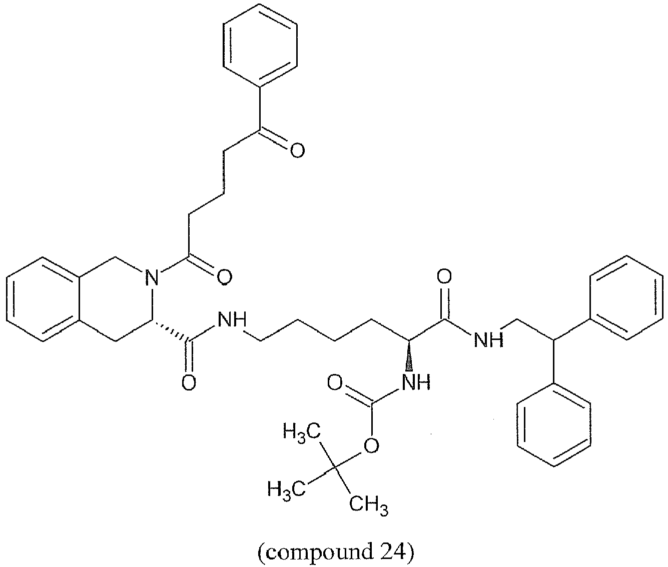 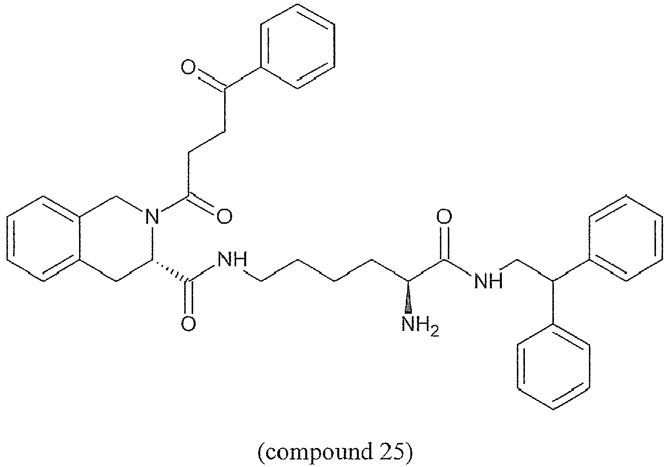 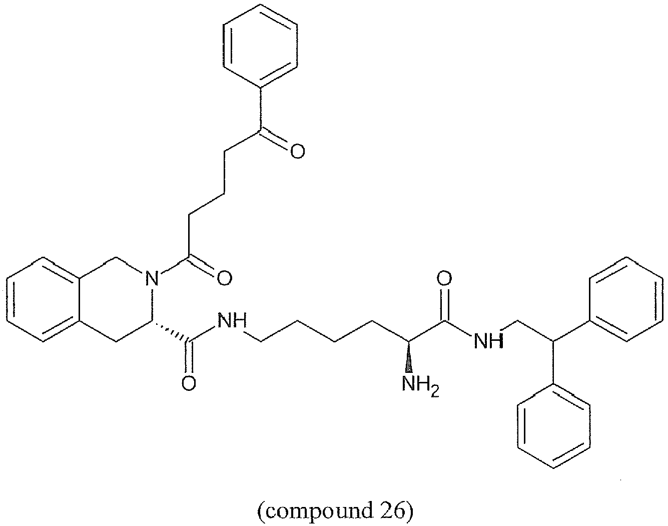 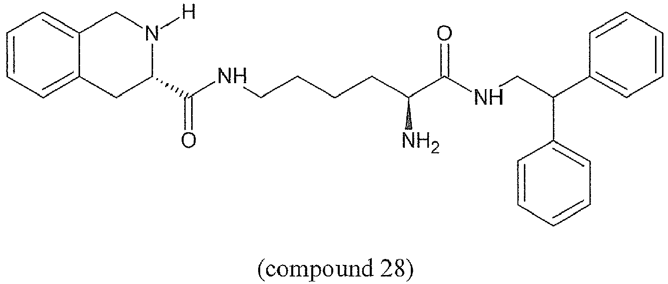 or epimers of said compound,
or a pharmaceutically acceptable salt thereof or of its epimers.

In a further aspect, the present invention also relates to a compound according to the present invention, for use as a pharmaceutical.

In a further aspect, the present invention relates to a compound according to the present invention for use in the treatment of an inflammatory disease, cancer, diabetes, transplant rejection, Alzheimer disease, HIV-induced dementia, ischemia, in particular cerebral ischemia, cardiac ischemia, stroke, myocardial infarction, or for use in the alleviation of wound healing.

In one embodiment, said inflammatory disease is a disease selected from rheumatoid arthritis, ankylosing spondylitis, inflammation post infection, atherosclerosis, myocardial infarction, stroke, pain, dermatitis, psoriasis, atopic skin disease, chronic granulomatous diseases such as tuberculosis, leprosy, sarcoidosis, silicosis, nephritis, amyloidosis, scleroderma, lupus, polymyositis, osteoporosis, sepsis, inflammatory bowel disease, ulcers, appendicitis, diverticulitis, Sjogren's syndrome, Reiter's syndrome, pelvic inflamnatory disease, orbital inflammatory disease, peritonitis, hepatitis , thrombotic disease, inappropriate allergic responses to environmental stimuli such as poison ivy, pollen, insect stings and certain foods, including atopic dermatitis and contact dermatitis, allergic encephalitis, asthma, lung inflammation, COPD, chronic bronchitis, multiple sclerosis,
and the cancer is selected from melanoma, osteosarcoma, prostate, colon and breast cancer and metastasis of the aforementioned cancers,
and the diabetes is selected from diabetes type 1.

In one embodiment, a suitable amount of said compound is administered to a patient in need thereof.

The present invention also relates to a method of treatment of an inflammatory disease, cancer, or diabetes, transplant rejection, Alzheimer disease, HIV-induced dementia, ischemia, in particular cerebral ischemia, cardiac ischemia, stroke, myocardial infarction, or a method of alleviation of wound healing, wherein said method comprises the administration of a suitable amount of a compound according to the present invention to a patient in need thereof.

Furthermore, the present invention relates to the use of a compound according to the present invention, as a chemical probe, in particular for elucidating functioning of the chemokine receptor CXCR3 and/or signalling pathway(s) associated therewith, wherein, preferably said use is in-vitro or in-vivo.

Furthermore, the present invention relates to a method of synthesis of a compound represented by formula (I) 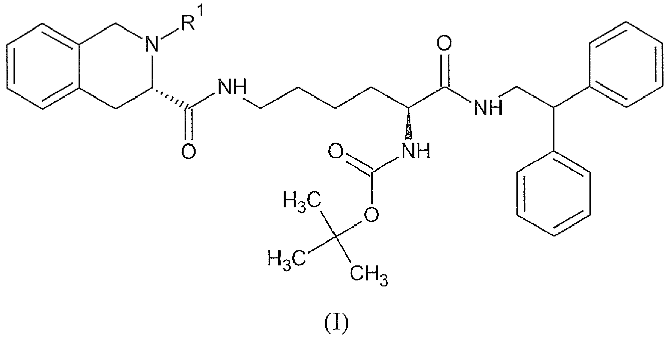 or of an epimer thereof,
wherein R¹ is H,
said method occurring in accordance with the following scheme and comprising the steps: 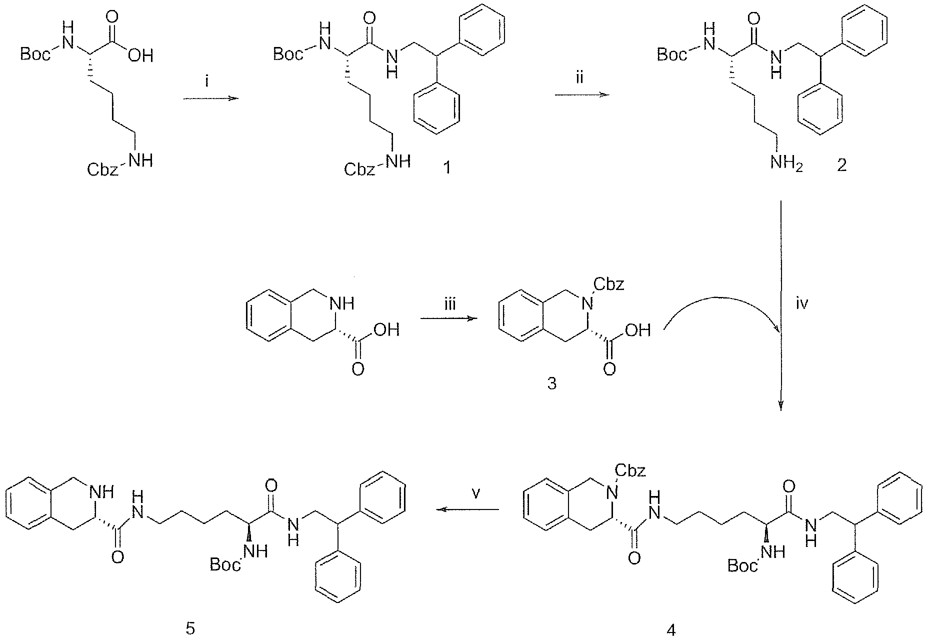
- acylation between Boc-Lys(Z)-OH and biphenylamine in the presence of 1-ethyl-3-(3-dimethylaminopropyl)carbodiimide (EDC) to yield compound 1,
- hydrogenolysis to afford primary amine 2,
- carboxybenzyl (Cbz) protection of S-2-((benzyloxy)carbonyl)-1,2,3,4-tetrahydroisoquinoline-3-carboxylic acid using benzyl chloroformate in basic medium to give compound 3,
- amide coupling of compound 3 and compound 2 to give compound 4,
- hydrogenolysis of compound 4 to yield compound 5.

Moreover, the present invention relates to a method of synthesis of a compound represented by formula (I) 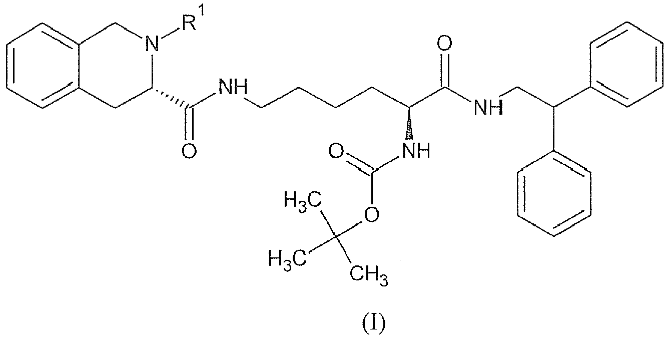 or of an epimer thereof,
wherein R¹ is a radical represented by formula II 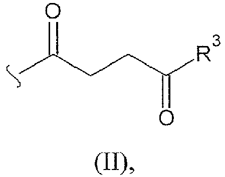 with R³ being methyl,
phenyl 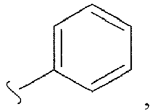 or 4-methoxy-phenyl 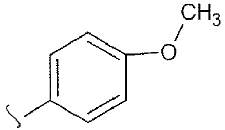 said method comprising the steps:
reacting compound 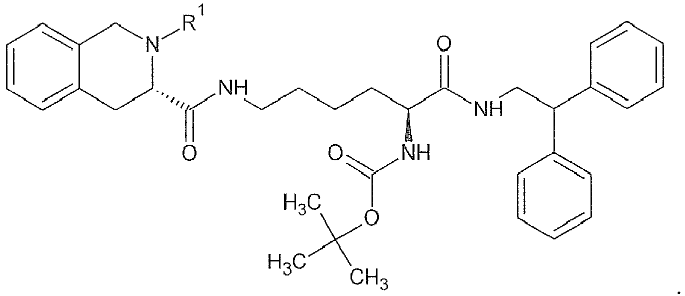 or an epimer thereof, wherein R¹ is H, with a carboxylic acid compound according to the scheme 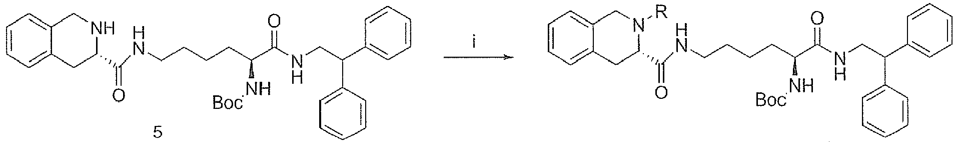 Wherein the carboxylic acid compound is 3-benzoylpropionic acid 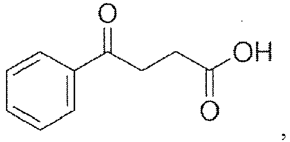 3-(4-methoxybenzoyl)propionic acid 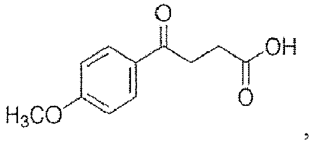 or 4-oxopentanoic acid 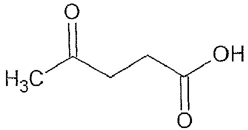 and wherein in the reaction product R is a radical represented by formula II 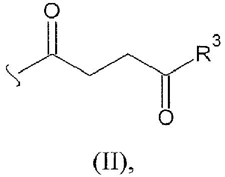 with R³ being methyl,
   phenyl 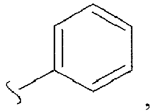 or 4-methoxy-phenyl

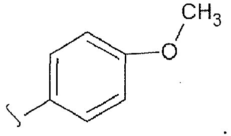

In a further aspect, the present invention also relates to the use of a compound according to the present invention for the manufacture of a medicament for the treatment of an inflammatory disease, cancer, diabetes, transplant rejection, Alzheimer disease, HIV-induced dementia, ischemia, in particular cerebral ischemia, cardiac ischemia, stroke, myocardial infarction, or for the manufacture of a medicament for the alleviation of wound healing. These diseases are as defined further above.

The present inventors have surprisingly found that it is possible to provide for allosteric agonists of the chemokine receptor CXCR3 the bias of which can be influenced. The inventors have provided strongly biased allosteric agonists of CXCR3 which selectively induce weak chemotaxis, receptor intermalisation and a β-arrestin 2 recruitment with potency comparable to the endogenous chemokine CXCL11 and an efficacy of more than 60 % without any activation of the G-protein signalling pathway. Inventors have also provided other allosteric agonists with a different bias wherein only G-proteins are activated and that induce a chemotaxis, but fail to induce receptor internalisation of β-arrestin 2 recruitment. Hence, it is possible to switch between the β-arrestin 2-patway and the G-protein-linked pathway. Furthermore, the inventors have also provided unbiased agonists of CXCR3.

It appears that the compound hereafter sometimes designated as "FAUC1036", also sometimes herein referred to as compound 11, activates the β2-arrestin recruitment but has no effect on the G-protein signalling. Similar results were obtained with the corresponding epimer thereof, which is herein sometimes also referred to as compound 34 or "FAUC1095". Furthermore, similar results were obtained with the unprotected compound 25 and its epimer, compound 35.

In the following, reference is made to the figures, wherein
Figure 1 shows previously reported CXCR3 agonists: tetrahydroisoquinoline VUF10661^{16,18} and PS372424¹⁶, pyrazinodiazepine¹⁶, and biaryl type ligands²².
Figure 2 shows an investigation of structural features for biased allosteric agonists based on the scaffold of FAUC1036.
Figure 3 shows that FAUC1036 causes the CXCR3 receptor desensitation and reduction of endogenous chemokine affinity. The CXCR3 expressing HEK293T cells were incubated with given concentrations of FAUC1036 and various concentrations of CXCL11 (Figure 3A) and CXCL10 (Figure 3B) and the β-arrestin 2 recruitment was measured.
Figure 4 shows internalization of CXCR3 upon treatment with FAUC1036. Figure 4A: In untreated cells, CXCR3 is mostly located at the cell surface (a). Stimulation with 10 nM endogenous ligand CXCL11 resulted in the internalization of CXCR3 (b).
   Treatment with FAUC1036 at concentrations 10 µM and 1 µM led to the internalization of a significant portion of the receptors (c, d). The scale bars in panel A-D) represent 20µm. Data shown is representative of at least three independent experiments.
   Figure 4B: Quantification of receptor internalization upon the treatment with the endogenous agonist CXCL11, FAUC1036 and FAUC1104, where only FAUC1036 and CXCL11 led to receptor internalization. Statistical analysis was performed with Student t test (**p <0.01).
Figure 5 shows that FAUC1036 and FAUC1104 cause a CXCR3-receptor mediated chemotaxis.
Figure 6 shows strongly biased allosteric agonists of CXCR3.
Figure 7 shows a tetrahydroisoquinoline scaffold based pharmacophore model depicting important structural elements that dictate biased agonism on allosteric agonists of CXCR3.

Reference is made to the following examples which are given to illustrate, not to limit the present invention.

### Examples

### Example 1

### Chemistry

### General methods

All reactions requiring anhydrous conditions were carried out under nitrogen and the glassware were dried in flame temperature under vaccum before use. All reactions were monitored by TLC using Kieselgel 60 F₂₅₄ plates. Visualization of the reaction components was achieved using UV fluorescence (254 nm) and KMnO₄ stain. Silica gel chromatography was carried out on Kieselgel 60. The yields are reported after purification. ¹H, ¹³C NMR spectra were recorded in deuterated solvents and chemical shifts (δ) are quoted in parts per million (ppm) calibrated to TMS (¹H and ¹³C δ = 0). Coupling constants (*J*) are measured in Hertz (Hz). The following abbreviations are used to describe multiplicities: s = singlet, d = doublet, dd = double doublet, dt = double triplet, t = triplet, q = quartet, br s = broad singlet, m = multiplet. Electrospray-ionization MS (ESI-MS) measurements were performed on a UHR-TOF Bruker Daltonik (Bremen, Germany) maXis plus 5G, an ESI-ToF MS capable of resolution of at least 60,000 FWHM. Detection was in positive ion mode, the source voltage was 2.8 kV. The flow rates were 180 µL/hour. The drying gas (N2), to aid solvent removal, was held at 180 °C and the spray gas was held at 20 °C. The machine was calibrated prior to every experiment via direct infusion of the Agilent ESI-TOF low concentration tuning mixture, which provided an m/z range of singly charged peaks up to 2700 Da in both ion modes. Purity and identity were assessed by analytical RP-HPLC (Agilent 1100 analytical series, column: Zorbax Eclipse XDB-C8 , 4.6 × 150 mm, 5 µm, flow rate: 0.5 ml/min, detection wavelength: 254 nm) coupled to a Bruker Esquire 2000 mass detector equipped with an ESI-ioniser. Purities of the products were assessed from HPLC-MS using MeOH / H₂O 0.1% HCOOH as the solvent system and employing the following gradient systems:

### LC-MS:

***Gradient A:*** 0-3 min: 10% MeOH, 3-18 min: 10-100% MeOH, 18-24 min: 100% MeOH, 24-30 min: 100-10% MeOH.

***Gradient B:*** 0-2 min: 10% MeOH, 2-10 min: 10-100% MeOH, 10-14 min: 100% MeOH, 14-16 min: 100-10% MeOH, 16-18 min: 10% MeOH.

### NMR performance at high temperature.

NMR analysis at high temperature was performed for compounds with multiple isomers, in order to avoid as much as possible multiple peaks due to isomerism. (or similar).

### Preparative HPLC

Preparative RP-HPLC was performed using Agilent 1100 preparative series. Column: Nucleodur C18 HTec, 32 × 250 mm, 5 µm particles, flow rate 32 mL /min, detection wavelengths: 210, 254 run. The solvent system, that was used for the preparative HPLC, was acetonitrile (ACN), water with 0.1% trifluoroacetic acid (TFA). The methods differ only in gradient.

### Method A

20-80% ACN in 0-20 min, 80-95% ACN in 20-22 min, 95-95% ACN in 22-25 min, 95-20% ACN in 25-30 min.

### Method B

30-70% ACN in 0-20 min, 70-95% ACN in 20-22 min, 95-95% ACN in 22-25 min, 95-30% ACN in 25-30 min.

### Method C

20-70% ACN in 0-20 min, 70-95% ACN in 20-22 min, 95-95% ACN in 22-25 min, 95-20% ACN in 25-30 min.

### Method D

40-90% ACN in 0-20 min, 90-95% ACN in 20-22 min, 95-95% ACN in 22-25 min, 95-40% ACN in 25-30 min.

### Method E

20-60% ACN in 0-20 min, 60-95% ACN in 20-22 min, 95-95% ACN in 22-25 min, 95-20% ACN in 25-30 min.

### General procedure 1 (GP1) for amide couplings: Representative experimental procedure for the amide formation using primary amines.

To solution of acid (1 eq.) in anhydrous DCM was added N-(3-Dimethylaminopropyl)-N'-ethylcarbodiimide (EDC) hydrochloride (1.1 eq.) at 0 °C and stirred for 1h. The primary amine (1.1 eq.) was added to the reaction mixture, and then it was warmed up to room temperature and stirred for 14-18 h. A saturated solution of NaHCO₃ was added and the aqueous phase was extracted with DCM (3 times). The combined organic extracts were washed with brine, dried over Na₂SO₄, filtered and evaporated *in vacuo.* The crude mixture was purified by flash column chromatography to afford the compounds **1, 4, 30, 32, 36, 37, 40, 41.**

### General procedure 2 (GP2) for amide couplings: Representative experimental procedure for the amide formation using secondary amines.

To a solution of acid (1.1 eq.) in anhydrous DCM were added *N*'-(3-Dimethylaminopropyl)-*N*'-ethylcarbodiimide (EDC) hydrochloride (1.1 eq.) and *N,N*-diisopropylethylamine (1.5 eq.) at 0 °C. The reaction mixture stirred for 30 min. The secondary amine (1 eq) was added, then the reaction mixture warmed up to room temperature and stirred for 16-20 h. A saturated solution of NaHCO₃ was added and the aqueous phase was extracted with DCM (3 times). The combined organic extracts were washed with brine, dried over Na₂SO₄, filtered and evaporated *in vacuo.* The crude mixture was purified by flash column chromatography to afford the compounds **11, 13-22, 24, 34, 35, 44, 45.**

### General procedure 3 (GP3) for hydrogenation: Representative experimental procedure for the Cbz deprotection.

The protected amine (1 eq.) was dissolved in anhydrous methanol and the solution was transferred to a dry schlenk flask containg Pd/C (0.2 eq.) under counter current at room temperature. The reaction mixture stirred for 6 h under H₂ atmosphere at room temperature. Then the catalyst was removed by filtration (celite pad) and the solvent was evaporated in *vacuo.* To afford the final compounds **2, 5, 31, 33, 28, 38, 39, 42, 43.** No further purification was required.

### General procedure 4 (GP4) for Boc deprotection: Representative experimental procedure for the TFA deprotection.

The Boc protected amine was added in a solution of DCM/TFA (4:1). The reaction mixture stirred for 3 h at room temperature. Then the reaction mixture evaporated *in vacuo.* The precipitate was neutralized with a saturated solution of NaHCO₃ and the aqueous phase was extracted with DCM (3 times). The combined organic extracts were washed with brine, dried over Na₂SO₄, filtered and evaporated *in vacuo.* The crude mixture was purified by preparative HPLC to afford the compounds **25, 26, 28, 36, 46, 47** as TFA salts.

### General procedure 5 (GP5) for Acylation: Representative experimental procedure for the Acylation using heterocycles with the anhydride.

To solution of succinic anhydride (1 eq.) in anhydrous DCM was added the heterocycle base (1 eq) and the reaction mixture was stirred for 2 h at rt. The DCM was evaporated and the crude compound was recrystallized from Hex/EtOAc to afford the compounds **6, 7, 8.**

### General procedure 6 (GP6) for Friedel Crafts reaction: Representative experimental procedure for the Friedel Crafts using heterocycles with the anhydride.

To solution of succinic anhydride (1 eq.) in anhydrous DCM was added anhydrous AlCl₃ (1.2 eq) at 0 C and the reaction mixture was stirred for 2 h at 0 °C. Then the aromatic heterocycle was added dropwise over a period of 1 h. The reaction mixture was stirred for 4 h at room temperature. 50 ml of ice-cold H₂O was added at 0 °C, followed by acidification with 2N HCl to pH 2. The aqueous phase was extracted with DCM (3 times). The combined organic extracts were dried over Na₂SO₄, filtered and evaporated *in vacuo.* The crude mixture was purified by recrystallization or flash column chromatography to afford the compounds **9, 10.**

### Benzyl tert-butyl (6-((2,2-diphenylethyl)amino)-6-oxohexane-1,5-diyl)(S)-dicarbamate (1)

Compound **1** was prepared according to general procedure 1 **(GP1).** The crude mixture was purified by flash column chromatography (DCM/MeOH: 99/1) to afford compound **1** (2.37 g, 4.24 mmol, 80%) as a white solid. ¹H-NMR (360 MHz, CDCl₃): δ (ppm) 7.28-7.14 (m, 5H), 5.94 (s, 1H), 5.01 (s, 2H), 4.92 (s, 1H), 4.71 (s, 1H), 4.10 (t, *J* = 8.0 Hz, 1H), 3.90-3.82 (m, 3H), 3.05-3.02 (m, 2H), 1.63-1.56 (m, 1H), 1.38-1.13 (m, 14H). ¹³C-NMR (150 MHz, CDCl₃): δ (ppm) 172.2 (CO), 156.8 (CO), 155.9 (CO), 142.0 (2 x CH), 136.9 (CH), 129.1 (4 x CH), 128.9 (2 x CH), 128.5 (3 x CH), 128.4 (4 x CH), 127.2 (2 x CH), 80.3 (C_{q}), 67.0 (CH₂), 54.6 (CH), 50.9 (CH), 44.0 (CH₂), 40.7 (CH₂), 32,3 (CH₂), 29.7 (CH₂), 28.6 (3 x CH₃), 22.6 (CH₂). MS (ESI) m/z (%): 307 (24), 263(10), 218 (15), 180 (35), 174 (13), 167 (29), 108 (10), 91 (100), 84 (19), 79 (13), 59 (12), 57 (43.4), 56 (14), 44 (12), 41 (31), 39 (12), 30 (26), 28 (11). MS (ESI) m/z calcd for C₃₃H₄₁N₃O₅ [M]⁺, 559.30; found, 559.31. LC-MS (ESI) m/z calcd. for C₃₃H₄₂N₃O₅Na [M+Na+H]²⁺, 583.3; found, 583.2. HPLC purity, 98%, t_{R} = 22.3 min, gradient A.

### Tert-butyl (S)-(6-amino-7-((2,2-dipheny/ethyl)amino)-1-oxohexan-2-y/)carbamate (2)

Compound 2 was prepared according to general procedure 3 **(GP3).** Afforded compound **2** (1.82 g, 4.28 mmol, 99%) as a white solid. ¹H-NMR (600 MHz, CDCl₃): δ (ppm) 7.23-7.17 (m, 4H), 7.16-7.13 (m, 6H), 6.29 (s, 1H), 4.96 (s, 1H), 4.11 (t, *J* = 8.0 Hz, 1H), 3.91-3.75 (m, 3H), 2.59-2.53 (m, 2H), 1.65-1.61 (m, 1H + H₂O), 1.40-1.27 (m, 12H), 1.18-1.14 (m, 2H). ¹³C-NMR (150 MHz, CDCl₃): δ (ppm) 172.3 (CO), 155.9 (CO), 142.1 (2 x Cq), 129.1 (4 x CH), 128.4 (4 x CH), 127.2 (2 x CH), 80.2 (Cq), 54.61 (CH), 51.0 (CH), 44.1 (CH₂), 41.8 (CH₂), 32.9 (2 x CH₂), 28.7 (3 x CH₃), 22.8 (CH₂). MS (ESI) m/z (%): (C₂₅H₃₅N₃O₃)425 (12), 258 (14), 202 (18), 181 (11), 180 (25), 173 (14), 167 (27), 165 (18), 145 (13), 139 (15), 129 (12), 84 (100), 82 (18), 57 (50), 56 (18), 41 (26), 30 (36). MS (ESI) m/z calcd for C₂₅H₃₆N₃O₃ [M+H]⁺, 426.27; found, 426.27. LC-MS (ESI) m/z calcd. for C₂₅H₃₆N₃O₃ [M+H]⁺, 426.3; found, 426.3. HPLC purity, 95%, t_{R} = 11.0 min, gradient B.

### (S)-2-((benzyloxy)carbonyl)-1,2,3,4-tetrahydroisoquinoline-3-carboxylic acid (3)

To a 1 M aqueous solution of NaOH (3.20 g, 80.0 mmol in 80 mL of H₂O) at rt were added (S)-1,2,3,4-tetrahydroisoquinoline-3-carboxylic acid (5.00 g, 28.2) and dioxane slowly until the suspension dissolves. Then benzylchloroformate was added (6.25 g, 5.59 µL, 36.7 mmol) dropwise over a period of 30 min at 0 °C. The reaction mixture was stirred for 16 h at room temperature. Dioxane was removed by evaporation and reaction mixture was acidified to pH 2 with 1 M HCl at 0 °C. The aqueous layer was extracted with EtOAc. The combined organic layers were dried over Na₂SO₄, filtered and evaporated *in vacuo* to afford the title compound 3 (8.68, 27.9 mmol, 99%) as a white foam. No further purification was required. ¹H-NMR (360 MHz, CDCl₃): δ (ppm) 7.32-6.99 (m, 9H), 5.19-5.10 (m) and 4.91 (t, *J =* 4.9 Hz, 3H), 4.70 (d, *J* = 16.3 Hz), 4.50 (m, 1H), 3.22-3.07(m, 2H). ¹³C-NMR (150 MHz, CDCl₃): δ (ppm) 175.4 (CO, rotamer 2) and 175.0 (CO, rotamer 1), 155.4 (CO, rotamer 1) and 154.4 (CO, rotamer 2), 135.2 (Cq), 131.9 (Cq), 131.2 (Cq), 130.4 (CH, rotamer 1) and 130.3 (CH, rotamer 2), 127.5 (CH), 127.2 (CH), 127.1 (CH), 127.0 (CH, rotamer 1) and 126.9 (CH, rotamer 2), 126.1 (CH), 126.0 (CH), 125.4 (CH), 125.2 (CH), 66.8 (CH₂, rotamer 1) and 66.6 (CH₂, rotamer 2), 52.4 (CH, rotamer 2) and 52.0 (CH, rotamer 1), 43.5 (CH₂, rotamer 2) and 43.3 (CH₂, rotamer 2), 30.2 (CH₂, rotamer 2) and 29.8 (CH₂, rotamer 1). Ratio of rotamers is 3:2. MS (ESI) m/z (%): 222 (14), (C₁₁H₁₀NO₄) 200(11), (C₁₀H₁₀NO₂) 176 (53), 130 (23), 104 (11), (C₇H₇) 91 (100). MS (ESI) m/z calcd for C₁₈H₁₇NO₄[M]⁺, 311.12; found, 311.12. LC-MS (ESI) m/z calcd. for C₁₈H₁₇NO₄Na [M+Na]⁺, 334.1; found, 334.6. HPLC purity, 96%, t_{R} = 20.5 min, Gradient A.

### Benzyl (S)-3-(((S)-5-((tert-butoxycarbonyl)amino)-6-((2,2-diphenylethyl)amino)-6-oxohexyl) carbamoyl)-3,4-dihydroisoquinoline-2(1H)-carboxylate (4)

Compound **4** was prepared according to general procedure 1 **(GP1).** The crude solid mixture was purified by flash column chromatography (DCM/MeOH: 97/3) to afford compound **4** (1.2 g, 1.8 mmol, 51%) as a white solid. ¹H-NMR (600 MHz, DMSO-*d*₆, 400 K): δ (ppm) 7.34-7.11 (m, 19H), 5.96 (br s, 1H), 5.15-5.10 (m, 2H), 4.60-4.65 (m, 2H), 4.50 (d, *J* = 15.8 Hz, 1H), 4.20 (t, *J =* 8.0 Hz, 1H), 3.77-3.74 (m, 2H), 3.69-3.64 (m, 1H), 3.08 (d, *J* = 5.5 Hz, 2H), 2.90-2.86 (m, 2H), 1.44-1.42 (m, 1H), 1.26-1.23 (m, 10H), 1.20-1.13 (2H), 1.05-0.99 (2H). ¹³C-NMR (150 MHz, CDCl₃): δ (ppm) 171.0 (2 x CO), 155.9 (2 x CO), 140.8 (C_{q}), 135.1 (Cq), 132.7 (Cq), 127.8 (4 x CH), 127.7 (CH), 127.4 (2 x CH), 127.0 (6 x CH), 125.8 (CH), 78.9 (Cq), 66.7 (CH), 55.2 (CH) 53.2 (CH), 49.5 (CH₂), 44.2(CH₂), 42.6 (CH₂), 37.5 (CH₂), 31.0 (2 x CH₂), 27.8 (CH₂), 27.3 (3 x CH₃), 21.1 (CH₂). LC-MS (ESI) m/z calcd. for C₄₃H₅₂N₄O₆ [M+2H]²⁺, 720.4; found, 720.2. HPLC purity, 96%, t_{R} = 22.2 min, gradient A.

### Tert-butyl ((S)-1-((2,2-diphenylethyl)amino)-1-oxo-6-((S)-1,2,3,4-tetrahydroisoquinoline-3-carboxamido)hexan-2-yl)carbamate (5)

Compound 5 was prepared according to general procedure 3 (**GP3**). Compound **5** (2.0 g, 3.4 mmol, 98%) was afforded as a white solid. ¹H-NMR (360 MHz, CDCl₃): δ (ppm) 7.24-7.07 (m, 15H + CDCl₃), 6.98-6.96 (m, 1H), 6.08 (t, *J* = 5.0 Hz, 1H), 4.97 (s, 1H), 4.12 (t, *J =* 8.0 Hz, 1H), 3.93-3.75 (m, 5H), 3.46 (dd, *J* = 10.6 Hz, 1H), 3.17-3.12 (m, 3H), 2.74 (dd, *J =* 5.6 Hz, *J=* 10.4 Hz, *J=* 16.3 Hz, 1H), 1.59 (m, 1H), 1.41-1.35 (m, 3H), 1.31 (s, 9H), 1.20-1.12 (m, 2H). ¹³C-NMR (150 MHz, CDCl₃): δ (ppm) 172.1 (CO), 170.9 (CO), 155.9 (CO), 140.7 (2 x Cq), 134.8(Cq), 133.3 (C_{q}), 128.1 (CH), 127.7 (4 x CH), 127.0 (4 x CH), 125.8 (2 x CH), 125.6 (CH), 125.1 (CH), 124.5 (CH), 78.9 (Cq), 55.5 (CH), 53.3 (CH), 49.6 (CH₂), 46.4 (CH), 42.7 (CH₂), 37.2 (CH₂), 30.8 (CH₂), 30.0 (CH₂), 28.0 (CH₂), 27.2 (3 x CH₃), 21.4 (CH₂). HRMS (ESI) m/z calcd for C₃₅H₄₄N₄O₄ + H⁺ [M+H]⁺, 585.3435; found, 585.3436. MS (ESI) m/z (%): (C₉H₁₁N) 133 (14), (C₉H₁₀N) 132 (100), 131 (20), 130 (11), 84 (11), 59 (32), 57 (12), 41 (17). MS (ESI) m/z calcd for C₃ₛH₄₄N₄O₄ [M]⁺, 584.34; found, 584.34. LC-MS (ESI) m/z calcd. for C₃₅H₄₄N₄O₄ + 2H⁺ [M+2H]⁺, 586.3; found, 586.1. HPLC purity, 97%, t_{R} = 19.5 min, gradient A.

### 4-oxo-4-(piperidin-1-yl)butanoic acid (6)²⁵

Compound 6 was prepared according to general procedure 5 **(GP5).** The crude solid mixture was purified by recrystallization (Hex/EtOAc: 1/2) to afford compound **6** (2.6 g, mmol, 94%) as a white solid. ¹H-NMR (600 MHz, CDCl₃): δ (ppm) 3.50 (t, *J* = 6.0 Hz, 2H), 3.36 (t, *J=* 6.0 Hz, 2H), 2.62-2.61 (m, 4H), 1.60-1.48 (m, 6H). ¹³C-NMR (90 MHz, CDCl₃): δ (ppm) 176.4 (CO), 170.7 (CO), 46.0 (CH₂), 43.4 (CH₂), 30.4 (CH₂), 28.5 (CH₂), 26.6 (CH₂), 25.8 (CH₂), 24.7 (CH₂).

### 4-moyholino-4-oxobutanoic acid (7)³⁴

Compound 7 was prepared according to general procedure 5 **(GP5).** The crude solid mixture was purified by recrystallization (Hex/EtOAc: 1/3) to afford compound 7 (2.05 g, mmol, 95%) as light yellow crystals. ¹H-NMR (360 MHz, CDCl₃): δ (ppm) 3.64-56 (m, 6H), 3.44-3.42 (m, 2H), 2.67-2.62 (m, 2H), 2.59-2.55 (m, 2H). ¹³C-NMR (90 MHz, CDCl₃): δ (ppm) 177.0 (CO), 170.5 (CO), 66.9 (CH₂), 66.6 (CH₂), 46.0 (CH₂), 42.4 (CH₂), 29.4 (CH₂), 27.9 (CH₂).

### 4-(4-methylpiperazin-1-yl)-4-oxobutanoic acid (8)^{25x}

Compound **8** was prepared according to general procedure 5 **(GP5).** The crude product was purified by recrystallization (Hex/EtOAc/EtOH: 2/4/1) to afford the titled compound **8** (1.71 g, 8.54 mmol, 86%) as yellowish solid. ¹H-NMR (360 MHz, CDCl₃): δ (ppm) 10.69 (br s, 1H), 3.62 (t, *J* = 6.0 Hz, 2H), 3.52 (t, *J=* 6.0 Hz, 2H), 2.57-2.44 (m, 8H), 2.30 (s, 3H). ¹³C-NMR (90 MHz, CDCl₃): δ (ppm) 175.2 (CO), 169.5 (CO), 66.9 (CH₂), 53.2 (CH₂), 52.3 (CH₂), 44.0 (CH₃), 43.4 (CH₂), 39.8 (CH₂), 28.9 (CH₂), 27.2 (CH₂).

### 4-oxo-4-(thiophen-2-yl)butanoic acid (9)²⁷

Compound **9** was prepared according to general procedure 9 **(GP6).** The crude product was purified by recrystallization (H₂O) to afford compound **9** (1.38 mg, 7.5 mmol), 63%) as a light yellow solid. ¹H-NMR (600 MHz, CDCl₃): δ (ppm) 7.70 (dd, *J=* 1.1, *J=* 3.8, 1H), 7.58 (dd, *J* = 1.1, *J* = 4.9, 1H), 7.07 (dd, *J =* 3.8, *J =* 4.9, 1H), 3.19 (t, *J* = 6.8 Hz, 2H), 2.74 (t, *J =* 6.7 Hz, 2H). ¹³C-NMR (150 MHz, CDCl₃): δ (ppm) 190.9 (CO), 178.5 (CO), 143.7 (Cq), 134.0 (CH), 132.3 (CH), 128.4 (CH), 33.9 (CH), 28.2 (CH).

### 4-(furan-2-yl)-4-oxobutanoic acid (10)²⁸

Compound **10** was prepared according to general procedure 6 **(GP6).** The crude product was purified by flash column chromatography (DCM/MeOH/NH₃: 96/4/0.1) to afford compound **10** (263 mg, 1.6 mmol, 57%) as a light yellow solid. ¹H-NMR (600 MHz, CDCl₃): δ (ppm) 7.52 (dd, *J =* 0.8, *J =* 1.7, 1H), 7.16 (dd, *J =* 0.8, J = 3.6, 1H), 6.48 (dd, *J =* 1.7, *J =* 3.6, 1H), 3.11 (t, *J =* 6,7 Hz, 2H), 2.73 (t, *J* = 6.7 Hz, 2H). ¹³C-NMR (150 MHz, CDCl₃): δ (ppm) 187.2 (CO), 178.3 (CO), 152.5 (Cq), 146.6 (CH), 117.3 (CH), 112.5 (CH), 33.0 (CH), 27.7 (CH).

### Tert-butyl ((S)-1-((2,2-diphenylethyl)amino)-1-oxo-6-((S)-2-(4-oxo-4-phenylbutanoyl)-1,2,3,4-tertahydroisoquinoline-3-carboxamido)hexan-2-yl)carbamate (11/FAUC1036)

FAUC1036 was prepared according to general procedure 2 **(GP2)** by using benzoyl propionic acid. The crude solid mixture was purified by flash column chromatography (Hex/EtOAc/EtOH: 8/3/1) to afford FAUC1036 (89 mg, 0.12 mmol, 70%) as a white solid. ¹H-NMR (600 MHz, DMSO-*d₆*, 400 K): (ppm) 7.94-7.92 (m, 2H), 7.63-7.58 (m, 1H), 7.50-7.47 (m, 2H), 7.24-7.13 (m, 15H), 5.96 (br s, 1H), 4.91 (br s, 1H), 4.80 (d, *J =* 15.9 Hz, 1H), 4.57 (br s, 1H), 4.19 (t, *J* = 7.7 Hz, 1H), 3.76-3.68 (m, 2H), 3.66-3.64 (m, 2H), 3.27 (t, *J* = 6.4 Hz, 2H), 3.18 (dd, *J =* 4.0 Hz, *J* = 15.7 Hz, 1H), 3.06 (br d, 1H), 2.96-2.75 (m, 4H + H₂O), 1.44-1.39 (m, 1H), 1.38 (s, 9H), 1.36-1.29 (m, 1H), 1.23-1.16 (m, 2H), 1.10-1.00 (m, 2H). ¹³C-NMR (150 MHz, DMSO-*d*₆, 350 K): δ (ppm) 172.5 (CO), 143.3 (2 x C_{q}), 137.6 (Cq), 133.4 (Cq), 129.1 (2 x CH), 128.8 (4 x CH), 128.4 (4 x CH), 128.3 (CH), 126.8 (2 CH), 126.5 (CH), 78.6 (C_{q}), 50.7 (CH), 43.8 (CH₂), 34.0 (CH₂), 32.3 (CH₂), 29.2 (CH₂), 28.7 (3 x CH₃), 28.2 (CH₂), 23.0 (CH₂). One Cq, two CH, two aromatic CH and three CH₂ carbons were not detected due to peak broadening caused by isomerism. The structure is verified by performing 2D NMR analysis (COSY and HSQC). HRMS (ESI) m/z calcd for C₄₅H₅₂N₄O₆ Na [M+Na]⁺, 767.3779; found, 767.3789. LC-MS (ESI) m/z calcd for C₄₅H₅₄N₄O₁ [M+2H]²⁺, 746.4; found, 746.4. HPLC purity, 100%, t_{R} = 22.7min, gradient A.

### Tert-butyl((S)-1-((2,2-diphenylethyl)amino)-1-oxo-6-((S)-2-(4-oxopentanoyl)-1,2,3,4-tetrahydroisoquinoline-3-carboxamido)hexan-2-yl)carbamate (12)

Compound **12** was prepared according to general procedure 2 **(GP2)** by using levulinic acid. The crude solid mixture was purified by flash column chromatography (Hex/EtOAc/EtOH: 6/3/1) to afford compound **12** (45 mg, 0.066 mmol, 78%) as a white solid. ¹H-NMR (600 MHz, DMSO-*d₆*, 350 K): δ (ppm) 7.33-7.19 (m, 15H), 6.02 (br d, *J =* 8.0 Hz, 1H), 4.93 (br s, 1H), 4.80 (d, *J =* 16.0 Hz, 1H), 4.60 (br s, 1H), 4.26 (t, *J =* 8.0 Hz, 1H), 3.84-3.79 (m, 2H), 3.75-3.71 (m, 1H), 3.27-3.16 (m, 2H), 3.08-2.96 (m, 2H), 2.76-2.57 (m, 4H), 2.16 (s, 3H), 1.51-1.41 (m, 10H), 1.37-1.30 (m, 1H), 1.27-1.21 (m, 2H), 1.11-1.04 (m, 2H). ¹³C-NMR (150 MHz, DMSO-*d₆*, 350 K): δ (ppm) 172.4 (CO), 170.5 (CO), 167.9 (CO), 157.3 (CO), 143.2 (2 x Cq), 128.8 (2 x CH), 128.7 (2 x CH), 128.3 (4 x CH), 126.7 (4 x CH), 78.5 (Cq), 50.6 (CH), 50.0 (CH), 47.9 (CH), 43.7 (CH₂), 38.3 (CH₂), 32.2 (CH₂), 30.4 (CH₂), 29.1 (CH₃), 28.7 (3 x CH₃), 22.8 (CH₂). Two Cq, one aromatic CH and four CH₂ carbons were not detected due to peak broadening caused by isomerism. HRMS (ESI) m/z calcd for C₄₀H₅₀N₄O₆Na [M+Na]⁺, 705.3623; found, 705.3625. LC-MS (ESI) m/z calcd. for C₄₀H₅₁N₄O₆ [M+H]⁺, 683.4; found, 683.7. HPLC purity, 95%, t_{R} = 12.9 min, gradient B.

### Tert-butyl ((S)-1-((2,2-diphenylethyl)amino)-6-((S)-2-(4-(4-fluorophenyl)-4-oxobutanoyl)-1,2,3,4-tetrahydroisoquinoline-3-carboxamido)-1-oxohexan-2-yl)carbamate (13)

Compound **13** was prepared according to general procedure 2 **(GP2)** by using 3-(4-fluorobenzoyl)propionic acid. The crude solid mixture was purified by flash column chromatography (Hex/EtOAc/EtOH: 6/3/1) to afford compound **13** (39 mg, 0.05 mmol, 60%) as a white solid. ¹H-NMR (600 MHz, DMSO-*d*₆, 400 K): δ (ppm) 8.09 -8.07 (m, 2H), 7.29-7.20 (m, 15H), 6.03 (br s, 1H), 4.97 (br s, 1H), 4.86 (d, *J =* 15.9 Hz, 1H), 4.64 (br s, 1H), 4.26 (t, *J =* 7.7 Hz, 1H), 3.84-3.80 (m, 2H), 3.72 (m, 1H), 3.33 (t, *J* = 7.2 Hz, 2H), 3.25 (dd, *J =* 4.0 Hz, *J* =15.7 Hz, 1H), 3.12 (br s, 1H), 2.95-2.88 (m, 2H), 1.44-1.31 (m, 11H), 1.29-1.20 (m, 2H), 1.11-1.05(m, 2H). ¹³C-NMR (150 MHz, DMSO-*d*₆, 350 K): δ (ppm) 158.9 (CO) 143.3 (2 x Cq), 140.0 (Cq), 131.3 (2 x Cq), 128.1 (CH), 128.8 (4 x CH), 128.4 (4 x CH), 126.8 (CH), 126.5 (CH), 55.1 (CH), 50.7 (CH), 43.8 (CH₂), 33.9 (CH₂), 32.3 (CH₂), 31.6 (CH₂), 29.2 (CH₂), 29.0 (CH₂), 28.7 (3 x CH₃), 23.0 (CH₂). One CH, seven aromatic CH and two CH₂ carbons were not detected due to peak broadening caused by isomerism. HRMS (ESI) m/z calcd for C₄₅H₅₃FN₄O₆ [M+H]⁺, 763.3865; found, 763.3865. LC-MS (ESI) m/z calcd for C₄₅H₅₄FN₄O₆ [M+2H]²⁺, 764.4; found, 764.3. HPLC purity, 99%, t_{R} = 21.8 min, gradient A.

### Tert-butyl ((S)-1-((2,2-diphenylethyl)amino)-6-((S)-2-(4-(4-methoxyphenyl)-4-oxobutanoyl)-1,2,3,4-tetrahydroisoquinoline-3-carboxamido)-1-oxohexan-2-yl)carbomate (14/FAUC1104)

**14**/FAUC1104 was prepared according to general procedure 2 **(GP2)** by using 3-(4-methoxybenzoyl)propionic acid. The crude solid mixture was purified by flash column chromatography (Hex/EtOAc/EtOH: 6/3/1) to afford 14/FAUC1104 (39 mg, 0.05 mmol, 58%) as a white solid. ¹H-NMR (600 MHz, DMSO-*d₆*, 400 K): δ (ppm) 7.95-7.94 (m, 2H), 7.28-7.16 (m, 15H), 7.04-7.02 (m, 2H), 5.98 (br s, 1H), 4.94 (br s, 1H), 4.83 (d, *J =* 16.0 Hz, 1H), 4.60 (br s, 1H), 4.22 (t, *J* = 8.0 Hz, 1H), 3.87 (s, 3H), 3.80-3.77 (m, 2H), 3.71-3.68 (m, 1H), 3.25 (t, *J =* 7.2 Hz, 2H), 3.21 (dd, *J* = 4.0 Hz, *J* = 15.7 Hz, 1H), 3.08 (br s, 1H), 2.94-2.75 (m, 4H + H₂O), 1.44-1.27 (m, 11H), 1.26-1.19 (m, 2H), 1.08-1.01(m, 2H). ¹³C-NMR (150 MHz, DMSO-*d*₆, 350 K): δ (ppm) 171.6 (CO), 169.6 (CO), 162.8 (CO), 154.6 (CO), 142.3 (2 x C_{q}), 133.0 (C_{q}), 129.7 (2 x CH), 129.5 (CH), 127.9 (4 x CH), 127.5 (4 x CH), 125.8 (3 x CH), 125.6 (CH), 113.6 (2 x CH), 77.7 (C_{q}), 55.1 (CH₃), 49.8 (CH), 42.8 (CH₂), 32.7 (CH₂), 31.4 (CH₂), 28.2 (CH₂), 27.8 (3 x CH₃), 27.7 (CH₂), 22.0 (CH₂).Two Cq, two CH and three CH₂ carbons were not detected due to peak broadening caused by isomerism. HRMS (ESI) m/z calcd for C₄₆H₅₄N₄O₇Na [M+Na]⁺, 797.3885; found, 797.3888. LC-MS (ESI) m/z calcd for C₄₆H₅₆N₄O₇ [M+2H]²⁺, 776.4; found, 776.6. HPLC purity, 99%, t_{R} = 21.8 min, gradient A.

### Tert-butyl ((S)-1-((2,2-diphenylethyl)amino)-6-((S)-2-(4-(4-(methylsulfonyl)phenyl)-4-oxobutanoyl)-1,2,3,4-tetrahydroisoquinoline-3-carboxamido)-1-oxohexan-2-yl)carbamate (15)

Compound **15** was prepared according to general procedure 2 **(GP2)** by using 3-(4-methylsulfonylbenzoyl)propionic acid. The crude solid mixture was purified by flash column chromatography (Hex/EtOAc/EtOH: 3/3/1) to afford compound **15** (56 mg, 0.068 mmol, 80%) as a white solid. ¹H-NMR (600 MHz, DMSO-*d*₆, 400 K): δ (ppm) 8.21-8.19 (m, 2H), 8.11-8.09 (m, 2H), 7.29-7.06 (m, 15H), 6.03-6.02 (br s, 1H), 4.96 (br s, 1H), 4.86 (d, *J=* 16.0 Hz, 1H), 4.64 (br s, 1H), 4.26 (t, *J =* 8.0 Hz, 1H), 3.86-3.81 (m, 2H), 3.74-3.71 (m, 1H), 3.38 (t, *J* = 7.1 Hz, 2H), 3.23 (s, 3H), 3.10 (br s, 1H), 3.08 (br s, 1H), 2.98-2.86 (m, 4H + H₂O), 1.40-1.22 (m, 13H), 1.11-1.06 (m, 2H). ¹³C-NMR (150 MHz, DMSO-*d₆*, 350 K): δ (ppm) 199.3 (CO), 172.5 (CO), 171.6 (CO), 155.6 (CO), 145.0 (C_{q}), 143.3 (2 x C_{q}), 141.3 (C_{q}), 129.3 (2 x CH), 128.9 (2 x CH), 128.8 (2 x CH), 128.4 (4 x CH), 127.8 (2 x CH), 126.8 (CH), 126.5 (CH), 126.3 (CH), 126.1 (CH), 78.7 (Cq), 56.7 (CH), 55.1 (CH), 50.7 (CH₂), 47.3 (CH), 44.0 (CH₃) 43.8 (CH₂), 38.8 (CH₂), 34.4 (CH₂), 32.4 (CH₂), 31.7 (CH₂), 29.3 (CH₂), 28.8 (3 x CH₃), 28.4 (CH₂), 23.1 (CH₂). Two Cq were not detected due to peak broadening caused by isomerism. HRMS (ESI) m/z calcd for C₄₆H₅₄N₄O₈SNa [M+Na]⁺, 845.3555; found, 845.3557. LC-MS (ESI) m/z calcd. for C₄₆H₅₅N₄O₈S [M+H]⁺, 824.0; found, 824.4. HPLC purity, 96%, t_{R} = 21.0 min, gradient A.

### Tert-butyl ((S)-1-((2,2-diphenylethyl)amino)-1-oxo-6-((S)-2-(4-oxo-4-(piperidin-1-yl)butanoyl)-1,2,3,4-tetrahydroisoquinoline-3-carboxamido)hexan-2-yl)carbamate (16)

Compound **16** was prepared according to general procedure 2 **(GP2)** by using compound **6.** The crude product was purified by flash column chromatography (DCM/MeOH: 97/3) to afford compound **16** (58 mg, 0.077 mmol, 91%) as a white solid. ¹H-NMR (600 MHz, DMSO-*d*₆, 400 K): δ (ppm) 7.26-7.12 (m, 15H), 5.94 (br s, 1H), 4.95 (br s, 1H), 4.79 (d, J = 15.9 Hz, 1H), 4.55 (br s, 1H), 4.19 (t, *J=* 7.7 Hz, 1H), 3.80-3.72 (m, 2H), 3.68-3.64 (m, 1H), 3.41 (m, 4H), 3.22 (dd, *J* = 4.0 Hz, *J* =15.7 Hz, 1H), 2.99 (br s, 1H), 2.92-2.83 (m, 2H), 2.78-2.58 (m, 3H), 2.54-2.49 (m, 1H), 1.61-1.57 (m, 2H), 1.49-1.44 (m, 4H), 1.41-1.34 (m, 10H), 1.31-1.23 (m, 1H), 1.20-1.15 (m, 2H), 1.03-0.97 (m, 2H). ¹³C-NMR (150 MHz, DMSO-*d*₆, 350 K): δ (ppm) 171.6 (CO), 169.4 (CO), 154.5 (CO), 142.3 (2 x C_{q}), 127.9 (4 x CH), 127.5 (4 x CH), 126.2 (CH), 125.9 (2 x CH), 125.8 (CH), 125.6 (2 x CH), 77.7 (Cq), 54.1 (CH), 51.8 (CH), 49.8 (2 x CH₂), 45.0 (CH₂), 42.9 (2 x CH₂), 38.2 (CH₂), 31.4 (CH₂), 30.4 (CH₂), 28.3 (CH₂), 27.8 (3 x CH₃), 23.6 (CH₂), 22.0 (CH₂), 21.5 (CH₂). Two C_{q} carbons one CH and two CH₂ carbons were not detected due to peak broadening caused by isomerism. HRMS (ESI) m/z calcd for C₄₄H₅₇N₅O₆ + H⁺ [M+H]⁺, 752.4382; found, 752.4379. LC-MS (ESI) m/z calcd. for C₄₄H₅₇N₅O₆ + Na⁺ [M+Na]⁺, 775.0; found, 774.6. HPLC purity, 98%, t_{R} = 13.2 min, gradient B.

### Tert-butyl ((S)-1-((2,2-diphenylethyl)amino)-6-((S)-2-(4-moipholino-4--oxobutanoyl)-1,2,3,4-tetrahydroisoquinoline-3-carboxamido)-1-oxohexan-2 yl)carbamate (17)

Compound **17** was prepared according to general procedure 2 **(GP2)** by using compound **7** The crude product was purified by flash column chromatography (DCM/MeOH: 96/4) to afford compound **17** (54 mg, 0.072 mmol, 83%) as a white solid. ¹H-NMR (600 MHz, DMSO-*d₆*, 400 K): δ (ppm) 7.25-7.13 (m, 15H), 5.95 (br s, 1H), 4.93 (br s, 1H), 4.79 (d, *J* = 15.8 Hz, 1H), 4.54 (br s, 1H), 4.19 (t, *J =* 7.7 Hz, 1H), 3.79-3.72 (m, 2H), 3.69-3.64 (m, 1H), 3.56 (m, 4H), 3.45 (m, 4H), 3.21 (dd, *J* = 4.0 Hz, *J* =15.7 Hz, 1H), 3.00 (br s, 1H), 2.89-2.86 (m, 2H), 2.75-2.54 (m, 4H), 1.40-1.34 (m, 10H), 1.29-1.23 (m, 1H), 1.20-1.15 (m, 2H), 1.03-0.98 (m, 2H). ¹³C-NMR (150 MHz, DMSO-*d*₆, 350 K): δ (ppm) 172.5 (CO), 171.2 (CO), 170.4 (CO), 155.5 (CO), 143.3 (2 x C_{q}), 134.0 (C_{q}), 133.8 (C_{q}), 128.9 (2 x CH), 128.8 (2 x CH), 128.4 (4 x CH), 127.2 (CH), 126.8 (2 x CH), 126.7 (CH), 126.5 (2 x CH), 78.6 (C_{q}), 66.6 (2 x CH₂), 55.3 (CH), 52.3 (CH), 50.7 (2 x CH₂), 46.0 (CH), 43.8 (CH₂), 39.0 (CH₂), 32.3 (CH₂), 31.3 (CH₂), 29.2 (CH₂), 28.7 (3 x CH₃), 28.6 (CH₂) 22.9 (CH₂). Two CH₂ carbon was not detected due to peak broadening caused by isomerism. HRMS (ESI) m/z calcd for C₄₃H₅₅N₅O₇Na [M+Na]⁺, 776.3994; found, 776.4016. LC-MS (ESI) m/z calcd. for C₄₄H₅₅N₅O₇ [M+H]⁺, 754.9; found, 754.5. HPLC purity, 99%, t_{R} = 12.9 min, gradient B.

### 4-(4-((S)-3-(((S)-5-((tert-butoxycarbonyl)amino)-6-((2,2-diphenylethyl)amino)-6-oxohexyl)carbamoyl)-3,4-dihydroisoquinolin-2(1H)-yl)-4-oxobutanoyl)-1-methylpiperazin-1-ium 2,2,2-trifluoroacetate (18)

Compound **18** was prepared according to general procedure 2 **(GP2)** by using compound **8.** The crude product was purified by preparative HPLC (tᵣ = 12.4 min, Method B) to afford compound **18** (48 mg, 0.063 mmol, 73%) as white solid. ¹H-NMR (600 MHz, DMSO-*d*₆, 400 K): δ (ppm) 7.26-7.13 (m, 15H), 5.98 (br s, 1H), 4.91 (br s, 1H), 4.78 (d, *J* = 15.8 Hz, 1H), 4.55 (br s, 1H), 4.20 (t, *J =* 7,7 Hz, 1H), 3.77-3.66 (m, 7H), 3.20 (dd, *J* = 4.0 Hz, *J* =15.7 Hz, 1H) and 3.03 (br s, 1H), 2.89-2.85 (m, 2H), 2.75-2.73 (m, 4H), 2.70-2.63 (m, 3H), 1.40-1.32 (m, 10H), 1.28-1.24 (m, 1H), 1.20-1.15 (m, 2H), 1.06-0.95 (m, 2H). ¹³C-NMR (150 MHz, DMSO-*d*₆, 350 K): (ppm) 172.5 (CO), 171.4 (CO), 170.5 (CO), 155.6 (CO), 143.3 (2 x C_{q}), 133.8 (C_{q}), 133.7 (Cq), 128.9 (2 x CH), 128.8 (2 x CH), 128.4 (4 x CH), 127.2 (CH), 126.8 (2 x CH), 126.5 (2 x CH), 78.7 (Cq), 55.1 (CH), 53.1 (2 x CH₂), 50.7 (2 x CH₂), 46.0 (CH), 43.8 (CH₂), 43.0 (CH₃), 39.0 (CH₂), 32.3 (CH₂), 31.5 (CH₂), 29.2 (CH₂), 28.8 (CH₂), 28.7 (3 x CH₃), 28.2 (CH₂) 22.9 (CH₂). One CH, one aromatic CH and one CH₂ carbons were not detected due to peak broadening caused by isomerism. HRMS (ESI) m/z calcd for C₄₄H₅₉N₆O₆ [M+H]⁺, 767.4491; found, 767.4489. LC-MS (ESI) m/z calcd. for C₄₄H₅₉N₆O₆ [M+H]⁺, 767.5; found, 767.6. HPLC purity, 95%, t_{R} = 11.6 min, gradient B.

### Tert-butyl ((S)-1-((2,2-diphenylethyl)amino)-1-oxo-6-((S)-2-(4-oxo-4-(thiophen-2-yl)butanoyl)-1,2,3,4-tetrahydroisoquinoline-3-carboxamido)hexan-2-yl)carbamate (19)

Compound **19** was prepared according to general procedure 2 **(GP2)** by using compound **9.** The crude product was purified by flash column chromatography (DCM/MeOH: 96/4) to afford compound **19** (45 mg, 0.060 mmol, 70%) as white solid ¹H-NMR (600 MHz, DMSO-*d₆,* 400 K): δ (ppm) 8.08 (s, 1H) 7.85 (d, *J =* 3.8 Hz, 1H), 7.83 (d, *J =* 5.0 Hz, 1H), 7.25-7.21 (m, 8H), 7.18-7.13 (m, 7H), 5.89 (br s, 1H), 4.91 (br s, 1H), 4.79 (d, J = 15.8 Hz, 1H), 4.57 (br s, 1H), 4.20 (t, *J =* 7.7 Hz, 1H), 3.78-3.74 (m, 2H), 3.70-3.68 (m, 1H), 3.24 (t, *J* = 6.55 2H), 3.21 (dd, *J =* 4.0 Hz, *J* = 15.7 Hz, 1H), 3.04 (br d, 1H), 2.92-2.86 (m, 3H), 2.77-2.71 (m, 1H + H₂O), 1.45-1.17 (m, 12H), 1.09-0.95 (m, 2H). ¹³C-NMR (150 MHz, DMSO-*d*₆, 350 K): δ (ppm) 191.4 (CO), 171.5 (CO), 171.0 (CO), 169.5 (CO), 154.5 (CO), 143.3 (2 x C_{q}), 142.2 (C_{q}), 133.5 (CH), 132.1 (CH), 127.9 (CH), 127.8 (4 x CH), 127.4 (5 x CH), 126.3 (CH), 125.8 (2 x CH), 125.5 (2 x CH), 77.6 (Cq), 54.1 (CH), 53.8 (CH), 49.7 (CH), 45.0 (CH₂), 42.9 (CH₂), 38.1 (CH₂), 33.6 (CH₂), 31.4 (CH₂), 30.4 (CH₂), 27.8 (CH₂), 27.7 (3 x CH₃), 27.2 (CH₂) 21.9 (CH₂). One aromatic CH carbon was not detected due to peak broadening caused by isomerism. HRMS (ESI) m/z calcd for C₄₃H₅₀N₄O₆Na [M+Na]⁺, 773.3343; found, 773.3341. LC-MS (ESI) m/z calcd. for C₄₃H₅₁N₄O₆S [M+H]⁺, 752.0; found, 751.8. HPLC purity, 99%, t_{R} = 13.0 min, gradient B.

### Tert-butyl ((S)-1-((2,2-diphenylethyl)amino)-6-((S)-2-(4-(furan-2-yl)-4-oxobutanoyl)-1,2,3,4-tetrahydroisoquinoline-3-carboxamido)-1-oxohexan-2-yl)carbamate (20)

Compound **20** was prepared according to general procedure 2 **(GP2)** by using compound **10.** The crude product was purified by flash column chromatography (DCM/MeOH: 96/4) to afford compound **20** (29 mg, 0.004 mmol, 43%) as white solid ¹H-NMR (600 MHz, DMSO-*d₆,* 400 K): δ (ppm) 8.08 (s, 1H), 7.79 (d, *J =* 1.7, 1H), 7.28 (d, *J =* 3.6, 1H), 7.25-7.21 (m, 8H), 7.15-7.13 (m, 7H), 6.61 (br s, 1H), 5.89 (br s, 1H), 4.90 (br s, 1H), 4.78 (d, *J* = 15.9, 1H), 4.55 (br s, 1H), 4.20 (t, *J* = 7.7, 1H), 3.81-3.74 (m, 2H), 3.70-3.65 (m, 1H), 3.19 (dd, *J =* 4.0 Hz, *J* = 15.7 Hz, 1H), 3.12 (t, *J =* 6.6, 2H), 3.05 (br s, 1H), 2.93-2.83 (m, 3H), 2.78 (m, 1H + H₂O), 1.45-1.37 (m, 1H), 1.34 (s, 9H), 1.32-1.24 (m, 2H), 1.22-1.14 (m, 2H), 1.05-0.96 (m, 2H). ¹³C-NMR (150 MHz, DMSO-*d*₆, 350 K): (ppm) 172.0 (CO), 152.2 (CO), 147.0 (C_{q}), 142.7 (C_{q}), 128.3 (2 x CH), 128.2 (2 x CH), 127.9 (4 x CH), 126.2 (CH), 125.9 (2 x CH), 117.4 (CH), 112.2 (CH), 78.1 (Cq), 50.2 (CH), 43.3 (CH₂), 33.2 (CH₂), 31.8 (CH₂), 28.7 (CH₂), 28.2 (3 x CH₃), 27.3 (CH₂), 22.4 (CH₂). Three C_{q}, two CH, aromatic CH and three CH₂ carbons were not detected due to peak broadening caused by isomerism. HRMS (ESI) m/z calcd for C₄₃H₅₁N₄O₇ [M+H]⁺, 735.3752; found, 735.3757. LC-MS (ESI) m/z calcd. for C₄₃H₅₁N₄O₇ [M+H]⁺: 735.38; found, 735.36. HPLC purity, 96%, t_{R} = 21.2 min. A different column was used in LC-MS measurement. Column: Kinetex 2.6u C8 100A, 75 × 2.1 mm, 2.6 µm particles, flow rate 0.3 mL /min, detection wavelengths: 254 nm. Solvent: Methanol (MeOH), water 0.1% HCOOH. Gradient: 0-10% MeOH in 0-3 min, 10-100% MeOH in 3-8%, 100% MeOH in 9%, 100-0% MeOH in 9-12 min.

### Tert-butyl ((S)-6-((S)-2-(4-(3,4-dimethoxyphenyl)-4-oxobutanoyl)-1,2,3,4-tetrahydroisoquinoline-3-carboxamido)-1-((2,2-diphenylethyl)amino)-1-oxohexan-2-yl)carbamate (21)

Compound **21** was prepared according to general procedure 2 **(GP2)** by using 3-(4-dimethoxybenzoyl)propionic acid. The crude solid mixture was purified by flash column chromatography (DCM/MeOH: 96/4) to afford compound **21** (52 mg, 0.065 mmol, 76%) as a white solid. ¹H-NMR (600 MHz, DMSO-*d*₆, 400 K): δ (ppm) 7.68 (d, *J* = 2.1 Hz, rotamer 1) and 7.67 (d, *J* = 2.1 Hz, rotamer 2, 2H), 7.55 (d, *J =* 2.1 Hz, 1H), 7.32-7.19 (m, 16 H), 7.11 (s, rotamer 1) and 7.10 (s, rotamer 2, 1H), 6.01 (d, *J =* 7.7 Hz, 1H), 4.98 (s, 1H), 4.87 (d, *J =* 15.9, 1H), 4.64 (br s, 1H), 4.26 (t, *J =* 8.0 Hz, 1H), 3.91 (s, 3H), 3.88 (s, 3H), 3.84-3.79 (m, 2H), 3.75-3.70 (m, 1H), 3.31 (t, *J* = 6.5 Hz, 1H), 3.25 (dd, *J =* 4.0 Hz, *J* = 15.7 Hz, 1H), 3.10 (br s, 1H), 2.98-2.91 (m, 3H), 2.88-2.77 (m, 1H + H₂O), 1.46-1.41(m, 1H), 1.40 (s, 9H), 1.37-1.29(m, 1H), 1.28-1.23 (m, 2H), 1.12-1.09 (m, 2H). ¹H-NMR assignment was complex due to presence of rotamers in a 1:1 ratio. ¹³C-NMR (150 MHz, DMSO-*d*₆, 350 K): δ (ppm): 198.5 (CO), 173.0 (CO), 172.6 (CO), 170.9 (CO), 156.0 (CO), 153.9 (CH), 149.4 (CH), 143.6 (2 x C_{q}), 134.3 (Cq), 134.0 (C_{q}), 130.5 (Cq), 129.2 (4 x CH) 128.6 (4 x CH) 127.6 (CH), 127.2 (2 x CH), 127.0 (CH), 123.5 (CH), 111.8 (CH), 111.1 (CH), 78.8 (Cq), 56.6 (CH₃), 56.4 (CH₃), 56.1 (CH), 55.1 (CH), 53.4 (CH), 50.1 (CH₂), 46.2 (CH₂), 44.1 (CH₂), 33.8 (CH₂), 32.6 (CH₂), 31.8 (CH₂), 29.6 (CH₂), 29.0 (3 x CH₃), 28.5 (CH₂), 22.9 (CH₂). HRMS (ESI) m/z calcd for C₄₇H₅₆N₄O₈ + Na⁺ [M+Na]⁺, 827.3990; found, 827.3997. LC-MS (ESI) m/z calcd. for C₄₇H₅₆N₄O₈ + H⁺ [M+H]⁺, 806.0; found, 806.1. HPLC purity, 100%, t_{R} = 21.3 min, gradient A.

### Tert-butyl ((S)-1-((2,2-diphenylethyl)amino)-1-oxo-6-((S)-2-(4-phenylbutanoyl)-1,2,3,4-tetrahydroisoquinoline-3-carboxammido)hexan-2-yl)carbamate (22)

Compound 22 was prepared according to general procedure 2 **(GP2)** by using 4-(phenyl)butyric acid. The crude solid mixture was purified by flash column chromatography (DCM/MeOH: 96/4) to afford compound 22 (59 mg, 0.081 mmol, 95%) as a white solid. ¹H-NMR (600 MHz, DMSO-*d*₆, 400 K): δ (ppm) 7.30-7.18 (m, 20 H), 6.02 (d, *J =* 8.1 Hz, 1H), 4.90 (br s, 1H), 4.76 (br s, 1H), 4.55 (d, *J =* 16.6 Hz, 1H), 4.26 (t, *J =* 7.7 Hz, 1H), 3.87-3.79 (m, 2H), 3.75-3.71 (m, 1H), 3.20 (dd, *J* = 4.0 Hz, *J* =15.7 Hz, 1H), 3.09 (br s, 1H), 2.95-2.90 (m, 2H + H₂O), 2.70 (t, *J* = 7.6 Hz, 2H), 2.54-2.42 (m, 2H + DMSO), 1.97-192 (m, 2H), 1.44-1.30 (m, 11H), 1.25-1.21 (m, 2H), 1.11-1.05 (m, 2H). ¹³C-NMR (150 MHz, DMSO-*d₆,* 350 K): δ (ppm) 173.0 (CO), 171.1 (CO), 170.8 (CO), 156.0 (CO), 143.6 (Cq), 142.9 (2 x C_{q}), 134.6 (Cq), 129.3 (CH), 129.2 (8 x CH), 128.8 (4 x CH), 127.2 (CH), 126.8 (CH), 125.8 (CH), 78.8 (C_{q}), 56.0 (CH), 55.1 (CH), 53.4 (CH), 51.0 (CH₂), 46.2 (CH₂), 43.9 (CH₂), 35.6 (CH₂), 33.4 (CH₂), 32.5 (CH₂), 29.7 (CH₂), 29.1 (3 x CH₃), 27.3 (CH₂), 23.3 (CH₂). One CH₂ carbon was not detected due to peak broadening caused by isomerism. HRMS (ESI) m/z calcd for C₄₅H₅₄N₄O₅Na [M+Na]⁺, 753.3986; found, 753.3994. LC-MS (ESI) m/z calcd. for C₄₅H₅₅N₄O₅Na [M+Na+H]²⁺, 754.4; found, 754.0. HPLC purity, 98%, t_{R} = 21.5 min, gradient A.

### Tert-butyl((S)-1-((2,2-diphenylethyl)amino)-1-oxo-6-((S)-2-(3-phenylpropanoyl)-1,2,3,4-tetrahydroisoquinoline-3-carboxamido)hexan-2-yl)carbamate (23)

To a solution of 3-phenylpropionic acid (29 mg, 0.19 mmol) in 2 mL of anhydrous DMF were added diisopropylethylamine (34 mg, 45 µl, 0.26 mmol) and HATU (52 mg, 0.22 mmol) at 0 °C. The reaction mixture stirred for 30 min. Then the reaction mixture warmed up at rt and compound 5 (100 mg, 0.17 mmol) was added and stirred for 16h. The reaction mixture was washed with H₂O, then a saturated solution of NaHCO₃ was added and the aqueous phase was extracted with DCM. The combined organic extracts were washed with brine, dried over Na₂SO₄, filtered and evaporated *in vacuo.* The crude solid mixture was purified by flash column chromatography (Hex/EtOAc/EtOH: 6/3/1) to afford the titled compound **23** (77 mg, 0.11 mmol, 63%) as a white solid. ¹H-NMR (600 MHz, DMSO-*d₆*, 400 K): δ (ppm) 7.64 (br s) and 7.50 (t, *J* = 5.4 Hz, 1H), 7.37-7.16 (m, 20H), 6.29 (br s, 1H), 5.03-4.46 (m, 3H), 4.24 (t, *J* = 7.8 Hz, 1H), 3.85-3.81 (ddd, *J=* 6.1 Hz, *J=* 7.9 Hz, *J=* 13.8 Hz, rotamer 1) and 3.71-3.66 (ddd, *J =* 5.8 Hz, *J =* 7.7 Hz, *J =* 13.2 Hz, rotamer2, 2H), 3.78 (br s, 1H), 3.24-3.16 (m, 1H), 3.31-2.75 (m, 8H + H₂O), 1.42-1.24 (m, 11H), 1.16-1.12 (m, 2H), 1.03-0.97 (m, 2H). ¹H-NMR assignment was complex due to presence of rotamers in a 1:1 ratio. ¹³C-NMR (150 MHz, DMSO-*d₆*, 350 K): δ (ppm) 172.5 (CO), 155.5 (CO), 143.3 (2 x C_{q}), 142.0 (C_{q}), 129.1 (CH), 128.9 (2 x CH), 128.8 (2 x CH), 128.4 (2 x CH), 126.8 (3 x CH), 126.3 (CH) 78.6 (C_{q}), 50.7 (CH), 43.8 (CH₂), 39.0 (CH₂), 35.2 (CH₂), 32.3 (CH₂), 31.0 (CH₂), 29.3 (CH₂), 28.8 (3 x CH₃), 22.9 (CH₂). Two Cq, three CH, five aromatic CH and two CH₂ carbons were not detected due to peak broadening caused by isomerism. HRMS (ESI) m/z calcd for C₄₄H₅₂N₄O₅Na [M+Na]⁺, 739.3830; found, 739.3842. LC-MS (ESI) *m*/*z* calcd. for C₄₅H₅₂N₄O₅Na [M+Na]⁺, 739.4; found, 740.1. HPLC purity, 98%, t_{R} = 22 min, gradient A.

### Tert-butyl ((S)-1-((2,2-diphenylelhyl)amino)-1-oxo-6-((S)-2-(5-oxo-5-phenylpentanoyl)-1,2,3,4-tetrahydroisoquinoline-3-carboxamido)hexan-2-yl)carbamate (24)

Compound **24** was prepared according to general procedure 2 **(GP2)** by using 4-benzoylboutyric acid. The crude solid mixture was purified by preparative HPLC employing method C (t_{R} = 19 min) to afford compound **24** (107 mg, 0.14 mmol, 45%) as a white solid. ¹H-NMR (600 MHz, DMSO-*d₆*, 400 K): δ (ppm) 7.98-7.96 (m, 2H), 7.65-7.62 (m, 1H), 7.55-7.53 (m, 2H), 7.32-7.19 (m, 15H), 6.03 (br s, 1H), 4.93 (br s, 1H), 4.80 (d, *J* = 15.9 Hz, 1H), 4.58 (br s, 1H), 4.26 (t, *J =* 7.7 Hz, 1H), 3.87-3.79 (m, 2H), 3.75-3.71 (m, 1H), 3.18 (dd, *J =* 4.0 Hz, *J* =15.7 Hz, 1H), 3.11-3.09 (m, 3H), 2.97-2.92 (m, 2H), 2.61-2.59 (m, 2H + DMSO), 2.0 (quint, *J =* 7.1 Hz, 2H), 1.45-1.31 (m, 11H), 1.27-1.21 (m, 2H), 1.11-1.05(m, 2H). ¹³C-NMR (150 MHz, DMSO-*d₆*, 350 K): δ (ppm) 200.5 (CO), 172.6 (CO), 155.6 (CO), 143.3 (2 x C_{q}), 137.5 (C_{q}), 133.4 (C_{q}), 129.2 (2 x CH), 128.9 (2 x CH), 128.8 (CH₂), 128.4 (4 x CH), 128.3 (2 x CH), 126.8 (2 CH), 78.6 (C_{q}) 55.1 (CH), 50.7 (CH), 46.0 (CH), 43.8 (CH₂), 39.0 (CH₂) 38.1 (CH₂), 32.9 (CH₂), 32.3 (CH₂), 29.3 (CH₂), 28.7 (3 x CH₃), 23.0 (CH₂), 20.1 (CH₂). One Cq, two aromatic CH and two CH₂ carbons were not detected due to peak broadening caused by isomerism. HRMS (ESI) m/z calcd for C₄₆H₅₄N₄O₆Na [M+Na]⁺, 781.3936; found, 781.3933. LC-MS (ESI) m/z calcd. for C₄₆H₅₅N₄O₆Na [M+Na+H]²⁺, 782.4; found, 782.6. HPLC purity, 100%, t_{R} = 23.3 min, gradient A.

### (S)-1-((2,2-diphenylethyl)amino)-1-oxo-6-((S)-2-(4-oxo-4-phenylbutanoyl)-1,2,3,4-tetrahydroisoquinoline-3-carboxamido)hexan-2-aminium 2,2,2-trifluoroacetate (25)

Compound **25** was prepared according to general procedure 4 **(GP4).** The crude solid mixture was purified by preparative HPLC employing method A (t_{R} = 13.5 min) to afford compound **25** as TFA salt (163 mg, 0.215 mmol, 75%) as a white solid. ¹H-NMR (600 MHz, DMSO-*d₆*, 400 K): δ (ppm) 7.97-7.95 (m, 2H), 7.63-7.61 (m, 1H), 7.53-7.51(m, 2H), 7.31-7.16 (m, 15H), 4.95 (br s, 1H), 4.85 (d, *J* = 15.8 Hz, 1H), 4.61 (br s, 1H), 4.25 (t, *J* = 7.8 Hz, 1H), 3.99-3.96(m, 1H), 3.68-3.51 (m, 2H), 3.32 (t, *J =* 6.5 Hz, 2H) 3.21 (dd, *J* = 4.0 Hz, *J* =15.7 Hz, 1H), 3.10-2.81 (m, 5H + H₂O), 1.50 (s, 2H), 1.22-1.19 (m, 2H), 1.06-1.03(m, 2H). ¹³C-NMR (150 MHz, DMSO-*d₆*, 400 K): δ (ppm) 199.1 (CO), 171.7 (CO), 170.1 (CO), 168.4 (CO), 142.4 (C_{q}), 142.3 (C_{q}), 137.0 (C_{q}), 132.9 (C_{q}), 128.6 (2 x CH), 128.4 (2 x CH), 128.3 (2 x CH), 127.8 (2 x CH), 127.7 (4 x CH), 126.4 (2 x CH), 126.2 (CH), 126.0 (CH), 52.3 (CH), 50.1 (CH), 43.4 (CH₂), 33.4 (CH₂), 30.7 (CH₂), 28.5 (CH₂), 27.6 (CH₂), 21.2 (CH₂). One Cq, one CH, one aromatic CH and three CH₂ carbons were not detected due to peak broadening caused by isomerism. HRMS (ESI) m/z calcd for C₄₀H₄₅N₄O₄ [M+H]⁺, 645.3435; found, 645.3434. MS (ESI) m/z (%): (C₉H₁₁N) 133 (14), (C₉H₁₀N) 132 (100), 131 (20), 130 (11), 84 (11), 59 (32), 57 (12), 41 (17). MS (ESI) m/z calcd for C₄₀H₄₄N₄O₄ [M]⁺ 644.34, found 644.82. LC-MS (ESI) m/z calcd. for C₄₀H₄₆N₄O₄ [M+2H]²⁺, 646.3; found, 646.2. HPLC purity, 99%, t_{R} = 19.5 min, gradient A.

### (S)-1-((2,2-diphenylethyl)amino)-1-oxo-6-((S)-2-(5-oxo-5-phenylpentanoyl)-1,2,3,4-tetrahydroisoquinoline-3-carboxamido)hexan-2-aminium 2,2,2-trifluoroacetate (26)

Compound **26** was prepared according to general procedure 4 **(GP4).** The crude solid mixture was purified by preparative HPLC employing method C to afford compound **26** as TFA salt (14 mg, 0.018 mmol, 43%). ¹H-NMR (600 MHz, DMSO-*d₆*, 350 K): δ (ppm) 7.95 (d, *J =* 7.6 Hz, 2H), 7.63-7.59 (m, 1H), 7.51 (t, *J* = 7.8 Hz, 2H), 7.44-7.15 (m, 15H), 4.98-4.47 (m, 3H), 4.22 (t, *J* = 7.9 Hz, 1H), 3.77-3.71 (m, 2H), 3.20-3.16 (m, 7H + H₂O), 2.90-2.85 (m, 2H), 2.62-2.30 (m, 2H + DMSO), 1.94-1.92 (m, 2H) 1.34 (br s, 2H), 1.17 (m, 2H), 1.01 (br, 2H). ¹³C-NMR (150 MHz, DMSO-*d₆*, 350 K): δ (ppm) 143.3 (2 x C_{q}), 137.6 (C_{q}), 133.4 (C_{q}), 131.1 (C_{q}), 129.2 (CH), 128.9 (2 x CH), 128.4 (4 x CH), 128.3 (2 x CH), 126.7 (3 x CH), 50.8 (CH) 43.5 (CH₂), 38.1 (CH₂), 35.1 (CH₂), 32.8 (CH₂), 29.5 (CH₂), 22.9 (CH₂), 20.1 (CH₂). Two CH, five aromatic CH and three CH₂ carbons were not detected due to peak broadening caused by isomerism. HRMS (ESI) m/z calcd for C₄₁H₄₇N₄O₄ [M+H]⁺, 659.3592; found, 659.3598. LC-MS (ESI) *m*/*z* calcd. for C₄₁H₄₈N₄O₄ [M+2H]⁺, 660.4; found, 660.2. HPLC purity, 99 %, t_{R} = 19.8 min, gradient A.

### 4-((S)-3-(((S)-5-((tert-butoxycarbonyl)amino)-6-((2,2-diphenylethyl)amino)-6-oxohexyl)carbamoyl)-3,4-dihydroisoquinolin-2(1H)-yl)-4-oxobutanoic acid (27)

To solution of succinic anhydride (43 mg, 0.43 mmol) in 2.5 ml anhydrous DCM was added compound **5** (50 mg, 0.085 mmol) was added to the reaction mixture, then it was warmed up at room temperature and stirred for 18 h. A saturated solution of NaHCO₃ was added and the aqueous phase was extracted with DCM. The combined organic extracts were washed with brine, dried over Na₂SO₄, filtered and evaporated in *vacuo.* The crude solid mixture was purified by preparative HPLC employing method D (t_{R} = 17.0 min) to afford compound **27** (47 mg, 0.069 mmol, 81%). ¹H-NMR (600 MHz, DMSO- d₆, 400 K): δ (ppm) 7.25-7.14 (m, 15H), 5.98 (br s, 1H), 4.90 (br s, 1H), 4.78 (d, *J=* 14.7 Hz, 1H), 4.55 (br s, 1H), 4.22 (t, *J=* 7.7 Hz, 1H), 3.80-3.76 (m, 2H), 3.71-3.66(m, 1H), 3.20-2.73 (several m, 8H + H₂O + DMSO), 1.36-1.16 (m, 13H), 1.08-1.00 (m, 2H) ¹³C-NMR (150 MHz, DMSO-*d₆*, 350 K): δ (ppm) 172.7 (CO), 171.0 (CO), 154.0 (CO), 141.7 (2 x Cq), 132.5 (2 x Cq), 127.3 (4 x CH), 126.9 (4 x CH), 125.7 (CH), 125.3 (CH), 125.0 (CH), 77.1 (C_{q}), 53.6 (CH), 51.5 (CH) 49.2 (CH), 44.4 (CH₂), 42.3 (CH₂), 37.4 (CH₂), 35.6 (CH₂), 30.8 (CH₂), 28.2 (CH₂), 27.7 (CH₂), 27.2 (3 x CH₃), 21.4 (CH₂). Two aromatic CH carbons were not detected due to peak broadening caused by isomerism. HRMS (ESI) m/z calcd for C₃₉H₄₈N₄O₇Na [M+Na]⁺, 707.3415; found, 707.3417. LC-MS (ESI) m/z calcd. for C₃₉H₄₉N₄O₇ [M+H]⁺, 685.8; found, 685.9. HPLC purity, 97 %, t_{R} = 21.0 min, gradient A.

### (S)-3-(((S)-5-ammonio-6-((2,2-diphenylethyl)amino)-6-oxohexyl)carbamoyl)-1,2,3,4-tetrahydroisoquinolin-2-ium 2,2,2-trifluoroacetate (28)

Compound **28** was prepared according to general procedure 4 **(GP4).** The crude solid mixture was purified by preparative HPLC (Method E) to afford compound **28** as TFA salt (36 mg, 0.05 mmol, 32%). ¹H-NMR (600 MHz, DMSO-*d₆*, 400 K): δ (ppm) 8.10 (s, 2H), 7.33-7.20 (m, 14H), 4.30-4.27 (m, 3H), 4.03 (dd, *J=* 4.9 Hz, *J* = 10.7 Hz, 1H), 3.98 (m, 1H), 3.65 (t, *J =* 7.4 Hz, 1H), 3.30-3.26 (dd, *J* = 4.9 Hz, *J* = 16.7 Hz, 1H), 3.13-3.03 (m, 2H + H₂O), 1.59-1.55 (m, 2H), 1.40-1.36 (m, 2H), 1.20-1.15 (m, 2H). ¹³C-NMR (150 MHz, DMSO-*d₆*, 350 K): δ (ppm) 168.9 (2 x CO), 142.9 (C_{q}), 142.8 (C_{q}), 136.0 (C_{q}), 129.4 (C_{q}), 128.9 (2 x CH), 128.8 (2 x CH), 128.1 (2 x CH), 128.0 (2 x CH), 127.3 (CH), 126.9 (4 x CH), 54.9 (CH), 52.8 (CH), 50.6 (CH), 43.9 (CH₂), 39.0 (CH₂), 31.2 (CH₂), 29.3 (CH₂), 28.9 (CH₂), 21.8 (CH₂). One CH₂ carbons was not detected due to peak broadening caused by isomerism. HRMS (ESI) m/z calcd for C₃₀H₃₇N₄O₄ [M+H]⁺, 485.2911; found, 485.2907. LC-MS (ESI) *m*/*z* calcd. for C₃₀H₃₇N₄O₄ [M+H]⁺, 485.3; found, 485.6. HPLC purity, 95 %, t_{R} = 9.2 min, gradient B.

### N6-((benzyloxy)carbonyl)-N2-(tert-butoxycarbonyl)-D-lysine (29)

To a 1M aqueous solution of NaOH (37 mLat rt were added Z-D-Lys(Boc)-OH (3.00 g, 12.2) and dioxane slowly until the suspension dissolves. Then benzyl chloroformate was added (2.70 g, 2.3 µL, 15.9) dropwise over a period of 30 min at 0 °C. The reaction mixture was stirred for 16 h at room temperature. Furthermore dioxane was removed *in vacuo* and reaction mixture was acidified to pH 2 with 1M HCl at 0 °C. The aqueous layer was extracted with EtOAc. The combined organic layers were dried over Na₂SO₄, filtered and evaporated *in vacuo* to afford compound **29** (4.4, 11.6 mmol, 95%) as a white foam. No further purification was required. ¹H-NMR (360 MHz, CDCl₃): δ (ppm) 7.37-7.23 (m, 5H), 6.18 (br s, 1H), 5.17-4.86 (m, 3H), 4.83 (s, 1H), 4.22-4.04 (m, 1H), 3.13-3.11 (m, 2H), 1.76-1.37 (m, 15H). ¹³C-NMR (150 MHz, CDCl₃)**:** δ (ppm) 176.4 (CO), 156.8 (CO), 156.0 (CO), 136.6 (C_{q}), 128.7 (2 x CH), 128.3 (2 x CH), 128.1 (CH), 80.4 (C_{q}) 66.9 (CH₂), 53.3 (CH), 40.7 (CH₂), 32.0 (CH₂), 29.3 (CH₂), 29.5 (3 x CH₃), 22.1 (CH₂). MS (ESI) *m*/*z* (%): (C₁₄H₂₀N₂O₄) 222 (14), (C₇H₁₂NO₄) 174 (12), 128 (32), 108 (27), 92 (11), (C₇H₇) 91 (100), 84 (20), 57 (52), 41 (20). MS (ESI) m/z calcd for C₁₄H₂₀N₂O₄ [M + H - C₅H₉O₂]⁺, 280.14; found 280.14. LC-MS (ESI) *m*/*z* calcd. for C₁₉H₂₈N₂O₆Na [M+Na]⁺, 403.2; found, 403.7. HPLC purity, 96%, t_{R} = 19.7 min, gradient A.

### Benzyl tert-butyl (6-((2,2-diphenylethyl)amino)-6-oxohexane-1,5-diyl)(R)-dicarbamate (30)

Compound **30** was prepared according to general procedure 1 **(GP1).** The crude solid mixture was purified by flash column chromatography (DCM/MeOH: 98/2) to afford compound **30** (3.00 g, 9.60, 5.36mmol, 80%) as a white solid. ¹H-NMR (600 MHz, CDCl₃): Identical with compound 1. ¹³C-NMR (150 MHz, CDCl₃): Identical with compound 1. MS (ESI) m/z (%): 307 (26), 263 (11), 218 (18), 180 (34), 174 (15), 167 (23), 108 (18), 107 (14), 91 (100), 84 (21), 79 (19), 77 (13), 59 (15), 57 (48), 41 (22), 30 (38). MS (ESI) m/z calcd for C₃₃H₄₁N₃O₅ [M]⁺ 559.30; found, 559.30. LC-MS (ESI) m/z calcd. for C₃₃H₄₁N₃O₅Na [M+Na]⁺, 582.7; found, 582.4. HPLC purity, 100%, t_{R} = 13.2min, gradient B.

### Tert-butyl (R)-(6-amino-1-((2,2-diphenylethyl)amino)-1-oxohexan-2-yl)carbamate (31)

Compound **31** was prepared according to general procedure 3 **(GP3),** to afford the titled compound **31** (2.21 g, 5.18 mmol, 98%) as a white solid. ¹H-NMR (600 MHz, CDCl₃): Identical with compound 2. ¹³C-NMR (150 MHz, CDCl₃): Identical with compound **2.** MS-ESI m/z (%): 425 (12) C₂₅H₃₅N₃O₃, 258 (14), 202 (18), 181 (11), 180 (25), 173 (14), 167 (27), 165 (18), 145 (13), 139 (15), 129 (12), 84 (100), 82 (18), 57 (50), 56 (18), 41 (26), 30 (36). MS (ESI) m/z calcd for C₂₅H₃₅N₃O₃ [M]⁺, 425.27, found 425.27. LC-MS (ESI) m/z calcd. for C₂₅H₃₆N₃O₃ [M+H]⁺, 426.3; found, 426.8. HPLC purity, 96%, t_{R} = 17.6 min, gradient A.

### Benzyl (S)-3-(((R)-5-((tert-butoxycarbonyl)amino)-6-((2,2-diphenylethyl)amino)-6-oxohexyl) carbamoyl)-3,4-dihydroisoquinoline-2(1H)-carboxylate (32)

Compound **32** was prepared according to general procedure 1 **(GP1).** The crude solid mixture was purified by flash column chromatography (Hex/EtOAc/EtOH: 9/3/1) to afford compound **32** (0.60 g, 0.84 mmol, 70%) as a white solid. ¹H-NMR (600 MHz, CDCl₃)**:** Identical with compound **4.** ¹³C-NMR (150 MHz, CDCl₃)**:** Identical with compound **4.** LC-MS (ESI) m/z calcd. for C₄₃H₅₁N₄O₆Na [M+Na+H]²⁺, 742.4; found, 742.2. HPLC purity, 100%, t_{R} = 22.4 min, gradient A.

### Tert-butyl ((R)-1-((2,2-diphenylethyl)amino)-1-oxo-6-((S)-1,2,3,4-tetrahydroisoquinoline-3-carboxamido)hexan-2-yl)carbamate (33)

Compound **33** was prepared according to general procedure 3 **(GP3),** to afford compound **33** (0.40 g, 0.68 mmol, 97%) as a white solid. No further purification was required for the next step. ¹H-NMR (600 MHz, *CDCl*₃): δ (ppm) Identical with compound **5.** ¹³C-NMR (150 MHz, CDCl₃): Identical with compound **5.** MS (ESI) *m*/*z* (%): (C₉H₁₁N) 133 (14), (C₉H₁₀N) 132 (100), 131 (20), 130 (11), 84 (11), 59 (32), 57 (12), 41 (17). MS (ESI) m/z calcd for C₃₅H₄₄N₄O₄ [M]⁺ 584.34, found 584.34. LC-MS (ESI) *m*/*z* calcd. for C₃₅H₄₅N₄O₄ [M+H]⁺, 585.3; found, 585.9. HPLC purity, 91%, t_{R} = 20.5min, gradient A.

### Tert-butyl ((R)-1-((2,2-diphenylethyl)amino)-1-oxo-6-((S)-2-(4-oxo-4-phenylbutanoyl)-1,2,3,4-tetrahydroisoquinoline-3-carboxamido)hexan-2-yl)carbamate (34)

Compound **34** was prepared according to general procedure 2 **(GP2)** by using 3-benzoylpropionic acid. The crude solid mixture was purified by flash column chromatography (Hex/EtOAc/EtOH: 9/3/1)) to afford compound **34** (93 mg, 0.13 mmol, 73%) as a white solid. ¹H-NMR (600 MHz, DMSO-*d₆*, 400 K): δ (ppm) Identical with compound **11.** ¹³C-NMR (150 MHz, DMSO-*d*₆, 350 K): Identical with compound **11.** HRMS (ESI) m/z calcd for C₄₅H₅₂N₄O₆Na [M+Na]⁺, 767.3779; found, 767.3774. LC-MS (ESI) m/z calcd. for C₄₅H₅₄N₄O₆ [M+2H]²⁺, 746.4; found, 746.2. HPLC purity, 98%, t_{R} = 21.7min, gradient A.

### (R)-1-((2,2-diphenylethyl)amino)-1-oxo-6-((S)-2-(4-oxo-4-phenylbutanoyl)-1,2,3,4-tetrahydroisoquinoline-3-carboxamido)hexan-2-aminium 2,2,2-trifluoroacetate (35)

Compound **35** was prepared according to general procedure 4 **(GP4).** The crude solid mixture was purified by preparative HPLC employing method C (t_{R} = 16.1 min) to afford compound **35** as TFA salt (45 mg, 0.059 mmol, 73%). ¹H-NMR (360 MHz, DMSO-*d₆*, 400 K): Identical with compound **25.** ¹³C-NMR (150 MHz, DMSO-*d₆*, 350 K): Identical with compound **25.** HRMS (ESI) m/z calcd for C₄₀H₄₅N₄O₄ + [M+H]⁺, 645.3435; found, 645.3433. LC-MS (ESI) *m*/*z* calcd. for C₄₀H₄₆N₄O₄ [M+2H]²⁺, 646.3; found, 646.0. HPLC purity, 99%, t_{R} = 18.2 min, gradient A.

### Benzyl tert-butyl (6-((2,2-diphenylethyl)amino)-6-oxohexane-1,5-diyl)(S)-dicarbamate (36)²⁸

Compound **36** was prepared according to general procedure 1 **(GP1).** The crude solid mixture was purified by flash column chromatography (DCM/MeOH: 96/4) to afford compound **36** ( 1.84 g, 3.3 mmol, 97%) as a white solid. ¹H-NMR (600 MHz, CDCl₃): δ (ppm) 7.40-7.06 (m, 15H + CDCl₃), 5.93 (br s, 1H), 5.25-5.22 (m, 1H), 4.98-4.93 (m, 2H), 4.45 (br s, 1H), 4.10 (t, *J=* 7.8 Hz, 1H), 3.89-3.73 (m, 3H), 2.95 (br s, 2H) 1.62 (br s, 1H), 1.41-1.11 (m, 14H). ¹³C-NMR (150 MHz, CDCl₃): δ (ppm) 170.4 (CO), 155.1 (2 x CO), 140.6 (2 x C_{q}), 135.1 (C_{q}), 127.8 (2 x CH), 127.7 (2 x CH), 127.5 (2 x CH), 127.2 (CH), 127.0 (2 x CH), 125.9 (2 x CH), 78.2 (C_{q}), 66.0 (CH₂), 53.8 (CH), 49.5 (CH₂), 42.7 (CH) 38.7 (CH₂), 29.9 (CH₂), 28.5 (CH₂), 27.4 (3 x CH₃), 21.2 (CH₂). MS (ESI) m/z (%): (C₁₄H₁₃) 181 (18), 180 (78), 168 (16), (C₁₃H₁₀) 166 (12), 165 (19), 152 (12), 128 (10.1), (C₇H₇) 91 (100), 84 (21), 82 (12), 57 (13), 30 (13). MS (ESI) m/z calcd for C₃₃H₄₁N₃O₅ [M]⁺ 559.30 , found 559.30. LC-MS (ESI) m/z calcd. for C₃₃H₄₂N₃O₅Na [M+Na+H]²⁺, 583.3; found, 583.3, t_{R} = 21.6 min, gradient A.

### Benzyl tert-butyl (6-((2,2-diphenylethyl)amino)-6-oxohexane-1,5-diyl)(R)-dicarbamate (37)

Compound **37** was prepared according to general procedure 1 **(GP1).** The crude solid was purified by flash column chromatography (DCM/MeOH: 96/4) to afford compound **37** (2.9mg, 5.2 mmol, 99%) as a white solid. ¹H-NMR (600 MHz, CDCl₃)**:** δ (ppm) 7.28-7.13 (m, 15H), 5.96 (br s, 1H), 5.27 (br s, 1H), 4.98-4.93 (m, 2H), 4.46 (br s, 1H), 4.09 (t, J = 7.8 Hz, 1H), 3.89-3.73 (m, 3H), 2.95 (br s, 2H) 1.62-1.59 (m, 1H), 1.35-1.09 (m, 14H). ¹³C-NMR (150 MHz, CDCl₃): δ (ppm) 170.5 (CO), 155.1 (2 x CO), 140.6 (2 x C_{q}), 135.1 (C_{q}), 127.7 (4 x CH), 127.5 (2 x CH), 127.2 (3 x CH), 127.0 (2 x CH), 125.8 (2 x CH), 78.2 (C_{q}), 66.0 (CH₂), 53.8 (CH), 49.5 (CH₂), 42.7 (CH), 38.7 (CH₂), 30.9 (CH₂), 28.6 (CH₂), 27.4 (3 x CH₃), 21.2 (CH₂). LC-MS (ESI) m/z calcd. for C₃₃H₄₂N₃O₅Na [M+Na+H]²⁺, 583.3; found, 583.1. HPLC purity, 100%, t_{R} = 21.6 min, gradient A.

### Tert-butyl (S)-(5-amino-6-((2,2-diphenylethyl)amino)-6-oxohexyl)carbamate (38)²⁸

Compound **38** was prepared according to general procedure 3 (**GP3**), to afford compound **38** (1.06 g, 2.49 mmol, 93%) as a yellow oil. No further purification was required for the next step. ¹H-NMR (600 MHz, CDCl₃): δ (ppm) 7.25-7.22 (m, 4H), 7.19-7.13 (m, 7H), 4.45 (s, 1H), 4.13 (t, *J* = 8.0 Hz, 1H), 3.86 (ddd, *J =* 6.2 Hz, *J* = 8.1 Hz, *J* = 14.2 Hz, 1H), 3.79 (ddd, *J* = 6.2 Hz, *J =* 8.1 Hz, *J =* 14.2 Hz, 1H), 3.19 (dd, *J =* 4.6 Hz, *J =* 7.8 Hz, 1H), 3.00-2.99 (m, 2H), 1.62-1.58 (m, 1H), 1.37-1.32 (m, 12 H), 1.19-1.14 (m, 2H). ¹³C-NMR (150 MHz, CDCl₃): δ (ppm) 175.2 (CO), 156.4 (CO), 142.3 (2 x C_{q}), 129.0 (4 x CH), 128.4 (4 x CH), 127.1 (2 x CH), 77.6 (C_{q}), 55.3 (CH), 51.1 (CH₂), 43.7 (CH), 40.5 (CH₂), 34.9 (CH₂), 31.5 (CH₂), 30.2 (CH₂), 28.8 (3 x CH₃), 23.0 (CH₂). MS (ESI) m/z (%): 201 (53), 180 (16), 167 (16), 165 (12), 158 (11), 145 (30), 139 (12), 128 (36), 127 (18), 101 (11), 84 (100), 82 (12), 57 (31), 56 (21), 44 (10), 43 (11), 41 (22), 30 (15). MS (ESI) m/z calcd for C₂₅H₃₅N₃O₃ [M]⁺ 425.27; found, 425.27. LC-MS (ESI) m/z calcd. for C₂₅H₃₇N₃O₃ [M+H]⁺, 426.3; found, 426.3, t_{R} = 11.2 min, gradient B.

### Tert-butyl (R)-(5-amino-6-((2,2-diphenylethyl)amino)-6-oxohexyl)carbamate (39)

Compound **39** was prepared according to general procedure 3 (**GP3**), to afford compound **39** (2.217 g, 5.10 mmol, 99%) as a yellow oil. No further purification was required for the next step. ¹H-NMR (600 MHz, CDCl₃): δ (ppm) 7.24-7.22 (m, 4H), 7.19-7.13 (m, 7H), 4.46 (s, 1H), 4.14 (t, *J =* 8.0 Hz, 1H), 3.83(ddd, *J* = 6.2 Hz, *J =* 8.1 Hz, *J =* 14.3 Hz, 1H), 3.78 (ddd, *J* = 6.2 Hz, *J =* 8.1 Hz, *J =* 14.3 Hz, 1H), 3.17 (dd, *J* = 4.5 Hz, *J =* 7.8 Hz, 1H), 3.00-2.99 (m, 2H), 1.62-1.58 (m, 1H), 1.37-1.30 (m, 12H), 1.19-1.14 (m, 2H). ¹³C-NMR (90 MHz, CDCl₃): δ (ppm) 173.8 (CO), 155.0 (CO), 141.0 (2 x C_{q}), 127.6 (4 x CH), 127.1 (4 x CH), 125.7 (2 x CH), 78.1 (C_{q}), 54.0 (CH), 49.7 (CH), 42.3 (CH₂), 39.2 (CH₂), 33.6 (2 x CH₂), 27.4 (3 x CH₃), 21.6 (CH₂). MS (ESI) *m*/*z* (%): 201 (54), 180 (16), 167 (16), 165 (13), 158 (11), 145 (34), 139 (15), 128 (36), 127 (11), 84 (100), 82 (14), 57 (34), 56 (20), 44 (12), 43 (11), 41 (24), 30 (16). MS (ESI) m/z calcd for C₂₅H₃₅N₃O₃ [M]⁺, 425.27; found, 425.27. LC-MS (ESI) *m*/*z* calcd. for C₂₅H₃₆N₃O₃ [M+H]⁺, 426.6; found, 426.9. HPLC purity, 98%, t_{R} = 17.5 min, gradient A.

### Benzyl (S)-3-(((S)-6-((tert-butoxycarbonyl)amino)-1-((2,2-diphenylethyl)amino)-1-oxohexan-2-yl)carbamoyl)-3,4-dihydroisoquinoline-2(1H)-carboxylate (40)

Compound **40** was prepared according to general procedure 1 **(GP1).** The crude solid mixture was purified by flash column chromatography (DCM/MeOH: 96/4) to afford compound **40** (1.01 g, 1.41 mmol, 48%) as a white solid. ¹H-NMR (600 MHz, CDCl₃): δ (ppm) 7.30-7.09 (m, 19H + CDCl₃), 6.24 (d, *J =* 8.0 Hz, 1H), 6.06 (s, rotamer 1) and 5.76 (s, rotamer 2, 1H), 5.18-5.09 (m, 2H), 4.66-4.37 (m, 4H), 4.11-3.91 (m, 2H), 3.80 (ddd, *J* = 6.4 Hz, *J=* 8.0 Hz, *J* = 14.2 Hz, 1H), 3.71 (br s, rotamer 1) and 3.51 (br s, rotamer 2, 1H) 3.19-3.16 (m, 1H), 2.95 (br s, 1H), 2.81 (br s, 2H), 1.48-0.99 (m, 15H). ¹H-NMR assignment was complex due to presence of rotamers in 2:1 ratio. ¹³C-NMR (150 MHz, CDCl₃): δ (ppm) 170.1 (CO), 155.9 (2 x CO), 140.8 (2 x C_{q}), 140.0 (C_{q}), 135.2 (C_{q}), 132.4 (C_{q}), 127.8 (4 x CH), 127.5 (CH), 127.3 (2 x CH), 127.0 (6 x CH), 126.3 (CH), 126.0 (CH), 125.2 (CH), 78.3 (C_{q}), 67.2 (CH), 64.5 (CH₂) 55.2 (CH), 51.6 (CH), 49.6 (CH₂), 44.4 (CH₂), 42.9 (CH₂), 39.1 (CH₂), 30.7 (CH₂), 28.4 (CH₂), 27.6 (3 x CH₃), 21.1 (CH₂). LC-MS (ESI) *m*/*z* calcd. for C₄₃H₅₁N₄O₆Na [M+Na+H]²⁺, 742.3; found, 742.3. HPLC purity, 93%, t_{R} = 20.5 min, gradient A.

### Benzyl (S)-3-(((R)-6-((tert-butoxycarbonyl)amino)-1-((2,2-diphenylethyl)amino)-1-oxohexan-2-yl)carbamoyl)-3,4-dihydroisoquinoline-2(1H)-carboxylate (41)

Compound **41** was prepared according to general procedure 1 **(GP1).** The crude solid mixture was purified by flash column chromatography (DCM/MeOH: 96/4) to afford compound **41** (0.45 g, 0.62 mmol, 53%) as a white solid. ¹H-NMR (600 MHz, CDCl₃): δ (ppm) 7.30-7.07 (m, 19H + CHCl₃), 6.30 (br s, rotamer 1) and 6.06 (br s, rotamer 2, 1H), 6.05 (br s, rotamer 1) and 5.45 (br s, rotamer 2, 1H), 5.14 (br s, 2H), 4.66-4.39 (m, 4H), 4.05 (br s, 2H), 3.67 (br s, 2H), 3.12-2.88 (m, 4H), 1.36-0.81 (m, 15H). ¹³C-NMR (150 MHz, CDCl₃): δ (ppm) 171.3 (2 x CO), 156.4 (CO), 142.1 (C_{q}), 141.8 (C_{q}), 136.4 (C_{q}) 133.8 (C_{q}), 129.0 (4 x CH), 128.9 (CH), 128.6 (CH), 128.4 (CH), 128.3 (CH), 128.0 (2 x CH), 127.9 (6 x CH), 127.3 (CH), 127.2 (CH), 126.4 (CH), 79.5 (C_{q}), 68.2 (CH₂), 65.7 (CH), 56.2 (CH), 53.2 (CH), 50.7 (CH₂), 45.3(CH₂), 44.2 (CH₂), 40.4 (CH₂), 30.6 (CH₂), 29.7 (CH₂), 28.8 (3 x CH₃), 22.5 (CH₂). LC-MS (ESI) *m*/*z* calcd. for C₄₃H₅₁N₄O₆Na [M+Na+H]²⁺, 742.4; found, 742.1. HPLC purity, 94%, t_{R} = 23.0 min, gradient A.

### Tert-butyl ((S)-6-((2,2-diphenylethyl)amino)-6-oxo-5-((S)-1,2,3,4-tetrahydroisoquinoline-3-carboxamido)hexyl)carbamate (42)

Compound **42** was prepared according to general procedure 3 **(GP3),** to afford compound **42** (0.81 g, 1.38 mmol, 100%) as a white solid. No further purification was required. ¹H-NMR (600 MHz, CDCl₃): δ (ppm) 7.44 (d, *J* = 8.4 Hz, 1H), 7.19-7.06 (m, 13H), 6.99-6.97 (m, 1H), 6.18 (t, *J=* 5.7 Hz, 1H), 4.47 (br s, 1H), 4.15-4.11 (m, 2H), 3.91-3.77 (m, 4H), 3.27 (dd, *J* = 5.3 Hz, J = 9.9 Hz, 1H), 3.04 (d, *J =* 5.3 Hz, rotamer 1) and 3.02 (d, *J =* 5.3 Hz, 2H, rotamer 2), 2.93 (br s, 2H), 2.69 (dd, *J* = 9.9 Hz, J = 16.1 Hz, 1H), 1.68-1.64 (m, 1H), 1.42-1.28 (m, 12H), 1.05 (br s, 2H). ¹H-NMR assignment was complex due to presence of rotamers in a 1:1 ratio. ¹³C-NMR (150 MHz, CDCl₃): δ (ppm) 172.5 (CO), 170.3 (CO), 155.0 (CO), 140.7 (C_{q}), 140.6 (C_{q}), 134.8 (C_{q}), 133.0 (C_{q}), 128.0 (CH), 127.7 (4 x CH), 127.0 (4 x CH), 125.8 (2 x CH), 125.7 (CH), 125.3 (CH), 124.6 (CH), 78.1 (C_{q}), 55.1 (CH), 51.5 (CH), 49.6 (CH), 46.1 (CH₂), 42.6 (CH₂), 39.0 (CH₂), 30.3 (CH₂), 29.9 (CH₂), 28.5 (CH₂), 27.4 (3 x CH₃), 21.6 (CH₂). LC-MS (ESI) m/z calcd. for C₃₅H₄₅N₄O₄ [M+H]⁺, 585.3; found, 585.9 [M+H]⁺. HPLC purity, 96%, t_{R} = 21 min, gradient A.

### Tert-butyl ((R)-6-((2,2-diphenylethyl)amino)-6-oxo-5-((S)-1,2,3,4-tetrahydroisoquinoline-3-carboxamido)hexyl)carbamate (43)

Compound **43** was prepared according to general procedure 3 **(GP3),** to afford the titled compound **43** (351 mg, 0.604 mmol, 97%) white solid. ¹H-NMR (600 MHz, CDCl₃): δ (ppm) 7.45 (d, *J =* 8.5 Hz, 1H), 7.17-7.05 (m, 14H + CDCl₃), 6.97 (m, 1H), 6.10 (br s, 1H), 4.48 (br s, 1H), 4.19 (dd, *J =* 8.0 Hz, *J* = 14.2 Hz, 1H), 4.11 (t, *J =* 8.0 Hz, 1H), 3.89 (br s, rotamer 1) and 3.87 (br s, rotamer 2, 1H), 3.81-3.78 (m, 3H), 3.40 (dd, *J =* 5.2 Hz, *J =* 10.6 Hz, 1H), 3.02-2.97 (m, 3H), 2.49 (dd, *J* = 10.6 Hz, *J=* 16.3 Hz, 1H), 1.74-1.70 (m, 1H), 1.48-1.45 (m, 1H), 1.36 (br s, 12H), 1.18-1.13 (m, 2H). ¹H-NMR assignment was complex due to presence of rotamers in a 1:2 ratio. ¹³C-NMR (150 MHz, CDCl₃): δ (ppm) 172.3 (CO), 170.3 (CO), 155.0 (CO), 140.6 (2 x C_{q}), 134.9 (C_{q}), 133.1 (C_{q}), 128.1 (CH), 127.7 (4 x CH), 127.0 (4 x CH), 125.8 (2 x CH), 125.6 (CH) and 125.3 (CH), 124.6 (CH), 78.1 (C_{q}), 55.1 (CH), 51.6 (CH), 49.6 (CH), 46.5 (CH₂), 42.5 (CH₂), 39.1 (CH₂), 30.3 (CH₂), 29.5 (CH₂), 28.6 (CH₂), 27.4 (3 x CH₃), 21.7 (CH₂). LC-MS (ESI) m/z calcd. for C₃₅H₄₅N₄O₄ [M+H]⁺, 585.3; found, 586.1. HPLC purity, 94%, t_{R} = 18.4 min, gradient A.

### Tert-buyl ((S)-6-((2,2-diphenylethyl)amino)-6-oxo-5-((S)-2-(4-oxo-4-phenylbutanoyl)-1,2,3,4-tetrahydroisoquinoline-3-carboxamido)hexyl)carbamate (44)²⁸

Compound **44** was prepared according to general procedure 2 **(GP2)** by using 3-benzoylpropionic acid. The crude solid mixture was purified by flash column chromatography (Hex/EtOAc/EtOH: 4/3/1) to afford compound **44** (237 mg, 0.30 mmol, 94%) as a white solid. ¹H-NMR (600 MHz, CDCl₃): δ (ppm) 7.93-7.84(m, 2H), 7.54-7.49 (m, 1H), 7.43-7.37 (m, 2H), 7.20-6.98 (m, 15H + CDCl₃), 6.64 (d, *J* = 8.0 Hz, rotamer 1), and 6.49 (d, *J =* 8.0 Hz, rotamer 2, 1H), 6.3 (t, *J =* 5.4 Hz, rotamer 1) and 6.2 (t, *J =* 5.4 Hz, rotamer 2, 1H), 4.92-4.86 (m, 2H), 4.67-4.38 (m, 2H), 4.03-3.82 (m, 2H), 3.64-3.52 (m, 2H), 3.33-3.18 (m, 2H), 3.07-2.81 (m, 4H), 2.50-2.36 (m, 1H), 1.73-1.67 (m, 1H), 1.44-1.32 (m, 10H), 1.19-1.07 (m, 2H), 0.81-0.58 (m, 2H). ¹³C-NMR (150 MHz, CDCl₃)**:** δ (ppm) 199 .5 (CO), 172.7 (CO), 169.4 (CO), 154.9 (CO), 141.0 (2 x C_{q}), 134.9 (C_{q}) 133.1 (C_{q}), 132.8 (C_{q}), 127.8 (CH), 127.7 (CH), 127.5 (2 x CH), 127.4 (CH), 127.2 (2 x CH), 127.1 (CH), 127.0 (2 x CH) 126.9 (CH), 126.4 (CH), 126.3 (CH), 126.1 (CH), 125.6 (CH) 125.5 (CH), 125.4 (CH), 125.0 (CH), 78.0 (C_{q}), 55.8 (CH), 53.6 (CH), 52.7 (CH), 52.0 (CH₂), 49.2 (CH₂), 45.7 (CH₂), 43.4 (CH₂), 42.8 (CH₂), 33.6 (CH₂), 28.1 (CH₂) 27.5 (3 x CH₃), 26.2 (CH₂), 21.6 (CH₂). HRMS (ESI) m/z calcd for C₄₅H₅₂N₄O₆Na [M+Na]⁺, 767.3779; found, 767.3792. LC-MS (ESI) m/z calcd. for C₄₅H₅₄N₄O₆ [M+2H]²⁺, 746.4; found, 746.4. HPLC purity, 98%, t_{R} = 21.9 min, gradient A.

### Tert-butyl ((R)-6-((2,2-diphenylethyl)amino)-6-oxo-5-((S)-2-(4-oxo-4-phenylbutanoyl)-1,2,3,4-tetrahydroisoquinoline-3-carboxamido)hexyl)carbamate (45)

Compound **45** was prepared according to general procedure 2 **(GP2)** by using 3-benzoylpropionic acid. The crude solid mixture was purified twice by flash column chromatography (Hex/EtOAc/EtOH: 7/3/1) to afford compound **45** (90 mg, 0.12 mmol, 71%) as a white solid. ¹H-NMR (600 MHz, CDCl₃): δ (ppm) 7.94-7.90 (m, 2H), 7.52 (m, 1H), 7.42-7.38 (m, 2H), 7.19-7.06 (m, 14H + CDCl₃), 6.99 (d, *J* = 7.4 Hz, 1H), 6.60 (d, *J=* 8.3 Hz, 1H), 5.96 (br s, 1H), 5.09 (t, *J* = 5.0 Hz, 1H), 4.76-4.40 (m, 2H), 4.12-3.96 (m, 2H), 3.64-3.47 (m, 2H), 3.04-2.66 (m, 4H), 2.56-2.51 (m, 1H), 1.71-1.66 (m, 1H), 1.39-1.32 (m, 10H), 1.27-1.14 (m, 2H), 1.05-0.90 (m, 2H). ¹³C-NMR (150 MHz, CDCl₃): δ (ppm) 199.5 (CO), 172.7 (CO), 169.4 (CO), 154.9 (CO), 141.0 (2 x Cq), 134.9 (C_{q}) 133.1 (C_{q}), 132.8 (C_{q}), 127.8 (CH), 127.7 (CH), 127.5 (2 x CH), 127.4 (CH), 127.2 (2 x CH), 127.1 (CH), 127.0 (2 x CH) 126.9 (CH), 126.4 (CH), 126.3 (CH), 126.1 (CH), 125.6 (CH) 125.5 (CH), 125.4 (CH), 125.0 (CH), 78.0 (C_{q}), 55.8 (CH), 53.6 (CH), 52.0 (CH), 49.2 (CH₂), 45.7 (CH₂), 43.4 (CH₂), 42.8 (CH₂), 33.6 (CH₂), 29.8 (CH₂), 29.4 (CH₂), 27.5 (3 x CH₃), 26.2 (CH₂), 21.6 (CH₂). HRMS (ESI) m/z calcd for C₄₅H₅₂N₄O₆Na [M+Na]⁺, 767.3779; found, 767.3781. LC-MS (ESI) m/z calcd. for C₄₅H₅₃N₄O₆Na [M+Na+H]²⁺, 768.4; found, 768.3. HPLC purity, 98 %, t_{R} = 21.8 min, gradient A.

### (S)-6-((2,2-diphenylethyl)amino)-6-oxo-5-((S)-2-(4-oxo-4-phenylbutanoyl)-1,2,3,4-tetrahydroisoquinoline-3-carboxamido)hexan-1-aminium 2,2,2-trifluoroacetate (VUF10661, 46)²⁸

Compound **46** was prepared according to general procedure 4 **(GP4).** The crude solid mixture was purified by preparative HPLC employing method B (t_{R} = 12 min) to afford compound **46** as TFA salt (35 mg, 0.047 mmol, 69 %). ¹H-NMR (600 MHz, DMSO-*d₆*, 400K): δ (ppm) 8.01-7.99 (m, 2H), 7.65-7.64 (m, 1H), 7.56-7.54 (m, 4H), 7.45 (m, 2H), 7.32-7.19 (m, 15H), 4.94 (t, *J =* 5.2 Hz, 1H), 4.84 (d, *J =* 15.7 Hz, 1H), 4.65 (br s, 1H), 4.22 (t, *J* = 7.7 Hz, 1H), 4.09 (dd, *J =* 7.9 Hz, *J* = 13.7 Hz, 1H), 3.80 (ddd, *J* = 5.4 Hz, *J =* 7.5 Hz, *J =* 13.1 Hz, 1H), 3.65 (ddd, *J =* 6.2 Hz, *J* = 7.9 Hz, *J =* 13.8 Hz, 1H), 3.35 (t, *J* = 6.5 Hz, 2H), 3.15 (br s, 2H), 2.83 (br s, 2H), 2.70 (dt, *J=* 2.1 Hz, *J* = 8.2 Hz, 2H), 1.53-1.47 (m, 1H), 1.45-1.40 (m, 2H), 1.37-1.31 (m, 1H), 1.05-0.97 (m, 2H). ¹³C-NMR (150 MHz, DMSO-*d₆*, 350 K): δ (ppm) 199.5 (CO), 171.5 (2 x CO), 143.1 (C_{q}), 143.0 (C_{q}), 137.4 (C_{q}), 133.4 (C_{q}), 129.2 (CH), 128.9 (2 x CH), 128.8 (4 x CH), 128.4 (2 x CH), 128.3 (6 x CH), 128.2 (CH), 126.9 (CH), 126.8 (CH), 126.6 (CH), 50.5 (CH), 43.7 (CH₂), 39.3 (CH₂), 33.9 (CH₂), 31.5 (CH₂), 28.1 (CH₂), 27.0 (CH₂), 22.1 (CH₂). One C_{q}, two CH and two CH₂ carbons were not detected due to peak broadening caused by isomerism. HRMS (ESI) m/z calcd for C₄₀H₄₅N₄O₄ [M+H]⁺, 645.3435; found, 645.3434. LC-MS (ESI) *m*/*z* calcd. for C₄₂H₄₇N₄O₄ [M+2H]²⁺ 646.3; found, 645.9. HPLC purity, 100 %, t_{R} = 18 min, gradient A.

### (R)-6-((2,2-diphenylethyl)amino)-6-oxo-5-((S)-2-(4-oxo-4-phenylbutanoyl)-1,2,3,4-tetrahydroisoquinoline-3-carboxamido)hexan-1-aminium 2,2,2-trifluoroacetate (47)

Compound **47** was prepared according to general procedure 4 **(GP4).** The crude solid mixture was purified by preparative HPLC employing method C (t_{R} = 15.3 min) to afford compound **47** as TFA salt (25 mg, 0.038 mmol, 76%). ¹H-NMR (600 MHz, DMSO-*d₆*, 400 K): δ (ppm) 7.94-7.93(m, 2H), 7.58 (t, *J =* 7.4 Hz, 1H), 7.49-7.47 (m, 4H), 7.34 (m, 1H), 7.25-7.14 (m, 15H), 4.91 (t, *J =* 5.2 Hz, 1H), 4.82 (d, *J =* 15.7 Hz, 1H), 4.53 (br s, 1H), 4.16 (t, *J* = 7.7 Hz, 1H), 4.03 (dd, *J* = 7.9 Hz, *J* = 13.7 Hz, 1H), 3.70 (ddd, *J* = 5.4 Hz, *J* = 7.5 Hz, *J* = 13.1 Hz, 1H), 3.61 (ddd, *J =* 6.2 Hz, *J =* 7.9 Hz, *J =* 13.8 Hz, 1H), 3.29 (t, *J =* 6.5 Hz, 2H), 3.10 (br s, 2H), 2.85-2.64 (m, 4H + H₂O), 1.51-1.46 (m, 1H), 1.42-1.37 (2H), 1.33 (m, 1H), 1.10-1.04 (m, 2H). ¹³C-NMR (150 MHz, DMSO-*d₆*, 350 K): δ (ppm) 199.8 (CO), 172.4 (CO), 172.1 (CO), 171.7 (CO) 143.6 (C_{q}), 143.1 (C_{q}), 137.6 (C_{q}), 135.0 (C_{q}), 134.5 (CH), 134.0 (C_{q}), 129.6 (CH), 129.3 (2 x CH), 129.2 (CH), 128.7 (10 x CH), 127.8 (CH), 127.4 (3 x CH), 126.9 (CH), 55.6 (CH), 54.2 (CH), 53.1 (CH), 50.1 (CH₂), 46.3 (CH₂), 39.5 (CH₂), 34.1 (CH₂), 32.1 (CH₂), 28.4 (CH₂), 27.5 (CH₂), 22.8 (CH₂). One CH₂ carbon was not detected due to peak broadening caused by isomerism. HRMS (ESI) m/z calcd for C₄₀H₄₅N₄O₄ [M+H]⁺, 645.3435; found, 645.3431. LC-MS (ESI) *m*/*z* calcd. for C₄₂H₄₇N₄O₄ [M+2H]²⁺ 646.3; found, 646.1. HPLC purity, 100%, t_{R} = 18min, gradient A.

### Biological characterization.

**Membrane preparation for allosteric radioligand RAMX3 displacement and [³⁵S]GTPγS incorporation assays:** The human embryonic kidney (HEK) cells stably expressing the human CXCR3 receptor were cultured in 150-mm cell culture plates in DMEM/F-12 supplemented with 10% fetal bovine serum (FBS), 2 mM L-glutamine, 1% penicillin-streptomycin, 300 µgmL⁻¹ Zeocin and incubated at 37 °C in a humid atmosphere with 5% CO₂. At 100% confluency cells were washed with PBS twice, treated with Tris EDTA buffer (10 mM Tris, 0.5 mM EDTA, 5 mM KCl, 140 mM NaCl, pH 7.4), and harvested using a cell scraper. Cells were pelleted at 1100×g for 8 min at 4 °C, resuspended in Tris-EDTA-MgCl₂ buffer (50mM Tris, 5 mM EDTA, 1.5 mM CaCl₂, 5 mM MgCl₂, 5 mM KCl, 120 mM NaCl, pH 7.4) and followed by lysis with an Ultra-Turrax. After centrifugation at 50,000•g, at 4°C for 18 min the membranes were resuspended in the binding buffer (50 mM Tris, 1 mM EDTA, 5 mM MgCl₂ and subsequently homogenized with a glass-Teflon homogenizer (20 strokes). The homogenized membranes were shock-frozen in liquid nitrogen and stored at -80°C. The protein concentration was determined with the Lowry method with bovine serum albumin used as a standard.³⁵

**Allosteric radioligand displacement assay:** Receptor binding studies were performed on membrane preparations of HEK cells expressing CXCR3. The tritium labeled RAMX3 (specific activity: 80.4 Ci/mmol) at the concentration of 1 nM was used for the assays. To determine unspecific binding 5µM of NBI-74330 was used. The assays were carried out in 96 well plates at a protein concentration of 30 µgmL⁻¹ in a total volume of 200 µL. The incubation buffer contained 20 mM HEPES, 10 mM MgCl₂, 100 mM NaCl and 0.1% BSA. (pH 7.4). After incubating for 1 h at 37°C, the binding was stopped by filtration through Whatman GF/B filters using a 96-channel cell harvester (Brandel, Unterföhring). The filters were rinsed five times with ice cold Tris-NaCl buffer. After drying for 3h at 60°C, filters were sealed with melt-on scintillator sheets Melti-Lex G/HS and the trapped radioactivity was measured in a microplate scintillation counter (Micro Beta Trilux scintillator). Three to five experiments per compound were performed with each concentration in triplicate. The *Kd* and *Bmax* values were determined in the homologous competition assay and have yielded following results: the *Kd* and *Bmax* values for the CXCR3 were 0.69 ± 0.13 and 5162 ± 367 fmol•mg⁻¹.

**[¹²⁵I]CXCL11 radioligand displacement assay:** *Oxidative iodination.* The CXCL11 protein (purchased from PeproTech as the mature form of isoform 1) was labeled in-house with the ¹²⁵I isotope (purchased from Perkin-Elmer, in 10 µM NaOH, pH 8-11) using the Chloramine-T method³⁶. CXCL11 was dissolved in 300 mM phosphate buffer, and 0.4 mCi ¹²⁵I was added to 0.6 nmol (10 µl) of the protein. 30 µl phosphate buffer supplemented with 30 mgl⁻¹ Chloramine-T was added slowly over a 5 min period while stirring, and the reaction was quenched by adding 400 µl H₂O with 0.1% trifluoroacetic acid (TFA). The product was purified by reverse-phase HPLC, using a C18 column and a gradient from H₂O with 0.1% TFA (buffer A) to acetonitrile with 0.1% TFA (buffer B). After allowing the passage of 8 mL flow-through at 20 % buffer B, CXCL11 is eluted by increasing buffer B to 50 % over a flow-volume of 40 mL, after which any possible remains of protein are eluted by further increasing buffer B to 80 % across 10 mL flow-through. The flow-through is collected in 1 mL aliquots, and the radioligand is identified in those aliquots by gamma radiation intensity, and verified with a preliminary competitive binding assay setup.

*Cell culture and transfection,.* Cos-7 cells expressing human CXCR3 (isoform 1) were used for binding assays. The cells were grown in DMEM 1885 (containing NaHCO₃) supplemented with 10% FBS, 0.029% L-Glutamine, 180 unitsml⁻¹ penicillin and 45 µgml⁻¹ streptomycin in tissue culture treated flasks (T75) to approximately 75% confluence. The calcium phosphate precipitation method³⁷ was used to transiently transfect the COS-7 cells before every experiment. 20 µg expression vector DNA (pcDNA3.1⁺ encoding CXCR3 under control of the CMV promoter, or empty vector for negative controls) in 210 µl TE-buffer was mixed with 30 µl CaCl₂, and this was titrated into 240 µl HEPES buffered saline. The mixture was dripped directly unto the cells after a 45 min equilibration period, and the cells were incubated for 5 h in growth medium containing 30 mL/L chloroquine, after which it was exchanged for regular growth medium.

*Competitive radioligand binding.* Assay points were performed on transiently transfected COS-7 cells seeded one day earlier at 5.000 cells/well in poly-D-Lysine treated white 96-well plates. The cells were washed twice in 100 µl 50 mM HEPES buffer supplemented with 5 gL⁻¹ BSA, then chilled to 4 °C for 15 min before addition of ligands, followed by addition of 10-15 pM radioligand aiming at 10% specific binding. Cells were incubated for 3 h at 4°C before being washed twice in 50 mM HEPES buffer with 5 gL⁻¹ BSA and 29.22 gL⁻¹ NaCl. 80 µl Gold Star (Liquid Scintillation Cocktail, Meridian, UK) was added per well before measuring remaining receptor-bound radioligand as scintillation on a Packard TopCount NXTtm.

**ApoTox-Glo Triplex Assay:** The ApoTox-Glo Triplex Assay (Promega) was performed according to the manufacturer's instructions. Briefly, 10 000 HEK293T cells stably expressing CXCR3 were seeded in a half-area white clear bottom 96-well plate and incubated overnight with compounds at the concentration of 10 µM in a final volume of 50 µL per well. After desired incubation time the Viability/cytotoxicity reagent containing both GF-AFC and bis-AAF-R119 substrate were added to the well and incubated for additional 30 min. The fluorescence was measured at 400_{Ex}/505_{Em} (Viability) and 485_{Ex}/520_{Em} (Toxicity) with a microplate reader Clariostar (BMG labtech, Ortenberg, Germany). After the measurement the Caspase-Glo 3/7 reagent was added to all wells, incubated for 30 min and the luminescence measured with the microplate reader. The increase in the luminescence is indicative of caspase activation and thus a hallmark of apoptosis.

[³⁵**S]GTPγS incorporation assay:** The [³⁵S]GTPγS incorporation assay was performed on membrane preparations of stably transfected HEK293 cells that expressed the CXCR3 receptor. The assay was carried out in 96-well plates at the final volume of 200 µL. The incubation buffer contained 20 mM HEPES, 10 mM MgCl₂•6H₂0,100 mM NaCl and 100 mg/L saponin (pH 7.4). Membranes (30 µgmL⁻¹ of membrane protein), various concentrations of test compound and CXCL11, and 1 µM GDP were preincubated in the absence of [³⁵S]GTPγS for 30 min at 37 °C. After the addition of 0.10 nM [³⁵S]GTPγS membranes were incubated for additional 30 min at 37 °C. Incubation was terminated by filtration through Whatman GF/B filters soaked with ice cold PBS. The filter-bound radioactivity was measured as described above. Three to four experiments per compound were performed with each concentration in triplicate.

**β-arrestin 2 recruitment assay:** The measurement of β-arrestin recruitment was performed utilizing the PathHunter assay purchased from DiscoveRx (DiscoveRx, Birmingham, U.K.) according to the manufacturer's protocol. HEK293 cells stably expressing β-gal enzyme acceptor were transiently transfected with the ProLink (PK1) tagged wildtype CXCR3 receptor using the TransIT-293 transfection reagent from Mirus (purchased from MoBiTec, Goettingen, Germany). The assay was carried out in 384-well plates containing 2000 cells in 20 µL. After incubation at 37°C (5% CO₂, 95% relative humidity) overnight, various concentrations of test compounds or CXCL11 (prepared in PBS, pH 7.4, containing 0.20% BSA) were added and the incubation continued for 4 h. Detection mix was then added and incubated for further 60 min at room temperature. Chemiluminescence was determined with a microplate reader Clariostar (BMG labtech, Ortenberg, Germany).

**Receptor internalization imaging:** The day before transfection HEK293T cells were seeded in 6-well plates at a density of 200,000 cells/well. Transient transfection of the C-terminally eGFP-tagged CXCR3 cDNA was performed using TransIT293(MIRUS). Cells were transferred to Poly-L-Lysine coated glass coverslips 24 h after transfection. 2 d after transfection, cells were washed with phosphate buffered saline (PBS) and treated with the endogenous chemokine CXCL11 or compound FAUC1036 at 37°C in 5% CO2 for 60min. Cells were fixed with 4% paraformaldehyde (RotiHistofix, Carl Roth) for 10min. After washing three times with PBS, the glass coverslips were mounted on microscope slides using Dako Fluorescent Mounting Medium and investigated using a Leica SP5 II confocal laser scanning microscope (Software LAS AF v2.7.3.9723.) equipped with Leica hybrid detectors. Excitation energy in all experiments was set to the same level to make all data set-ups comparable in intensity. Post image processing (adjusting brightness and contrast) was performed for a better visualization.

**Enzyme-linked immunosorbent assay based internalization assay.** The day after transfection with FLAG-CXCR3, cells were trypsinized, resuspended into fresh culture medium and plated in poly(D-lysine)-coated 48-well assay plates. 48h after transfection the medium was replaced by fresh DMEM w/o serum containing one concentration of the endogenous ligand CXCL11 (100 nM) or the positive allosteric modulators (10 µM) and incubated for 1h at 37°C. The cells were fixed with 4% formaldehyde solution and stained with monoclonal Anti-Flag antibody (Sigma-Aldrich Life Science, St. Louis, MO) and subsequently with Anti-Mouse IgG-Peroxidase Antibody produced in rabbit. *O-*phenylendiamine was used as a substrate for the enzyme coupled to the secondary antibody. The reaction was stopped with 1M H₂SO₄ and the resulting color was detected at 492 nm using a microplate reader Clariostar (BMG labtech, Ortenberg, Germany).

**Transwell migration assay.** HEK-293 cells were maintained in DMEM medium supplemented with 10% FBS and 1% penicillin/streptomycin. For DNA transfection, 5 µg plasmid DNA (CXCR3) was mixed with TE buffer to give a final of 105 µl solution and 15 µl CaCl₂ (2M) was added. The DNA mixture was then dripped into 120 µl 2x HBSS solution, and the mixture was allowed to form calcium-phosphate precipitates for 45 minutes. The DNA/calcium-phosphate precipitate was dripped onto HEK293 cells (50% confluent in T25 flask), where the medium had been renewed and reduced to 1.5 ml 30 minutes prior to transfection. The cells were split and used for assays 48 h after transfection.

Migration of serum-starved HEK293 cell transiently transfected with CXCR3 was assessed using Transwell™ membranes (Costar; 8-µm pore). Filters were coated with 10 µg•mL⁻¹ fibronectin (in PBS 30 minutes at room temperature). The fibronectin solution was removed and the filters allowed to air-dry. The filters were placed in a 24-well dish that contained low serum (0.2%) DMEM supplemented with agonists or control buffer. HEK293 cells suspended in 0.2% serum DMEM containing test compounds were added to the upper chamber (1 × 10⁵ cells/well). Cells were allowed to migrate for 5 h at 37 °C. Nonmigrated cells were removed from the top filter surface with a cotton swab. Migrated cells, attached to the bottom surface, were fixed in 3.7% formaldehyde (in PBS) and dyed with crystal violet. Transmigrated cells were counted in 5 predefined areas on the membrane using 20X objective.

**Data analysis.** The resulting competition curves of the allosteric radioligand displacement and activity assays were analyzed by nonlinear regression using the algorithms in PRISM 5.0 (GraphPad Software, San Diego, CA). Competition curves were fitted to the sigmoid curve by non-linear regression analysis in which the logEC₅₀ value and the Hill coefficient were free parameters.

### Example 2

### Characterization of compounds according to the present invention

### Results and Discussion

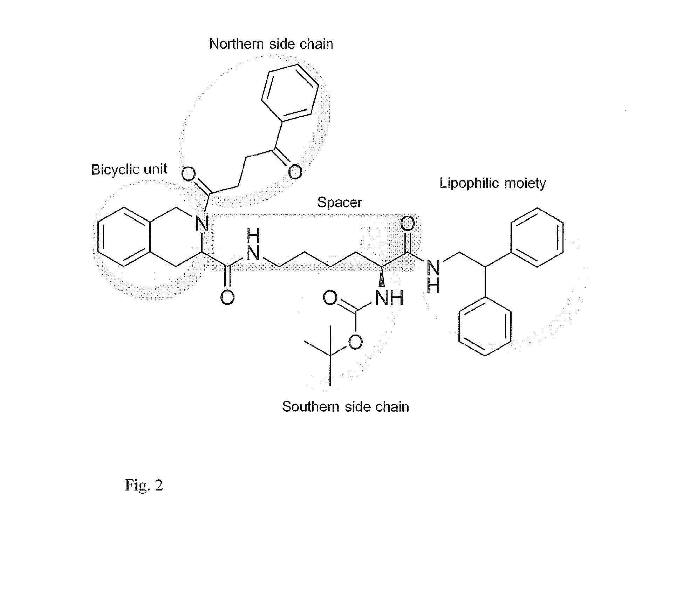

Based on FAUC1036 (= compound 11), strongly biased allosteric agonist, the inventors synthesized a series of derivatives focused on head group of the northern fragment and its spacer (Figure 2) to further probe unique mechanisms of allosteric modulation driven by this ligand. The inventors combined this approach with detailed pharmacological characterization of novel compounds that allowed to gain novel insight into the structure-activity relationships for the fine control of allosteric modulator potency, cooperativity and biased signaling at CXCR3.

### Synthesis of allosteric ligands

Typical peptide synthesis strategy was followed to develop peptidomimetic ligands. The preparation of the FAUC1036 (Figure 2) began with an acylation step between Boc-Lys(Z)-OH and biphenyl ethylamine in presence of EDC hydrocloride (compound **1**), followed by hydrogenolysis to afford the primary amine **2.** Then, Cbz protection of the commercially available (*S*)-2-((benzyloxy)carbonyl)-1,2,3,4-tetrahydroisoquinoline-3-carboxylic acid using benzyl chloroformate in basic medium (compound 3) followed by an amide coupling step using **2** to generate compound **4.** A second hydrogenolysis yielded compound **5,** a precursor ready to be coupled to the northern side chain in one step (Scheme 1).

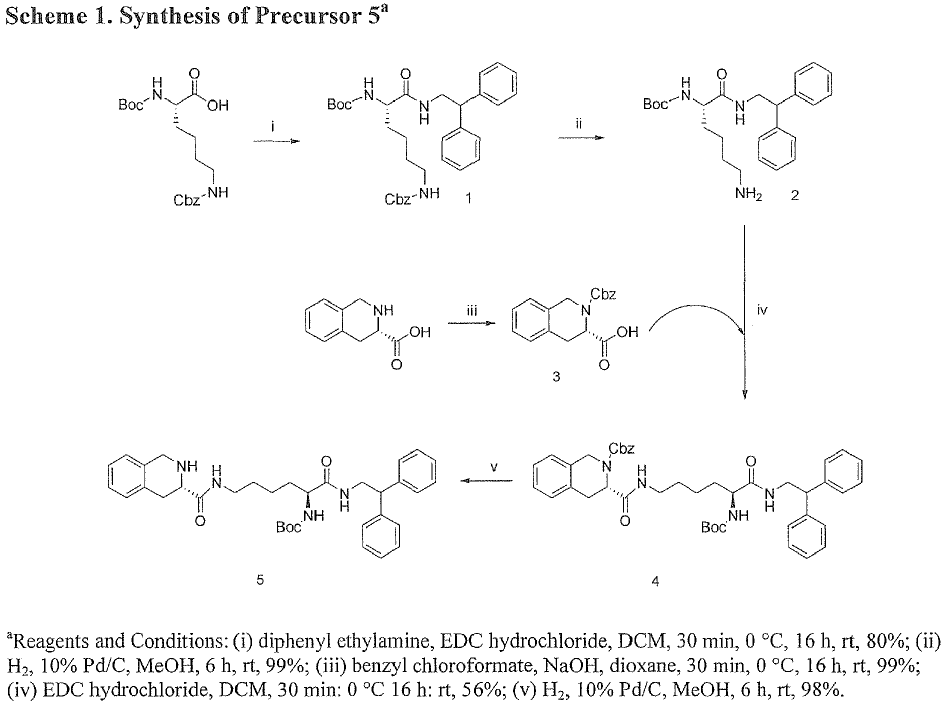

For the preparation of the northern side and its variations non-aromatic heterocyclic acids **6-8** were synthesized by acylating piperidine, morpholine and *N*-methyl piperazine with succinic anhydride at room temperature^{25,26}. The aromatic heterocyclic acids **9, 10** were obtained by a Friedel Crafts acylation of furan and thiophene with succinic anhydride as well^{27,28} (Scheme 2). The remaining carboxylic acids were commercially available. All acids were coupled to **5** using EDC hydrochloride and DIPEA to afford the final compounds **11**/FAUC1036 - **24** (Scheme 3). Compound **27** was produced by acylating succinic anhydride with **5.** The Boc group of compounds **11**/FAUC1036, **24** and 5 was removed with TFA to yield the protonated compounds **25, 26, 28** respectively.

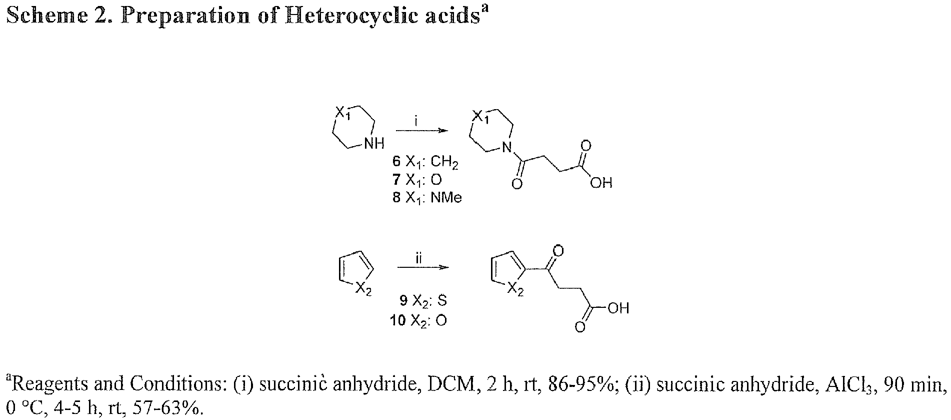

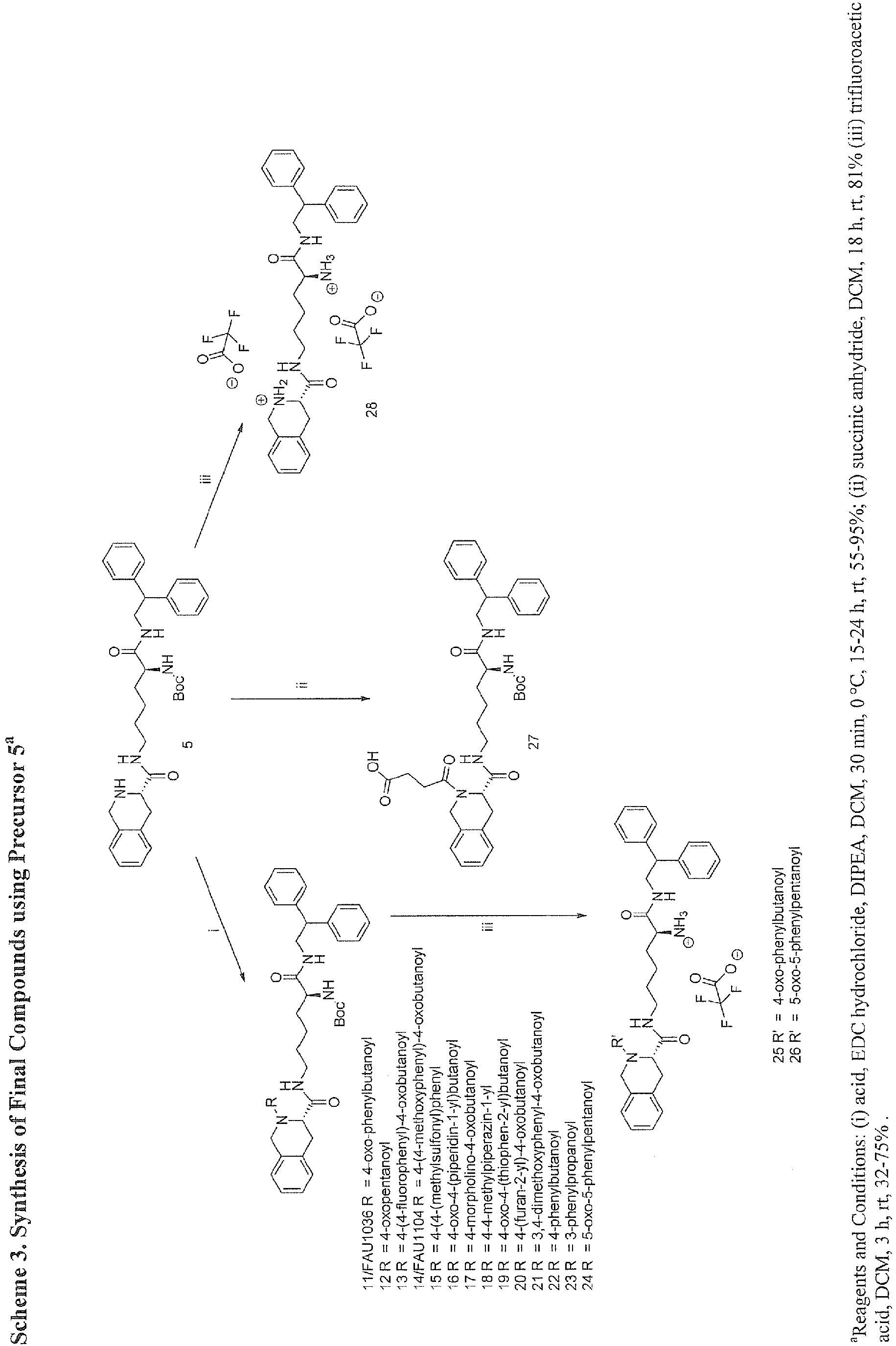

Furthermore, S-configured-lysine moiety of FAUC1036 and **25** was replaced with the R-configured one resulting in the compounds **34**/FAUC1095 and **35.** The synthetic strategy of **29-35** was identical to that of FAUC1036 (Supplemental Scheme 1).

To enable appropriate comparison of reference compound **46**(S,S)/VUF10661 with the novel ligands, the inventors developed modulators by modifying the stereogenic center of lysine moiety **47**(S,R) or using a Boc group for both epimers **44**(S,S) and **45**(S,R), in order to investigate the biological activity and to replace the positive charge of the lysine side chain, which was reported to be crucial for receptor activation^{17,18}, with a bulky Boc group. For **46**/VUF10661 and its analogues, the inventors followed similar synthetic strategy to the one reported above, which gave significantly higher yields than the ones found in the literature¹⁸. Either the enantiopure Z-Lys(Boc)-OH or D- Z-Lys(Boc)-OH were used in the synthesis.

### Pharmacological characterization

The binding of endogenous agonists CXCL11, CXCL10 and CXCL9 to CXCR3 results in the activation of Gαi proteins, recruitment of β-arrestins, receptor internalization and chemotaxis. Signaling through Gαi has been shown to activate CXCR3 induced migration through multiple pathways, including p38 and PI3K in human epithelial airway cells²⁹ and PI3K and PTEN in human neutrophils³⁰. No direct studies on CXCR3 induced β-arrestin mediated migration has been published, but it is possible that CXCR3 chemokines convey some of their migratory stimulation through β-arrestin induced signaling, as reported for CXCL11 at the decoy receptor CXCR7 and for angiotensin II at the angiotensin II type la receptor^{31,32}. To elucidate the pharmacological properties of derivatives of FAUC1036, the inventors focused on the ability of derivatives to modulate G protein activation and β-arrestin 2 recruitment. For the most prominent derivatives FAUC1036 and FAUC1104 their capacity to induce CXCR3 receptor internalization and chemotaxis was investigated in detail. Prior to functional characterization the potential cytotoxicity of derivatives was determined with ApoTox-Glo Triplex assay (Promega), which easily assesses viability, cytotoxicity and apoptosis events in the same cell-based assay. None of derivatives exhibited unspecific effect on the cells.

The activation of G proteins was explored in terms of CXCR3 mediated [³⁵S]GTPγS incorporation. In the [³⁵S]GTPγS incorporation assay the membrane preparations of HEK293T cells stably expressing CXCR3 were used. The activation of G proteins was monitored as the incorporation of [³⁵S]GTPγS and thus increase in the incorporated radioactivity. The potency and efficacy of the novel compounds was first compared to the reference compound **46**/VUF10661. Compound **46**/VUF10661 produced a dose-dependent increase in the [³⁵S]GTPγS incorporation with a potency of 0.6 µM (*pEC₅₀=* 6.2 ± 0.1), which is in accordance with the published data (Table 1)¹⁸. The epimer of **46**/VUF10661 compound **47** as well as the Boc protected derivatives **44** and **45** showed no significant changes in potency and efficacy. Although it was reported that the charge at the amine on the side chain of the lysine moiety was crucial for the biological activity of **46**/VUF10661^{17,18}, the inventors clearly demonstrate that this charge is fully dispensable for the activation of G proteins by the allosteric CXCR3 agonists according to the present invention.

**Table 1. The ability of the 46/VUF10661 based positive allosteric modulators to activate CXCR3 as measured in the [³⁵S]GTPγS incorporation assay.^{[a]}**

| | | | | |
|---|---|---|---|---|
| 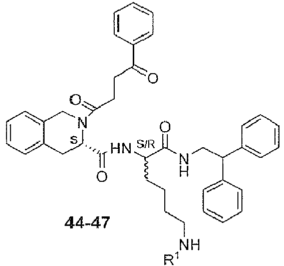 | | | | |

| **Cpd** | **Stereo conf.** | **R¹** | **CXCR3** | |
|---|---|---|---|---|
| | | | ***pEC₅₀* ± SEM^{[b]}** | **Efficacy (%) ± SEM^{[c]}** |
| **CXCL11** | - | - | 8.88±0.13 | 100 |
| **44** | S,S | Boc | 5.88±0.10 | 47±3 |
| **45** | S,R | Boc | 5.97±0.34 | 33±7 |
| **46/VUF10661** | S,S | H | 6.20±0.06 | 109±3 |
| **47** | S,R | H | 5.94±0.09 | 85±5 |

| | | | | |
|---|---|---|---|---|
| ^{[a]} The ability of positive allosteric modulators to activate CXCR3 was determined by the [³⁵S]GTPγS incorporation assay with the membrane preparations of HEK293T stably expressing CXCR3.^{[b]} The values reflect the mean *pEC₅₀*±SEM of three experiments performed in triplicate.^{[c]} The efficacy represents the % of CXCL11 mediated effect. | | | | |

Strikingly, compound **11**/FAUC1036 failed to induce CXCR3 mediated G protein activation, just as its unprotected form **25** and its epimer **34**/FAUC1095 (Table 2). The epimer of **35** compound **35** showed some weak partial agonism at *pEC₅₀* of 8.01±0.31. Compound **22,** where the keto group of the benzoyl moiety was removed, showed very low potency (*pEC₅₀* = 5.07±0.50) as well. To determine the influence of substitution pattern using polar groups at the *para* position of the phenyl ring on the northern side chain, fluorine **(13)** (weak activator), methoxy (**14**/FAU 1104) (strong activator) and methyl sulfonyl **(15)** (strong deactivator) groups were introduced. This modification resulted in an increase of the intrinsic agonist properties of compounds. The introduction of methoxy group (**14**/FAUC1104) caused the most significant increase in the efficacy (50%; the *pEC₅₀* value of 5.22±0.31). In order to increase the electron density near the receptor, an addition of a second methoxy group at the *meta* position **(21)** caused a surprising reduction of the efficacy (18%), which indicates that the activity is not dependent on the electron density of the phenyl ring. Therefore, the inventors removed the aromaticity by replacing the phenyl ring of the northern side chain with a piperidine moiety **(16),** however no reactivity was observed. Knowing that the polar substitution of the phenyl ring increase the reactivity of the ligands in G protein activation pathway, the inventors introduced a polar morpholine **(17)** and a charged *N*-methyl piperazine moiety **(18),** in order to improve the reactivity of the compound **16.** The results showed that non-aromatic heterocycles have no effect in [³⁵S]GTPγS assay. Similar results were obtained for the aromatic heterocycles **19** and **20,** which might indicate that the length of the northern side chain is too short for creating a possible hydrogen bond/s with the receptor compared to **14**/FAUC1104. The inventors therefor generated analogues **5, 23** and **24** in which the length of a spacer of the northern side chain was varied. Compound **5** showed the most interesting results, where the efficacy in the [³⁵S]GTPγS incorporation increased to 34% with the *EC₅₀* value of 2.2 µM (*pEC₅₀* = 5.7 ± 0.34) indicating the importance of the specific side chain for keeping the G protein inactive. Finally, Boc-free compounds **26** and **28** with modified spacer of the northern side chain showed no significant changes compared to their Boc analogues **24** and **5,** respectively, showing that the Boc group or the positive charge are not crucial for the G protein activation.

Importantly, all novel ligands that showed no intrinsic agonist activity were tested for the ability to inhibit the CXCL11 or CXCL10 mediated activation of G proteins. The compounds had no inhibitory of stimulatory effect on the endogenous agonists in this pathway.

**Table 2. The ability of the compound 5 based positive allosteric modulators to activate CXCR3 as measured in the [³⁵S]GTPγS incorporation assay.^{[a]}**

| | | | | | | | | | | | |
|---|---|---|---|---|---|---|---|---|---|---|---|
| 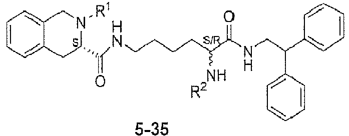 | | | | | | | | | | | |

| **Cpd** | **R¹** | **R²** | **Stereo conf.** | **CXCR3** | | **Cpd** | **R¹** | **R²** | **Stereo conf.** | **CXCR3** | |
|---|---|---|---|---|---|---|---|---|---|---|---|
| | | | | ***pEC₅₀* ± SEM^{[b]}** | **Efficacy (%) ± SEM^{[c]}** | | | | | ***pEC₅₀* ± SEM^{[b]}** | **Efficacy (%) ± SEM^{[c]}** |
| **CXCL11** | | | | 8.88±0.13 | 100 | **20** | 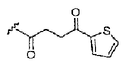 | Boc | S,S | No effect | No effect |
| **5** | H | Boc | S,S | 5.66±0.34 | 34±8 | **21** | 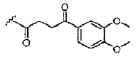 | Boc | S,S | 7.30±0.17 | 18±1 |
| **11/FAUC1036** | 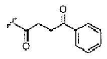 | Boc | S,S | No effect | No effect | **22** | 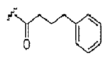 | Boc | S,S | 5.07±0.50 | 29±15 |
| **12** | 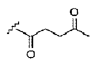 | Boc | S,S | No effect | No effect | **23** | 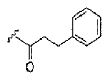 | Boc | S,S | 5.07±0.50 | 30±17 |
| **13** | 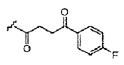 | Boc | S,S | 6.29±0.31 | 20±3 | **24** | 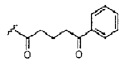 | Boc | S,S | No effect | No efffect |
| **14/FAUC1104** | 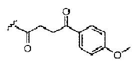 | Boc | S,S | 5.22±0.31 | 50±19 | **25** | 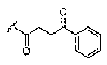 | H | S,S | No effect | No effect |
| **15** | 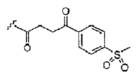 | Boc | S,S | 5.91±0.20 | 25±3 | **26** | 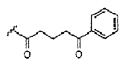 | H | S,S | No effect | No effect |
| **16** | 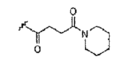 | Boc | S,S | No effect | No effect | **27** | 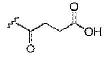 | Boc | S,S | No effect | No effect |
| **17** | 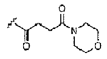 | Boc | S,S | No effect | No effect | **28** | H | H | S,S | 5.65±0.23 | 40±7 |
| **18** | 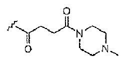 | Boc | S,S | No effect | No effect | **34/FAUC1095** | 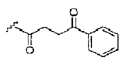 | Boc | S,R | No effect | No effect |
| **19** | 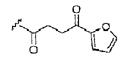 | Boc | S,S | No effect | No effect | **35** | 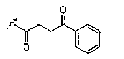 | H | S,R | 8.01±0.31 | 25±4 |

| | | | | | | | | | | | |
|---|---|---|---|---|---|---|---|---|---|---|---|
| ^{[a]} The ability of positive allosteric modulators to activate CXCR3 was determined by the [³⁵S]GTPγS incorporation assay with membrane preparations of HEK293T stably expressing CXCR3.^{[b]} The values reflect the mean *pEC₅₀*±SEM of three experiments performed in triplicate.^{[c]} The efficacy represents the % of CXCL11 mediated effect. | | | | | | | | | | | |

The ability of the novel ligands according to the present invention to induce CXCR3-mediated β-arrestin 2 recruitment was investigated in the assay system PathHunter (DiscoveRx), which detects the recruitment of β-amestin 2 to the activated receptor using β-galactosidase (β-gal) enzyme fragment complementation. The inventors used two different ProLink tags, ProLink1 (PK1) and ProLink2 (PK2), of which PK2 has higher affinity for the enzyme acceptor to detect weak protein-protein interactions. The CXCR3-PK2 construct is readily able to detect the differences in the propensity of CXCL11 and CXCL10 to induce β-arrestin 2 recruitment at CXCR3. As reported the reference compound **46**/VUF10661 showed the *pEC₅₀* value 5.89 ± 0.07 and nearly 40% higher efficacy than CXCL11¹⁸. Contrary to the G protein activation, the enantiomer of **46**/VUF 10661 compound **47** and the Boc protected derivatives **44** and **45** had a reduced ability to induce recruitment of β-arrestin 2 to CXCR3 (Table 3). The most striking difference was observed for the Boc protected enantiomer of **46**/VUF10661, compound **45,** which showed the reduced efficacy of about 36% compared to **46**/VUF10661. The presence of the positive charge at the amine on the southern side chain seems to significantly contribute to the activation of the β-arrestin 2 pathway.

**Table 3. Ability of the 46/VUF10661 based positive allosteric modulators to activate the CXCR3 as measured in the β-arrestin 2 recruitment assay (PathHunter, DiscoveRx).^{[a]}**

| | | | | |
|---|---|---|---|---|
| 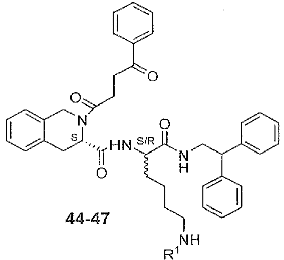 | | | | |

| **Cpd** | **Stereoconf.** | **R¹** | **CXCR3-PK1** | **CXCR3-PK2** |
|---|---|---|---|---|
| | | | ***pEC₅₀* ± SEM^{[b]} (%)^{[c]}** | ***pEC₅₀* ± SEM (%)^{[c]}** |
| **CXCL11** | | | 7.76±0.06 (100) | 7.75±0.17 (100) |
| **44** | S,S | Boc | >5.0 (70%@10µM) | nd^{[d]} |
| **45** | S,R | Boc | 6.63±0.17 (36±2) | 7.23±0.69 (30±9) |
| **46/VUF10661** | S,S | H | 5.89±0.07 (139±5) | nd^{[d]} |
| **47** | S,R | H | 5.96±0.08 (108±5) | 6.12±0.11 (198±12) |

| | | | | |
|---|---|---|---|---|
| ^{[a]}The ability of the positive allosteric modulators to recruit β-arrestin 2 to CXCR3 was determined with HEK293-T cells expressing all of the components of PathHunter (DiscoveRx) and CXCR3-PK1 or CXCR3-PK2. ^{[b]} The values reflect the mean *pEC₅₀* ± SEM of two to three experiments performed in triplicate. ^{[c]} The efficacy represents the % of CXCL11 mediated effect. ^{[d]} Not determined. | | | | |

FAUC1036 emerged as a strongly biased allosteric partial agonist as it activated the recruitment of β-arrestin 2 to CXCR3 with a *pEC₅₀* of 6.64 ± 0.38 and the efficacy of 30% (measured with PK1 construct, Table 4), whereas no G protein activation could be observed for this compound. Every substitution on the phenyl ring of the northern side chain, fluorine **(13),** methoxy (**14**/FAUC1104), sulfone **(15),** dimethoxy **(21)** groups resulted in complete loss of intrinsic agonistic properties in the β-arrestin 2 recruitment assay, results that suggest phenyl ring polar substitutions enhance biased agonism by preferentially activating G protein over recruitment of β-arrestin 2. Replacement of the benzoyl group with an acetyl group (compound **12**) or increase of the spacer length (compound **24**) behaved as strongly biased allosteric agonists with efficacies and potencies comparable to FAUC1036 (Table 4). For the compounds **22, 23,** where the keto group of the benzoyl moiety was removed, a significant loss of efficacy is observed indicating the importance of that group with respect to β-arrestin 2 recruitment. The S,R-epimer **34** reached the levels of full agonism in assays using PK2 tag. The charged analogues **25, 28** and **35** showed a decrease of efficacy. Compound **5,** which is devoid of the entire northern fragment, retained its ability to recruit β-arrestin 2 to CXCR3 (the *pEC₅₀* value 6.50±0.17, efficacy 31%), and showed no bias between the investigated pathways. Finally, the rest of the compounds, **16-20** and **27,** proved to have no agonistic effect in the given assays.

To investigate the efficacy of identified partial agonists in the assay format, the inventors used the CXCR3-PK2 construct, which is characterized by higher sensitivity to detect β-arrestin 2 recruitment. The compounds which appeared as weak partial agonists under CXCR3-PK1 conditions appeared as strong partial agonists under CXCR3-PK2 conditions with the efficacy increased up to 80% (Table 4). FAUC1036 reached the efficacy of 66%. No influence on the potency was observed (the *pEC*₅₀ value of 6.64 ± 0.30).

**Table 4. Ability of the compound 5 based positive allosteric modulators to activate the CXCR3 as measured in the β-arrestin 2 recruitment assay (PathHunter, DiscoveRx).^{[a]}**

| | | | | | | | | | | | |
|---|---|---|---|---|---|---|---|---|---|---|---|
| 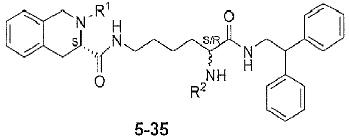 | | | | | | | | | | | |

| **Cpd** | **R¹** | **R²** | **Stereo conf.** | **CXCR3-PK1 *pEC_{S0}* ± SEM^{[b]} (%)^{[c]}** | **CXCR3-PK2 *pEC₅₀* ± SEM^{[b]} (%)^{[c]}** | **Cpd** | **R¹** | **R²** | **Stereo conf.** | **CXCR3-PK1 *pPEC₅₀* ± SEM^{[b]} (%)^{[c]}** | **CXCR3-PK2 *pEC₅₀* ± SEM (%)^{[c]}** |
|---|---|---|---|---|---|---|---|---|---|---|---|
| **CXCL11** | | | | 7.76±0.06 (100) | 7.75±0.17 (100) | **20** | 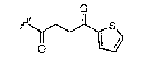 | Boc | S,S | No effect | nd^{[d]} |
| **5** | H | Boc | S,S | 6.50±0.17 (31±2) | 6.71±0.33 (79±12) | **21** | 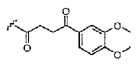 | Boc | S,S | No effect | nd^{[d]} |
| **11/FAUC103 6** | 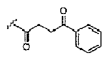 | Boc | S,S | 6.64±0.38 (30±5) | 6.58±0.30 (66±8) | **22** | 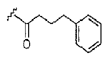 | Boc | S,S | No effect | nd^{[d]} |
| **12** | 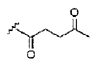 | Boc | S,S | 6.78±0.17 (34±2) | 6.64±0.33 (63±9) | **23** | 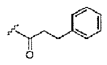 | Boc | S,S | 7.21±0.17 (19±1) | 6.71±0.30 (29±4) |
| **13** | 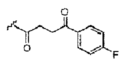 | Boc | S,S | No effect | nd^{[d]} | **24** | 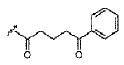 | Boc | S,S | 6.84±1.7 (20±2) | 6.78±0.35 (56±9) |
| **14/FAUC110 4** | 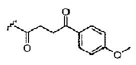 | Boc | S,S | No effect | nd^{[d]} | **25** | 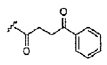 | H | S,S | 6.80±0.21 (17±1) | 6.51±0.34 (27±3) |
| **15** | 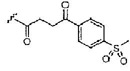 | Boc | S,S | No effect | nd^{[d]} | **26** | 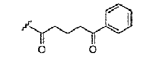 | H | S,S | 6.73±0.22 (20±2) | 6.90±0.21 (91±9) |
| **16** | 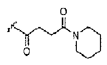 | Boc | S,S | No effect | nd^{[d]} | **27** | 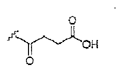 | Boc | S,S | No effect | No effect |
| **17** | 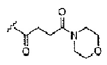 | Boc | S,S | No effect | nd^{[d]} | **28** | H | H | S,S | 6.80±0.45 (14±3) | 6.55±0.26 (85±10) |
| **18** | 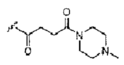 | Boc | S,S | No effect | nd^{[d]} | **34/ FAUC1095** | 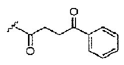 | Boc | S,R | 6.95±0.50 (16±3) | 6.86±0.19 (88±8) |
| **19** | 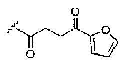 | Boc | S,S | No effect | nd^{[d]} | **35** | 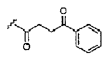 | H | S,R | 7.16±0.33 (28±4) | 6.52±0.43 (79±16) |

| | | | | | | | | | | | |
|---|---|---|---|---|---|---|---|---|---|---|---|
| ^{[a]}The ability of the positive allosteric modulators to recruit β-arrestin 2 to CXCR3 was determined with HEK293-T cells expressing all of the components of PathHunter (DiscoveRx) and CXCR3-PK1 or CXCR3-PK2. ^{[b]} The values reflect the mean *pEC*₅₀ ± SEM of two to three experiments performed in triplicate. ^{[c]} The efficacy represents the % of CXCL11 mediated effect. ^{[d]} Not determined. | | | | | | | | | | | |

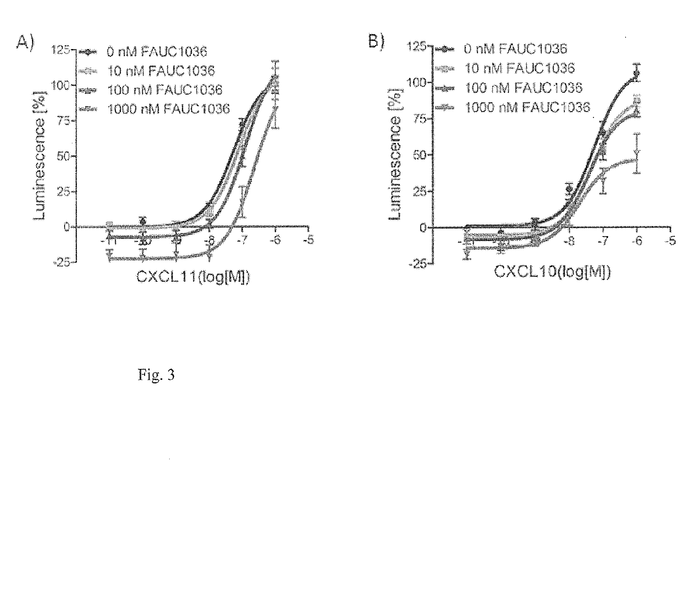

The effect of the β-arrestin 2 biased allosteric agonist FAUC1036 on the CXCL11 and CXCL10-induced β-arrestin 2 recruitment was estimated in a series of experiments where the HEK293T cells expressing CXCR3 were concomitantly incubated with FAUC1036 and chemokines CXCL11 or CXCL10 (Figure 3). FAUC1036 demonstrated distinct influence on CXCL11 *versus* CXCL10. In the presence of CXCL11 FAUC1036 influenced only the *pEC₅₀* of CXCL11 (7.26±0.08 in the absence of FAUC1036, 6.68±0.02 in the presence of 1000 nM FAUC1036) and had not influence on the efficacy (Figure 3A). In the presence of CXCL10 FAUC1036 had no significant influence on the *pEC₅₀* of CXCL10 (7.26±0.10 in the absence of FAUC1036, 7.69±0.27 in the presence of 1000 nM FAUC1036), but noticeable effect on the efficacy (Figure 3B). These observations imply that FAUC1036 behaves as a functional negative allosteric modulator (NAM) in the presence of endogenous agonists in the β-arrestin 2 pathway. Without wishing to be bound by any theory, the inventors assume that this observation could be based on the fact that direct allosteric agonism is not sustained for the duration of experiment (four hours) and thus does not results in elevated baseline and depression of maxima. FAUC1036 has namely no effect on the binding affinity of CXCL11 on CXCR3 (Supplementary Figure 3).

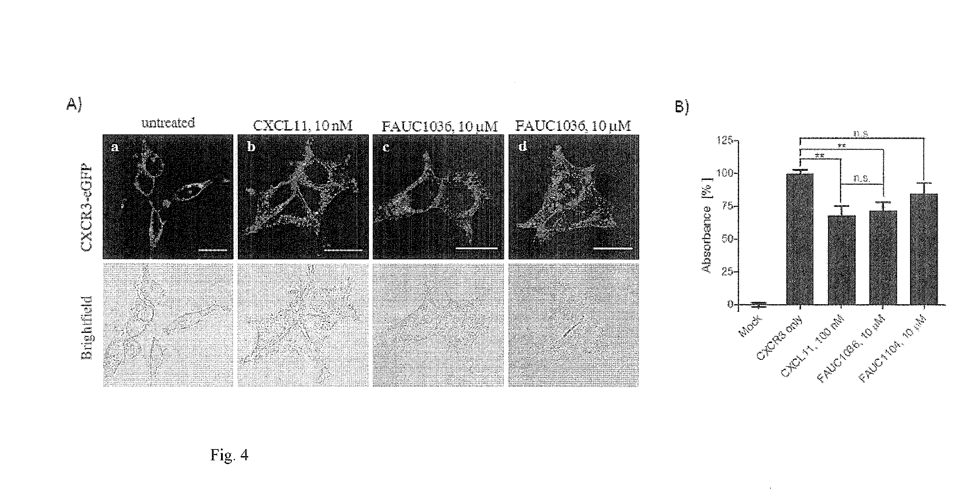

The effect of FAUC1036 on receptor internalization was investigated by scanning laser confocal microscopy and ELISA-based assay. The HEK293T cells were transiently transfected by CXCR3-eGFP and either left untreated or stimulated with CXCL11 and FAUC1036 for one hour. As evident in Figure 4A, the treatment with either CXCL11 or FAUC1036 induced the CXCR3 internalization. Untreated cells also show minor internalization of CXCR3. This observation is in the accordance with the published observation that CXCR3 shows constitutive internalization to some extent³³. The quantification of receptor internalization upon the treatment with the endogenous agonists and with either β-arrestin 2 biased FAUC1036 or G-protein biased FAUC1104 was performed with ELISA based assay. The HEK293T cells transiently expressing FLAG-CXCR3 were stimulated with compounds and chemokines for one hour, washed, fixed and the amount of FLAG-CXCR3 present on the surface was detected by anti-FLAG antibody. As evident from Figure 4B, only CXCL11 and FAUC1036 induced receptor internalization.

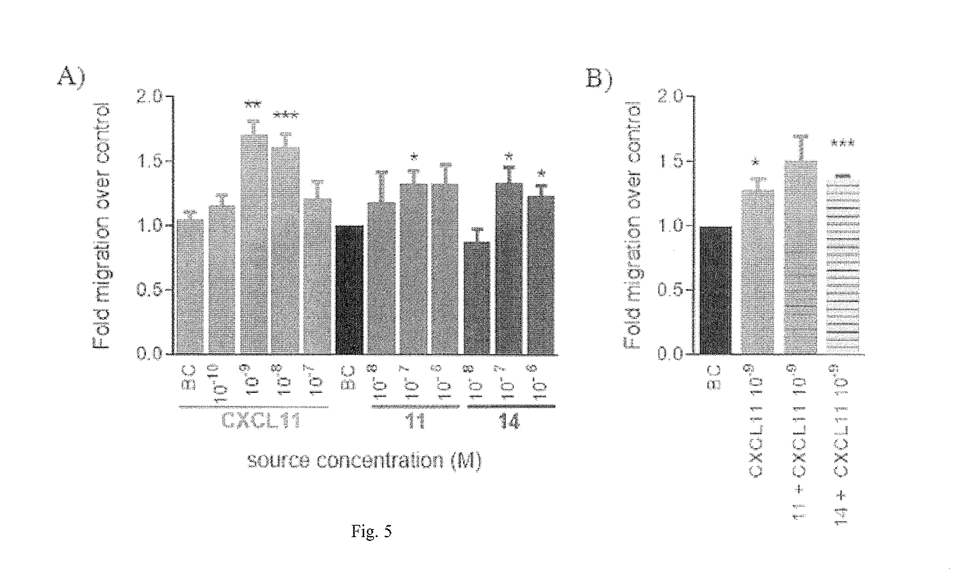

To investigate the influence of FAUC1036 and FAUC1104 on CXCR3 induced migration, the inventors used these compounds alongside CXCL11 in a standard transwell assay with HEK293 cells transiently transfected with CXCR3. When applied to the source reservoir both compounds on their own induce migration approximately 0.25 fold compared to buffer control, which is less than the maximal effect observed with CXCL11 (0.7 fold), but still significant, indicating that both compounds positively influence migration (Figure 5A). To test whether FAUC1036 and FAUC1104 influence CXCL11 induced migration, the inventors pre-incubated the cells with these compounds for 15 minutes and then set up the migration assay in the presence of either compound in both the top chamber (sink) containing the cells and in the lower chamber (source) containing CXCL11 as chemoattractant. The inventors observed no antagonizing effect of these compounds on CXCL11 induced migration, but rather a significantly higher CXCL11 induced migration in the presence of FAUC1104 (Figure 5B).

Previously reported CXCR3 agonists (Figure 1) were shown to induce migration in the absence of endogenous chemokines^{16,18,21}. Only PS372424 was tested for its influence on CXCL11 induced migration and it was, interestingly, found to antagonize CXCL11 induced migration in activated T-cells²¹.

Overall, detailed pharmacological characterization of most prominent derivatives FAUC1036 and FAUC1104 demonstrated the multiple dimensions of biased agonism induced by these compounds. FAUC1036, strongly biased agonist, induces β-arrestin 2 recruitment, CXCR3 internalization and some cell migration without any involvement of G proteins. It is tempting to suggest that FAUC1036 stimulates weak migration of CXCR3⁺ HEK293 cells through β-arrestin induced signaling. In the presence of CXCL11, FAUC1036 does not inhibit or potentiate chemotaxis. Its derivative FAUC1104 does not induces β-arrestin 2 recruitment and CXCR3 internalization, but activates G proteins. This compound induces stronger cell migration compared to FAUC1036. In the presence of CXCL11, FAUC1104 enhances the chemotaxis driven by CXCL11 and is thus positive allosteric modulator in this pathway.

### Conclusions

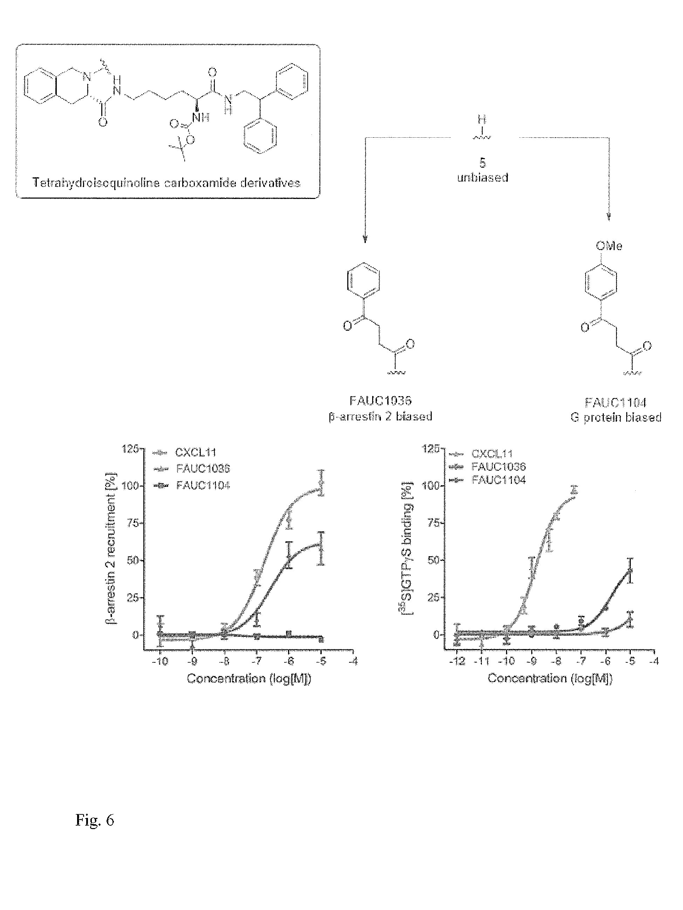

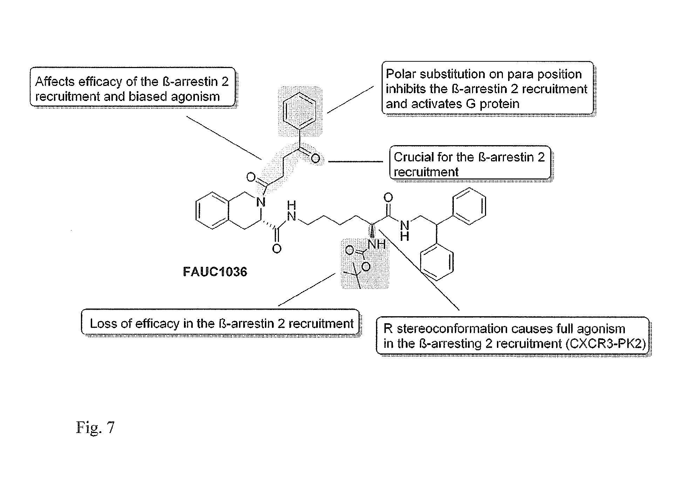

Selective regulation of CXCR3 signaling by an appropriate allosteric agonist is an attractive therapeutic strategy in inflammatory diseases. However, the complex pharmacology of the CXCR3 signaling system poses a nontrivial challenge for the design of biased ligands that selectively activate desired pathways. In this application, the inventors report unique strongly biased allosteric agonists of CXCR3 that contain a privileged isoquinoline carboxamide core. FAUC1036 (Figure 6) is the first strongly biased allosteric agonist of CXCR3 that exclusively stimulates the receptor internalization and the β-arrestin 2 recruitment with the potency comparable to the endogenous chemokine CXCL11 and the efficacy of 66% (using CXCR3-PK2) without any activation of G proteins (Figure 6). An additional methoxy group leads to the strongly biased allosteric agonist FAUC1104 that activated solely G proteins (the *pEC₅₀* value 5.22, efficacy 50%). Concomitant structure-activity studies identified molecular switches which steer the ligand bias between the β-arrestin 2 and G protein pathway (Figure 7). Detailed analysis of the influence of FAUC1036 and FAUC1104 on receptor internalization and chemotaxis showed that FAUC1036 induces receptor internalization and weak chemotaxis, FAUC1104 on the other hand does not induce receptor internalization but strong chemotaxis. Overall, the information presented is crucial for rational design and further development of strongly biased allosteric agonists of CXCR3.

### REFERENCES

(1) Kenakin, T.; Christopoulos, A. Signalling Bias in New Drug Discovery: Detection, Quantification and Therapeutic Impact. Nat. Rev. Drug Discov. 2013, 12, 205-216.
(2) Christopoulos, A.; Kenakin, T. G Protein-Coupled Receptor Allosterism and Complexing. Pharmacol. Rev. 2002, 54, 323-374.
(3) Stallaert, W.; Christopoulos, A.; Bouvier, M. Ligand Functional Selectivity and Quantitative Pharmacology at G Protein-Coupled Receptors. Expert Opinion on Drug Discovery, 2011, 6, 811-825.
(4) Tschammer, N.; Christopoulos, A.; Kenakin, T. Chemokines: Chemokines and Their Receptors in Drug Discovery. In Chemokines; Tschammer, N., Ed.; Topics in Medicinal Chemistry; Springer Berlin Heidelberg, 2015; pp. 84-118.
(5) Groom, J. R.; Luster, A. D. CXCR3 Ligands: Redundant, Collaborative and Antagonistic Functions, Immunol. Cell Biol. 2011, 89, 207-215.
(6) Groom, J. R.; Luster, A. D. CXCR3 in T Cell Function. Exp. Cell Res. 2011, 317, 620-631.
(7) Kouroumalis, A.; Nibbs, R. J. B.; Aptel, H.; Wright, K. L.; Kolios, G.; Ward, S. G. The Chemokines CXCL9, CXCL10, and CXCL11 Differentially Stimulate G Alpha I-Independent Signaling and Actin Responses in Human Intestinal Myofibroblasts. J. Immunol. 2005, 175, 5403-5411.
(8) Tsubaki, T.; Takegawa, S.; Hanamoto, H.; Arita, N.; Kamogawa, J.; Yamamoto, H.; Takubo, N.; Nakata, S.; Yamada, K.; Yamamoto, S.; Yoshie, O.; Nose, M. Accumulation of Plasma Cells Expressing CXCR3 in the Synovial Sublining Regions of Early Rheumatoid Arthritis in Association with Production of Mig/CXCL9 by Synovial Fibroblasts. Clin. Exp. Immunol. 2005, 141, 363-371.
(9) Lee, J. H.; Kim, H. N.; Kim, K. O.; Jin, W. J.; Lee, S.; Kim, H. H.; Ha, H.; Lee, Z. H. CXCL10 Promotes Osteolytic Bone Metastasis by Enhancing Cancer Outgrowth and Osteoclastogenesis. Cancer Res. 2012, 72, 3175-3186.
(10) Kawada, K.; Hosogi, H.; Sonoshita, M.; Sakashita, H.; Manabe, T.; Shimahara, Y.; Sakai, Y.; Takabayashi, A.; Oshima, M.; Taketo, M. M. Chemokine Receptor CXCR3 Promotes Colon Cancer Metastasis to Lymph Nodes. Oncogene 2007, 26, 4679-4688.
(11) Fulton, A. M. The Chemokine Receptors CXCR4 and CXCR3 in Cancer. Curr. Oncol. Rep. 2009, 11, 125-131.
(12) Viola, A.; Luster, A. D. Chemokines and Their Receptors: Drug Targets in Immunity and Inflammation. Annu Rev Pharmacol Toxicol 2008, 48, 171-197.
(13) Wijtmans, M.; Verzijl, D.; Leurs, R.; de Esch, I. J. P.; Smit, M. J. Towards Small-Molecule CXCR3 Ligands with Clinical Potential. ChemMedChem 2008, 3, 861-872.
(14) Jenh, C. H.; Cox, M. A.; Cui, L.; Reich, E. P.; Sullivan, L.; Chen, S. C.; Kinsley, D.; Qian, S.; Kim, S. H.; Rosenblum, S.; Kozlowski, J.; Fine, J. S.; Zavodny, P. J.; Lundell, D. A Selective and Potent CXCR3 Antagonist SCH 546738 Attenuates the Development of Autoimmune Diseases and Delays Graft Rejection. BMC Immunol 2012, 13, 2.
(15) Heise, C. E.; Pahuja, a; Hudson, S. C.; Mistry, M. S.; Putnam, a L.; Gross, M. M.; Gottlieb, P. a; Wade, W. S.; Kiankarimi, M.; Schwarz, D.; Crowe, P.; Zlotnik, a; Alleva, D. G. Pharmacological Characterization of CXC Chemokine Receptor 3 Ligands and a Small Molecule Antagonist. J Pharmacol Exp Ther 2005, 313, 1263-1271.
(16) Stroke, I. L.; Cole, A. G.; Simhadri, S.; Brescia, M.-R.; Desai, M.; Zhang, J. J.; Merritt, J. R.; Appell, K. C.; Henderson, I.; Webb, M. L. Identification of CXCR3 Receptor Agonists in Combinatorial Small-Molecule Libraries. Biochem. Biophys. Res. Commun. 2006, 349, 221-228.
(17) Nedjai, B.; Li, H.; Stroke, I. L.; Wise, E. L.; Webb, M. L.; Merritt, J. R.; Henderson, I.; Klon, A. E.; Cole, A. G.; Horuk, R.; Vaidehi, N.; Pease, J. E. Small Molecule Chemokine Mimetics Suggest a Molecular Basis for the Observation That CXCL10 and CXCL11 Are Allosteric Ligands of CXCR3. Br. J. Pharmacol. 2012, 166, 912-923.
(18) Scholten, D. J.; Canals, M.; Wijtmans, M.; de Munnik, S.; Nguyen, P.; Verzijl, D.; de Esch, I. J. P.; Vischer, H. F.; Smit, M. J.; Leurs, R. Pharmacological Characterization of a Small-Molecule Agonist for the Chemokine Receptor CXCR3. Br. J. Pharmacol. 2012, 166, 898-911.
(19) Bodnar, R. J.; Yates, C. C.; Rodgers, M. E.; Du, X.; Wells, A. IP-10 Induces Dissociation of Newly Formed Blood Vessels. J. Cell Sci. 2009, 122, 2064-2077.
(20) O'Boyle, G.; Fox, C. R. J.; Walden, H. R.; Willet, J. D. P.; Mavin, E. R.; Hine, D. W.; Palmer, J. M.; Barker, C. E.; Lamb, C. A.; Ali, S.; Kirby, J. A. Chemokine Receptor CXCR3 Agonist Prevents Human T-Cell Migration in a Humanized Model of Arthritic Inflammation. Proc. Natl. Acad. Sci. U.S. A. 2012, 109, 4598-4603.
(21) Ali, S.; O'Boyle, G.; Mellor, P.; Kirby, J. A. An Apparent Paradox: Chemokine Receptor Agonists Can Be Used for Anti-Inflammatory Therapy. Mol. Immunol. 2007, 44, 1477-1482.
(22) Wijtmans, M.; Scholten, D. J.; Roumen, L.; Canals, M.; Custers, H.; Glas, M.; Vreeker, M. C. A.; De Kanter, F. J. J.; De Graaf, C.; Smit, M. J.; De Esch, I. J. P.; Leurs, R. Chemical Subtleties in Small-Molecule Modulation of Peptide Receptor Function: The Case of CXCR3 Biaryl-Type Ligands. J. Med. Chem. 2012, 55, 10572-10583.
(23) Bernat, V.; Admas, T. H.; Brox, R.; Heinemann, F. W.; Tschammer, N. Boronic Acids as Probes for Investigation of Allosteric Modulation of the Chemokine Receptor CXCR3. ACS Chem. Biol. 2014, 9, 2664-2677.
(24) Bernat, V.; Brox, R.; Heinrich, M. R.; Auberson, Y. P.; Tschammer, N. Ligand-Biased and Probe-Dependent Modulation of Chemokine Receptor CXCR3 Signaling by Negative Allosteric Modulators. ChemMedChem 2015, 10, 566-574.
(25) Banik, B. K.; Becker, F. F. Polycyclic Aromatic Compounds as Anticancer Agents: Structure-Activity Relationships of Chrysene and Pyrene Derivatives. Bioorganic and Medicinal Chemistry, 2001, 9, 593-605.
(26) Staszewski, M.; Walczyński, K. 1-Phenoxyalkyl-4-[(N,N-Disubstitutedamino)alkyl]piperazine Derivatives as Non-Imidazole Histamine H3-Antagonists. Med. Chem. Res. 2013, 22, 1287-1304.
(27) Zhao, M.; Li, W.; Li, X.; Ren, K.; Tao, X.; Xie, X.; Ayad, T.; Ratovelomanana-Vidal, V.; Zhang, Z. Enantioselective Ruthenium (II)/Xyl-SunPhos/Daipen-Catalyzed Hydrogenation of Γ-Ketoamides, J. Org. Chem. 2014, 79, 6164-6171.
(28) Harel, D.; Schepmann, D.; Wünsch, B. New Combination of Pharmacophoric Elements of Potent ∑ 1 Ligands: Design, Synthesis and ∑ Receptor Affinity of Aminoethyl Substituted Tetrahydrobenzothiophenes. Eur. J. Med. Chem. 2013, 69, 490-497.
(29) Shahabuddin, S.; Ji, R.; Wang, P.; Brailoiu, E.; Dun, N.; Yang, Y.; Aksoy, M. O.; Kelsen, S. G. CXCR3 Chemokine Receptor-Induced Chemotaxis in Human Airway Epithelial Cells: Role of p38 MAPK and PI3K Signaling Pathways. Am. J. Physiol. Cell Physiol. 2006, 291, C34-C39.
(30) Wang, F.; Herzmark, P.; Weiner, O. D.; Srinivasan, S.; Servant, G.; Bourne, H. R. Lipid Products of PI(3)Ks Maintain Persistent Cell Polarity and Directed Motility in Neutrophils. Nat. Cell Biol. 2002, 4, 513-518.
(31) Rajagopal, S.; Kim, J.; Ahn, S.; Craig, S.; Lam, C. M.; Gerard, N. P.; Gerard, C.; Lefkowitz, R. J. Beta-Arrestin- but Not G Protein-Mediated Signaling by the "Decoy" Receptor CXCR7. Proc. Natl. Acad. Sci. U. S. A. 2010, 107, 628-632.
(32) Simard, E.; Kovacs, J. J.; Miller, W. E.; Kim, J.; Grandbois, M.; Lefkowitz, R. J. B-Arrestin Regulation of Myosin Light Chain Phosphorylation Promotes AT1aR-Mediated Cell Contraction and Migration. PLoS One 2013, 8, e80532.
(33) Meiser, A.; Mueller, A.; Wise, E. L.; McDonagh, E. M.; Petit, S. J.; Saran, N.; Clark, P. C.; Williams, T. J.; Pease, J. E. The Chemokine Receptor CXCR3 Is Degraded Following Internalization and Is Replenished at the Cell Surface by de Novo Synthesis of Receptor. J. Immunol. 2008, 180, 6713-6724.
(34) Staszewski, M.; Walczyński, K. 1-Phenoxyalkyl-4-[(N,N-Disubstitutedamino)alkyl]piperazine Derivatives as Non-Imidazole Histamine H3-Antagonists. Med. Chem. Res. 2013, 22, 1287-1304.
(35) Lowry, O. H.; Rosebrough, N. J.; Farr, L.; Randall, R. J. Protein Measurement with the Folin Phenol Reagent. J. Biol. Chem. 1951, 193, 265-275.
(36) McConahey, P. J.; Dixon, F. J. Radioiodination of Proteins by the Use of the Chloramine-T Method. Methods Enzymol. 1980, 70, 210*.*
(37) Graham, F. L.; Van der Eb, A. J. A New Technique for the Assay of Infectivity of Human Adenovirus 5 DNA. Virology 1973, 52, 456-467.

The features of the present invention disclosed in the specification, and/or the claims may, both separately and in any combination thereof, be material for realizing the invention in various forms thereof.

## Claims

1. A compound having the structure represented by formula I 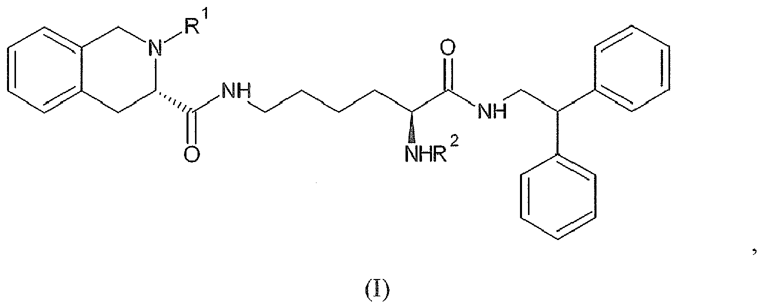 wherein
R¹ is H or a radical represented by formula II 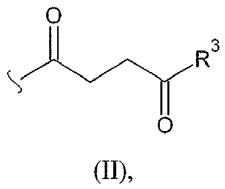 or formula III 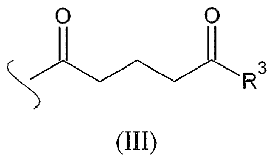 wherein R² is H or tert-butyloxycarbonyl (Boc),
with R³ being methyl,
phenyl 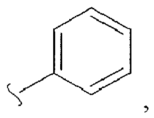 or 4-methoxy-phenyl 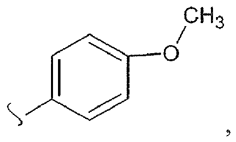 or epimers of said compound,
or a pharmaceutically acceptable salt thereof or of its epimers.

2. The compound according to claim 1, wherein
R¹ is H, R² is tert-butyloxycarbonyl (Boc) or H,
or epimers of said compound,
or a pharmaceutically acceptable salt thereof or of its epimers.

3. The compound according to claim 1 wherein R¹ is
a radical represented by formula II 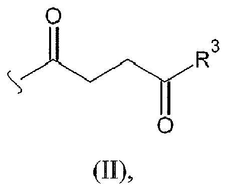 R² is tert-butyloxycarbonyl (Boc) or H,
with R³ being methyl or phenyl,
or epimers of said compound,
or a pharmaceutically acceptable salt thereof or of its epimers.

4. The compound according to claim 1, wherein R¹ is
a radical represented by formula II 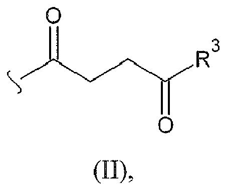 R² is tert-butyloxycarbonyl (Boc) or H,
with R³ being 4- methoxy-phenyl,
or epimers of said compound,
or a pharmaceutical salt thereof or of its epimers.

5. The compound according to claim 1, wherein R¹ is a radical represented by formula III 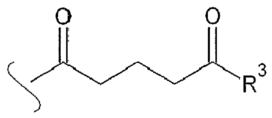 R² is tert-butyloxycarbonyl (Boc) or H, and R³ is phenyl
or epimers of said compound,
or a pharmaceutical salt thereof or of its epimers.

6. The compound according to claim 1, being selected from 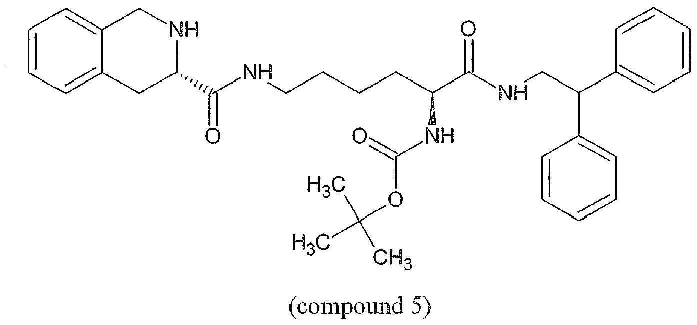 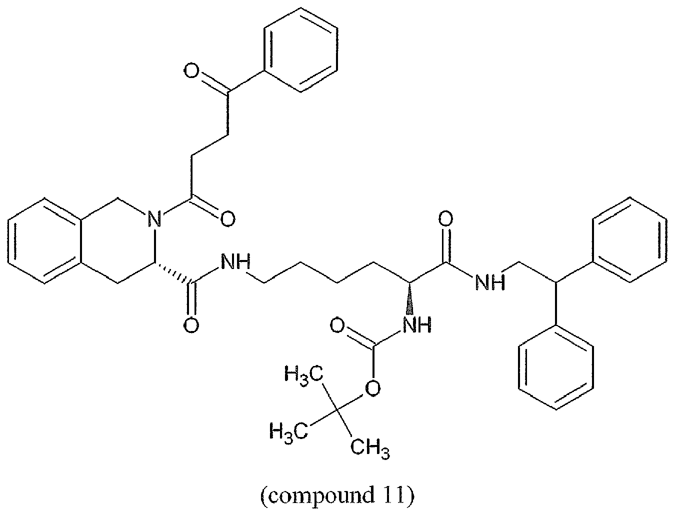 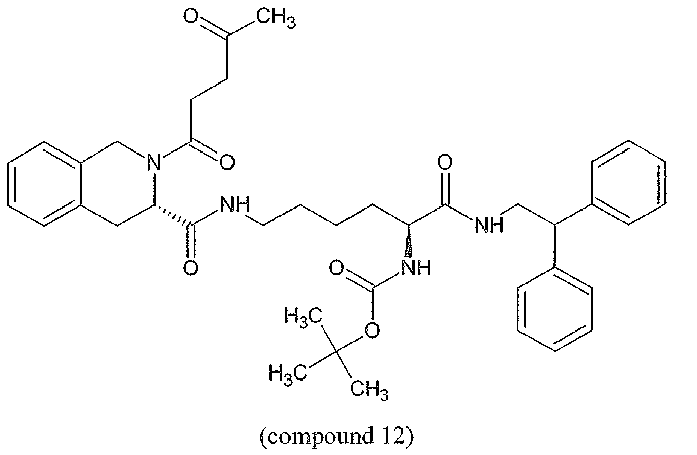 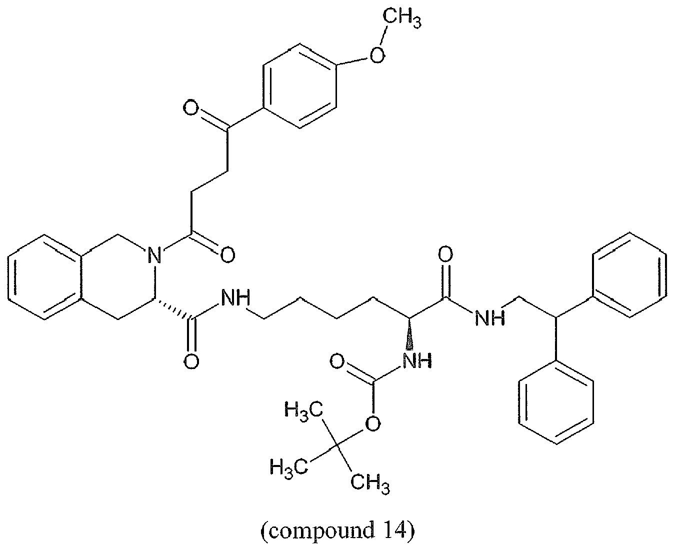 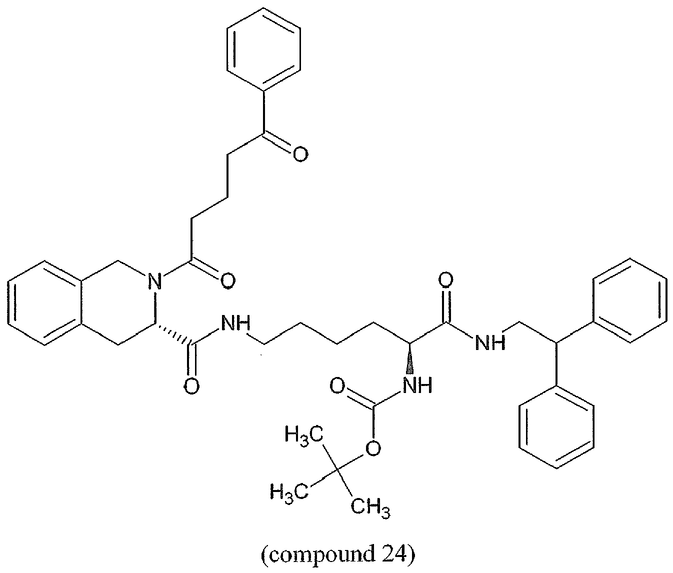 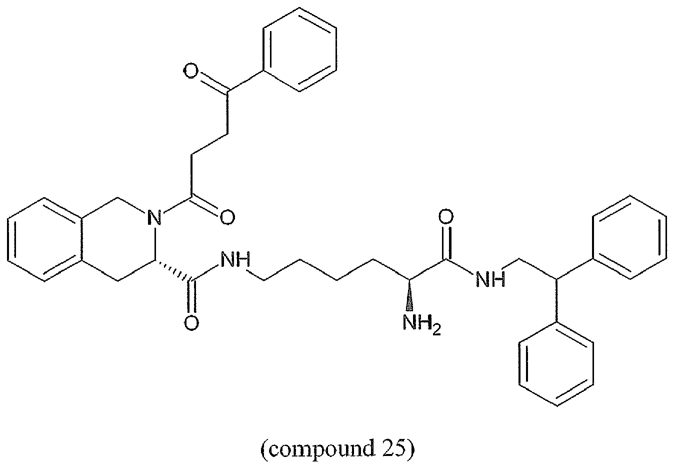 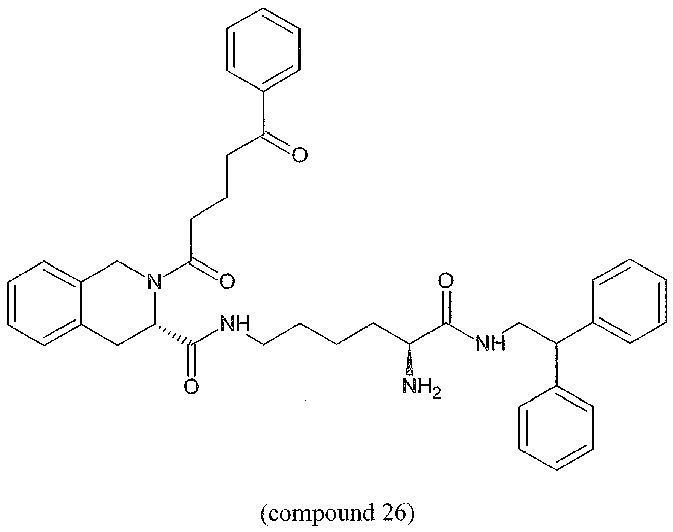 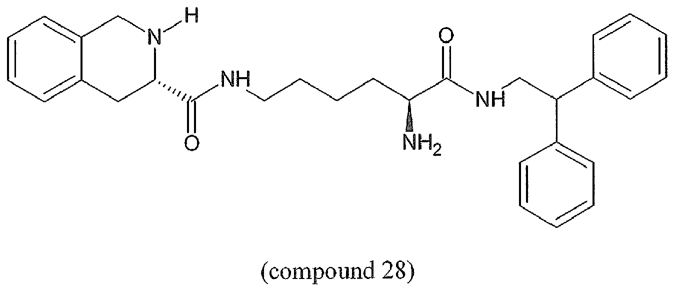 or epimers of said compound,
or a pharmaceutically acceptable salt thereof or of its epimers.

7. The compound according to any of the foregoing claims, for use as a pharmaceutical.

8. The compound according to any of claims 1 - 6, for use in the treatment of an inflammatory disease, cancer, diabetes, transplant rejection, Alzheimer disease, HIV-induced dementia, ischemia, in particular cerebral ischemia, cardiac ischemia, stroke, myocardial infarction, or for use in the alleviation of wound healing.

9. The compound for use according to claim 8, wherein said inflammatory disease is a disease selected from rheumatoid arthritis, ankylosing spondylitis, inflammation post infection, atherosclerosis, myocardial infarction, stroke, pain, dermatitis, psoriasis, atopic skin disease, chronic granulomatous diseases such as tuberculosis, leprosy, sarcoidosis, silicosis, nephritis, amyloidosis, scleroderma, lupus, polymyositis, osteoporosis, sepsis, inflammatory bowel disease, ulcers, appendicitis, diverticulitis, Sjogren's syndrome, Reiter's syndrome, pelvic inflammatory disease, orbital inflammatory disease, peritonitis, hepatitis, thrombotic disease, inappropriate allergic responses to environmental stimuli such as poison ivy, pollen, insect stings and certain foods, including atopic dermatitis and contact dermatitis, allergic encephalitis, asthma, lung inflammation, COPD, chronic bronchitis, multiple sclerosis,
and wherein the cancer is selected from melanoma, osteosarcoma, prostate, colon and breast cancer and metastasis of the aforementioned cancers,
and wherein the diabetes is selected from diabetes type 1.

10. Compound for use according to any of claims 7 - 9, wherein a suitable amount of said compound is administered to a patient in need thereof.

11. A method of treatment of an inflammatory disease, cancer, or diabetes, transplant rejection, Alzheimer disease, HIV-induced dementia, ischemia, in particular cerebral ischemia, cardiac ischemia, stroke, myocardial infarction, or a method of alleviation of wound healing, wherein said method comprises the administration of a suitable amount of a compound according to any of claims 1 - 6 to a patient in need thereof.

12. Use of a compound according to any of claims 1 - 6, as a chemical probe, in particular for elucidating functioning of the chemokine receptor CXCR3 and/or signalling pathway(s) associated therewith, wherein, preferably said use is in-vitro or in-vivo.

13. A method of synthesis of a compound represented by formula (I) 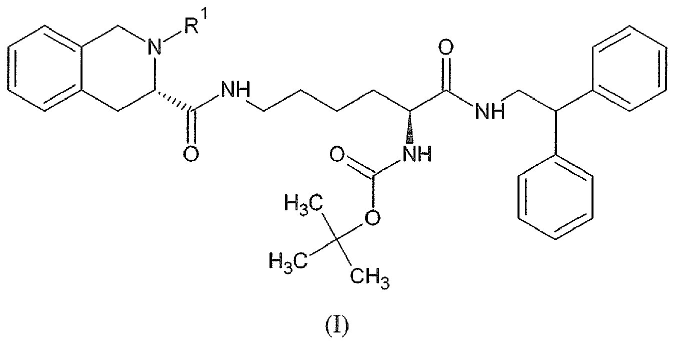 or of an epimer thereof,
wherein R¹ is H,
said method occurring in accordance with the following scheme and comprising the steps: 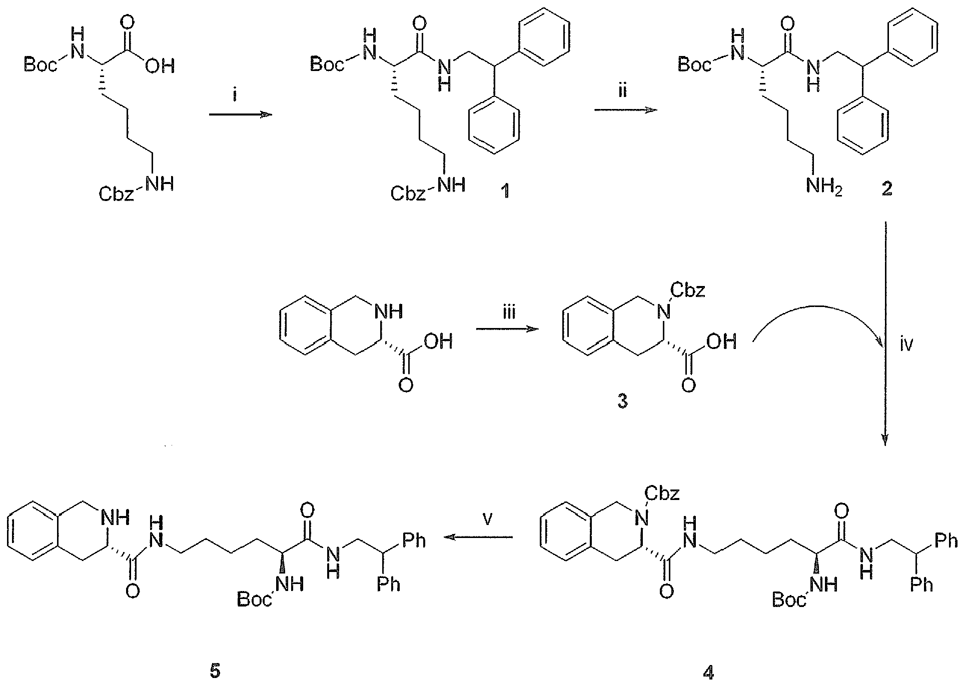
- acylation between Boc-Lys(Z)-OH and biphenylamine in the presence of 1-ethyl-3-(3-dimethylaminopropyl)carbodiimide (EDC) to yield compound 1,
- hydrogenolysis to afford primary amine 2,
- carboxybenzyl (Cbz) protection of S-2-((benzyloxy)carbonyl)-1,2,3,4-tetrahydroisoquinoline-3-carboxylic acid using benzyl chloroformate in basic medium to give compound 3,
- amide coupling of compound 3 and compound 2 to give compound 4,
- hydrogenolysis of compound 4 to yield compound 5.

14. A method of synthesis of a compound represented by formula (I) 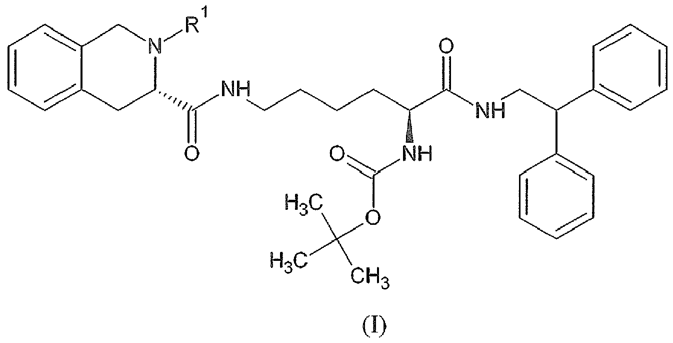 or of an epimer thereof,
wherein R¹ is a radical represented by formula II 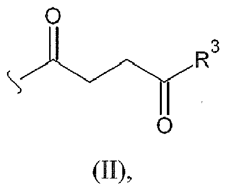 with R³ being methyl,
phenyl 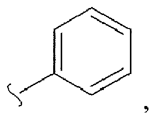 or 4-methoxy-phenyl 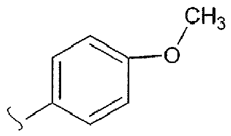 said method comprising the steps:
reacting compound 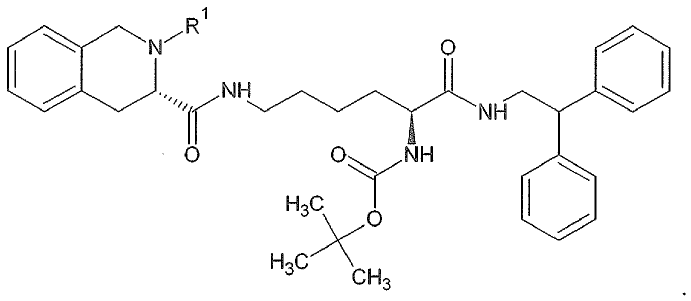 or an epimer thereof, wherein R¹ is H, with a carboxylic acid compound according to the scheme 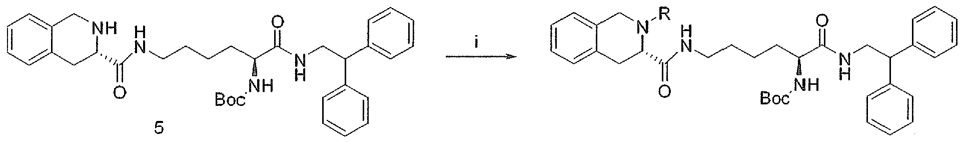 Wherein the carboxylic acid compound is 3-benzoylpropionic acid 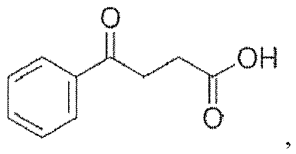 3-(4-methoxybenzoyl)propionic acid 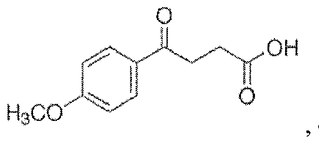 or 4-oxo-pentanoic acid 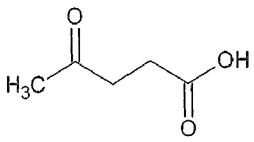 and wherein in the reaction product R is a radical represented by formula II 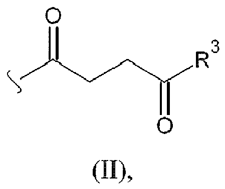 with R³ being methyl,
phenyl 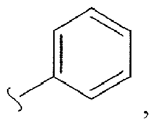 or 4-methoxy-phenyl
